# EUROPÄISCHE PATENTSCHRIFT

(11) **EP 2 502 907 B1**
(45) Veröffentlichungstag und Bekanntmachung des Hinweises auf die Patenterteilung: **29.08.2018**
(21) Anmeldenummer: 12003856.7
(22) Anmeldetag: 25.03.2009
(51) Int. Cl.: C07D 209/12, C07D 209/14, C07C 211/40, C07C 215/42, C07C 233/36, C07C 311/18, A61K 31/404, A61K 31/137, A61P 25/00, C07C 215/14, C07C 217/74, C07C 223/04, C07C 229/16, C07C 233/40, C07C 233/41, C07C 233/62, C07C 233/79, C07C 235/54, C07C 237/06, C07C 275/24, C07C 311/07

(54) **Substituierte 4-Aminocyclohexan-Derivate**
Substituted 4-aminocyclohexane derivatives
Dérivés de 4-aminocyclohexane substitués

(30) Priorität: 27.03.2008 EP 08005748
(43) Veröffentlichungstag der Anmeldung: 26.09.2012
(62) Teilanmeldung aus: 09725217.5
(73) Patentinhaber: Grünenthal GmbH, 52078 Aachen (DE)
(72) Erfinder: Nolte, Bernd, 53902 Bad Münstereifel (DE); Schröder, Wolfgang, 52074 Aachen (DE); Linz, Klaus, 53343 Wachtberg (DE); Englberger, Werner, 52223 Stolberg (DE); Schick, Hans, 13086 Berlin-Weissensee (DE); Graubaum, Habil Heinz, 12557 Berlin (DE); Braun, Birgit, 12101 Berlin (DE); Ozegowski, Sigrid, 12459 Berlin (DE); Bálint, József, 12526 Berlin (DE); Sonnenschein, Helmut, 10245 Berlin (DE)
(74) Vertreter: Kutzenberger Wolff & Partner

(56) Entgegenhaltungen:
- WO-A-01/87838
- WO-A-02/090317
- WO-A1-00/25770
- DE-A1- 2 839 891
- US-A- 4 115 589
- CHU ET AL: "Synthesis and DNA binding studies of bis-intercalators with a novel spiro-cyclic linker", TETRAHEDRON, ELSEVIER SCIENCE PUBLISHERS, AMSTERDAM, NL, Bd. 62, Nr. 23, 5. Juni 2006 (2006-06-05), Seiten 5536-5548, XP005423848, ISSN: 0040-4020

## Beschreibung

Die vorliegende Erfindung betrifft substituierte Cyclohexan-Derivate, welche eine Affinität an den µ-Opioid-Rezeptor und den ORL1-Rezeptor aufweisen, Arzneimittel enthaltend diese Verbindungen und die Verwendung dieser Verbindungen zur Herstellung von Arzneimitteln.

Cyclohexan-Derivate, welche eine Affinität an den µ-Opioid-Rezeptor und den ORL1-Rezeptor aufweisen, sind im Stand der Technik bekannt. In diesem Zusammenhang kann beispielsweise vollumfänglich verwiesen werden auf WO2002/090317, WO2002/90330, WO2003/008370, WO2003/008371, WO2003/080557, WO2004/043899, WO2004/043900, WO2004/043902, WO2004/043909, WO2004/043949, WO2004/043967, WO2005/063769, WO2005/066183, WO2005/110970, WO2005/110971, WO2005/110973, WO2005/110974, WO2005/110975, WO2005/110976, WO2005/110977, WO2006/018184, WO2006/108565, WO2007/079927, WO2007/079928, WO2007/079930, WO2007/079931, WO2007/124903, WO2008/009415 und WO2008/009416.

Die bekannten Verbindungen sind jedoch nicht in jeglicher Hinsicht zufrieden stellend und es besteht ein Bedarf an weiteren Verbindungen mit vergleichbaren oder besseren Eigenschaften.

So zeigen die bekannten Verbindungen in geeigneten Bindungsassays mitunter eine gewisse Affinität zum hERG-lonenkanal, zum L-Typ Calcium-Ionenkanal (Phenylalkylamin-, Benzothiazepin-, Dihydropyridin-Bindungsstellen) bzw. zum Natrium-Kanal im BTX-Assay (Batrachotoxin), was jeweils als Anzeichen für kardiovaskuläre Nebenwirkungen gedeutet werden kann. Ferner zeigen zahlreiche der bekannten Verbindungen eine nur geringe Löslichkeit in wässrigen Medien, was sich u.a. negativ auf die Bioverfügbarkeit auswirken kann. Darüber hinaus ist die chemische Stabilität der bekannten Verbindungen oft nur unzureichend. So zeigen die Verbindungen mitunter keine ausreichende pH-, UV- bzw. Oxidationsstabilität, was sich u.a. negativ auf die Lagerstabilität und auch auf die orale Bioverfügbarkeit auswirken kann. Ferner weisen die bekannten Verbindungen teilweise ein ungünstiges PK/PD (Pharmakokinetik/Pharmakodynamik)-Profil auf, was sich z.B. in einer zu langen Wirkdauer zeigen kann.

Auch die metabolische Stabilität der bekannten Verbindungen scheint verbesserungsbedürftig. Eine verbesserte metabolische Stabilität kann auf eine erhöhte Bioverfügbarkeit hinweisen. Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Ferner zeigen die bekannten Verbindungen mitunter eine nur geringe Selektivität gegenüber dem kappa-Opioid-Rezeptor auf, welcher für Nebenwirkungen, insbesondere Dysphorie, Sedation, Diurese verantwortlich ist. Darüber hinaus zeigen die bekannten Verbindungen mitunter eine sehr hohe Affinität an den µ-Opioid-Rezeptor, welcher im Zusammenhang mit anderen Nebenwirkungen, insbesondere Atemdepression, Obstipation und Suchtabhängigkeit zu stehen scheint.

WO 01/87838 offenbart NK-1-Rezeptorantagonisten.

J. Med. Chem. 1996, 9, 911-920; J. Am. Chem. Soc. 1950, 72, 2411-2417; und Tetrahedron 2006, 62, 5536-5548 offenbaren jeweils u.a. geminal substituierte Cyclohexyl-1,4-diamine, bei denen die Aminogruppen jedoch durchweg mit Wasserstoffatomen substituiert sind.

DE 28 39 891 A1 offenbart u.a. 4-(Dimethylamino)-1-methyl-4-p-tolylcyclohexyl acetat.

WO 00/25770 offenbart carbocyclische Kaliumkanalhemmer.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen.

Diese Aufgabe wird durch den Gegenstand der Patentansprüche gelöst.

Es wurde überraschend gefunden, dass substituierte Cyclohexan-Derivate hergestellt werden können, welche eine Affinität an den µ-Opioid-Rezeptor und den ORL1-Rezeptor aufweisen.

Die Erfindung betrifft Verbindungen der allgemeinen Formel (2.2) 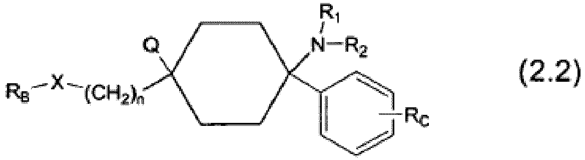 wobei
Q für -C₁₋₈-Aliphat, -Aryl, -C₁₋₈-Aliphat-Aryl, -Heteroaryl, -C(=O)-Heteroaryl oder -C(=NH)-Heteroaryl steht;
R₁ für -CH₃ steht;
R₂ für -H oder -CH₃ steht; oder
R₁ und R₂ gemeinsam einen Ring bilden und für -(CH₂)₃₋₄- stehen;
X für -O- oder -NR_{A}- steht;
R_{A} für -H oder -C₁₋₈-Aliphat steht;
R_{B} für -C(=O)-C₃₋₈-Cycloaliphat-Aryl, -C(=O)-C₃₋₈-Cycloaliphat-Heteroaryl, -C(=O)NH-C₁₋₆-Aliphat, -S(=O)₁₋₂-C₁₋₈-Aliphat, -S(=O)₁₋₂-Aryl, -S(=O)₁₋₂-Heteroaryl, -S(=O)₁₋₂-C₁₋₈-Aliphat-Aryl, -S(=O)₁₋₂-C₁₋₈-Aliphat-Heteroaryl, -S(=O)₁₋₂-C₃₋₈-Cycloaliphat-Aryl oder -S(=O)₁₋₂-C₃₋₈-Cycloaliphat-Heteroaryl steht; oder R_{A} und R_{B} gemeinsam einen Ring bilden und für -(CH₂)₃₋₄- stehen;
R_{C} für -H, -F, -Cl, -Br, -I, -CN, -NO₂, -CF₃, -OH oder -OCH₃ steht; und
n für 0, 1, 2, 3 oder 4 steht;
wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf); wobei ggf. ein oder zwei Kohlenstoffatome unabhängig voneinander durch ein Heteroatom S, N oder O ersetzt sind;
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)-NHR₀, -NH-C(=O)N(R₀)₂, -Si(R₀)₃ und -PO(OR₀)₂ verstanden wird;
wobei R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃ oder -PO(OR₀)₂ verstanden wird;
wobei ggf. vorhandene N-Ringatome jeweils oxidiert sein können;
wobei R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
in Form eines einzelnen Stereoisomers oder deren Mischung, der freien Verbindungen und/oder ihrer physiologisch verträglichen Salze.

Bei der Zusammenfassung verschiedener Reste, beispielsweise Y₁, Y₁', Y₂, Y₂', Y₃, Y₃', Y₄ und Y₄' sowie der Zusammenfassung von Resten an deren Substituenten, wie z. B. -OR₀, -OC(=O)R₀, -OC(=O)NHR₀, kann ein Substituent, z.B. R₀, für zwei oder mehrere Reste, beispielsweise -OR₀, -OC(=O)R₀, -OC(=O)NHR₀, innerhalb einer Substanz unterschiedliche Bedeutungen annehmen.

Die erfindungsgemäßen Verbindungen zeigen gute Bindung an den ORL1-Rezeptor und den µ-Opioid-Rezeptor.

In einer bevorzugten Ausführungsform weisen die erfindungsgemäßen Verbindungen ein Verhältnis ORL1/µ-Affinität von wenigstens 0,1 auf. Das ORL1/µ-Verhältnis ist definiert als 1/[K_{i(ORL1)}/K_{i(µ)}]. Besonders bevorzugt beträgt das ORL1/µ-Verhältnis wenigstens 0,2 oder wenigstens 0,5, bevorzugter wenigstens 1,0 oder wenigstens 2,0, noch bevorzugter wenigstens 3,0 oder wenigstens 4,0, am bevorzugtesten wenigstens 5,0 oder wenigstens 7,5 und insbesondere wenigstens 10 oder wenigstens 15. In einer bevorzugten Ausführungsform liegt das ORL1/µ-Verhältnis im Bereich von 0,1 bis 30, bevorzugter 0,1 bis 25.

In einer anderen bevorzugten Ausführungsform weisen die erfindungsgemäßen Verbindungen ein Verhältnis ORL1/µ-Affinität von mehr als 30, bevorzugter wenigstens 50, noch bevorzugter wenigstens 100, am bevorzugtesten wenigstens 200 und insbesondere wenigstens 300 auf.

Die erfindungsgemäßen Verbindungen weisen bevorzugt einen Kᵢ-Wert am µ-Opioid-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am µ-Opioid-Rezeptor sind dem Fachmann bekannt. Bevorzugt erfolgt die Bestimmung wie im Zusammenhang mit den Beispielen beschrieben.

Die erfindungsgemäßen Verbindungen weisen bevorzugt einen Kᵢ-Wert am ORL1-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am ORL1-Rezeptor sind dem Fachmann bekannt. Bevorzugt erfolgt die Bestimmung wie im Zusammenhang mit den Beispielen beschrieben.

Überraschenderweise hat sich gezeigt, dass Verbindungen mit Affinität zum ORL1- und µ-Opioid-Rezeptor, bei denen das durch 1/[K_{i(ORL1)}/K_{i(µ)}] definierte Verhältnis von ORL1 zu µ im Bereich von 0,1 bis 30 liegt, vorzugsweise von 0,1 bis 25, ein pharmakologisches Profil aufweisen, das verglichen mit dem anderer Opioidrezeptorliganden deutliche Vorteile aufweist:
1. Die erfindungsgemäßen Verbindungen zeigen eine Wirksamkeit in Akutschmerzmodellen, die mitunter vergleichbar ist mit der gebräuchlicher Stufe-3 Opioide. Gleichzeitig zeichnen sie sich aber durch eine gegenüber klassischen µ-Opioiden deutlich bessere Verträglichkeit aus.
2. Im Gegensatz zu gebräuchlichen Stufe-3 Opioiden zeigen die erfindungsgemäßen Verbindungen eine deutlich höhere Wirksamkeit in Mono- und Polyneuropathieschmerzmodellen, was auf einen Synergismus von ORL1- und µ-Opioid Komponente zurückzuführen ist.
3. Im Gegensatz zu gebräuchlichen Stufe-3 Opioiden zeigen die erfindungsgemäßen Verbindungen in neuropathischen Tieren eine weitgehende, bevorzugt eine vollständige, Trennung von antiallodynischer bzw. antihyperalgetischer Wirkung und antinociceptivem Effekt.
4. Im Gegensatz zu gebräuchlichen Stufe-3 Opioiden zeigen die erfindungsgemäßen Verbindungen in Tiermodellen für chronischen Entzündungsschmerz (u.a. Carrageenan- oder CFA-induzierte Hyperalgesie, viszeraler Entzündungsschmerz) eine deutliche Wirkverstärkung gegenüber dem Akutschmerz.
5. Im Gegensatz zu gebräuchlichen Stufe-3 Opioiden sind µ-opioidtypische Nebenwirkungen (u.a. Atemdepression, opioidinduzierte Hyperalgesie, körperliche Abhängigkeit/Entzug, psychische Abhängigkeit/Sucht) bei den erfindungsgemäßen Verbindungen im therapeutisch wirksamen Dosisbereich deutlich reduziert, bzw. vorzugsweise nicht zu beobachten.

Aufgrund der reduzierten µ-opioiden Nebenwirkungen einerseits und der erhöhten Wirksamkeit bei chronischem, bevorzugt neuropathischem Schmerz andererseits zeichnen sich die gemischten ORL1/µ-Agonisten somit durch deutlich vergrößerte Sicherheitsabstände gegenüber reinen µ-Opioiden aus. Daraus resultiert ein deutlich vergrößertes "therapeutisches Fenster" bei der Behandlung von Schmerzzuständen, bevorzugt chronischen Schmerzen, noch bevorzugter neuropathischen Schmerzen.

Q steht für -C₁₋₈-Aliphat, -Aryl, -Heteroaryl, -C₁₋₈-Aliphat-Aryl, -C(=O)-Heteroaryl oder - C(=NH)-Heteroaryl.

Dabei können -Aryl und -Heteroaryl jeweils unsubstituiert oder ein- oder mehrfach substituiert sein, vorzugsweise mit Substituenten, welche unabhängig voneinander ausgewählt sind aus der Gruppe bestehend aus -C₁₋₈-Aliphat, -OH, -OC₁₋₈-Aliphat, -C₁₋₈-Aliphat-O-C₁₋₈-Aliphat (z.B. -CH₂-O-CH₃), -CF₃, -F, -Cl, -Br, -NO₂, -CN, -Heteroaryl, -C₁₋₈-Aliphat-Aryl und -C₁₋₈-Aliphat-Heteroaryl.

In einer bevorzugten Ausführungsform ist Q ausgewählt aus der Gruppe bestehend aus -C₁₋₃-Alkyl, -Phenyl, -Benzyl, -Pyrrolyl, -Furyl, -Thienyl, Pyridyl, -Indolyl, -Benzofuryl und -Benzothienyl, wobei diese jeweils unsubstituiert oder ein- oder mehrfach substituiert sein können, vorzugsweise mit Substituenten, welche unabhängig voneinander ausgewählt sind aus der Gruppe bestehend aus -C₁₋₈-Aliphat, -OH, -OC₁₋₈-Aliphat, -C₁₋₈-Aliphat-O-C₁₋₈-Aliphat, -CF₃, -F, -Cl, -Br, -NO₂, -CN, -Heteroaryl, -C₁₋₈-Aliphat-Aryl und -C₁₋₈-Aliphat-Heteroaryl (z.B. -Ethyl-4-Pyridyl). Besonders bevorzugt ist Q ausgewählt aus der Gruppe bestehend aus:

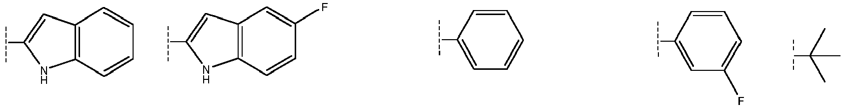

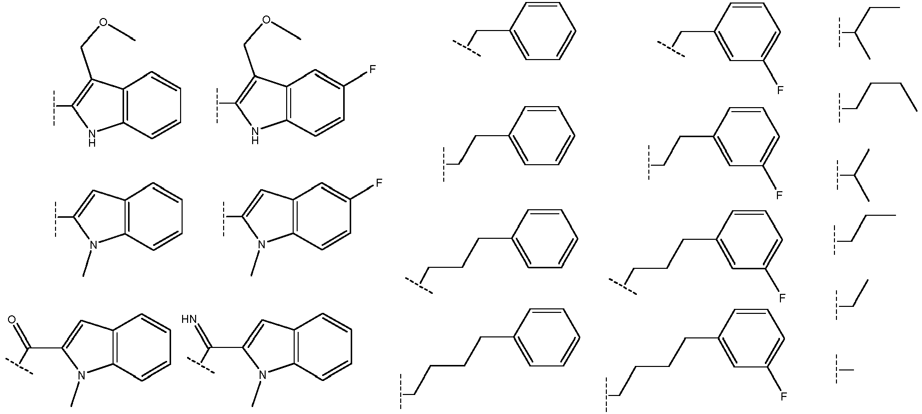

R₀ steht bevorzugt jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl oder -C₁₋₈-Aliphat-Heteroaryl. Dabei bedeuten -C₁₋₈-Aliphat-C₃₋₁₂-Cycloaliphat, -C₁₋₈-Aliphat-Aryl oder -C₁₋₈-Aliphat-Heteroaryl, dass die Reste -C₃₋₁₂-Cycloaliphat, -Aryl oder -Heteroaryl jeweils über eine zweibindige Brücke -C₁₋₈-Aliphat- gebunden sind. Bevorzugte Beispiele für -C₁₋₈-Aliphat-Aryl sind -CH₂-C₆H₅, -CH₂CH₂-C₆H₅, und -CH=CH-C₆H₅.

R₁ und R₂ bilden zusammen mit dem Stickstoffatom, an welches sie gebunden sind, eine der folgenden funktionellen Gruppen:

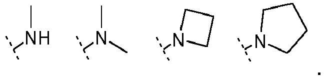

Bevorzugt steht n für 0, 1, 2 oder 3,bevorzugter für 0 oder 1, besonders bevorzugt ist n = 0.

R_{B} steht für -C(=O)-C₃₋₈-Cycloaliphat-Aryl, -C(=O)-C₃₋₃-Cycloaliphat-Heteroaryl, -C(=O)NH-C₁₋₈-Aliphat, -S(=O)₁₋₂-C₁₋₈-Aliphat, -S(=O)₁₋₂-Aryl, -S(=O)₁₋₂-Heteroaryl, -S(=O)₁₋₂-C₁₋₈-Aliphat-Aryl, -S(=O)₁₋₂-C₁₋₈-Aliphat-Heteroaryl, -S(=O)₁₋₂-C₃₋₈-Cycloaliphat-Aryl oder -S(=O)₁₋₂-C₃₋₈-Cycloaliphat-Heteroaryl.

X steht für -O- oder -NR_{A}-, bevorzugt für -NR_{A}-.

Steht X für -NR_{A}-, so steht R_{A} für -H oder -C₁₋₈-Aliphat; bevorzugt für -H oder -CH₃; oder R_{A} bildet gemeinsam mit R_{B} einen Ring und steht für -(CH₂)₃₋₄-.

Bevorzugt steht die Gruppe "R_{B}-X-(CH₂)ₙ-" für

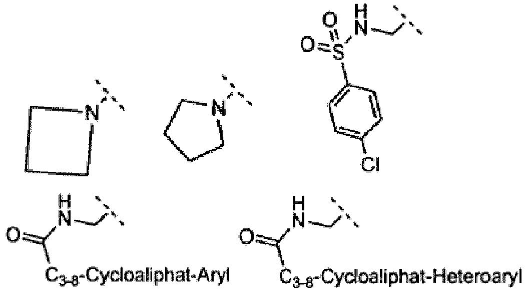

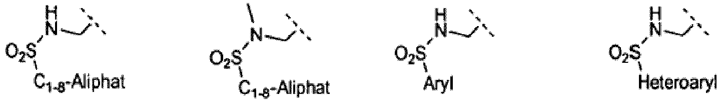

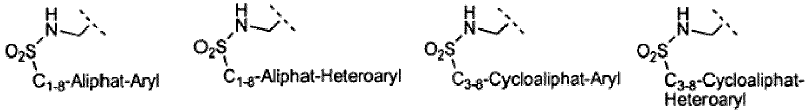

In einer bevorzugten Ausführungsform der erfindungsgemäßen Verbindungen ist R_{A} = R_{B}. In einer anderen bevorzugten Ausführungsform der erfindungsgemäßen Verbindungen ist R_{A} ≠ R_{B}.

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl).

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nicht-aromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist).

Innerhalb der cyclischen Substituenten gilt demnach bevorzugt folgende Priorität bei der Zuordnung: Heteroaryl > Aryl > Cycloaliphat.

Zum Zwecke der Beschreibung werden einbindige und mehrbindige, z.B. zweibindige Kohlenwasserstoffreste nicht begrifflich unterschieden, d.h. "C₁₋₃-Aliphat" umfasst, je nach Sinnzusammenhang, z.B. sowohl -C₁₋₃-Alkyl, -C₁₋₃-Alkenyl und -C₁₋₃-Alkinyl, als auch z.B. -C₁₋₃-Alkylen-, -C₁₋₃-Alkenylen- und C₁₋₃-Alkinylen-.

Bevorzugt ist Aliphat jeweils ein verzweigter oder unverzweigter, gesättigter oder ein ein- oder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend ausgewählt aus der Gruppe bestehend aus -F, -Cl, -Br, -I, -CN, -NO₂, -CHO, =O, -R₀, -C(=O)R₀, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂. So umfasst "Aliphat" acyclische gesättigte oder ungesättigte Kohlenwasserstoffreste, die verzweigt oder geradkettig sein können, d.h. Alkanyle, Alkenyle und Alkinyle. Dabei weisen Alkenyle mindestens eine C=C-Doppelbindung und Alkinyle mindestens eine C=C-Dreifachbindung auf. Bevorzugte unsubstituierte einbindige Aliphaten umfassen -CH₃, -CH₂CH₃, -CH₂CH₂CH₃, -CH(CH₃)₂, -CH₂CH₂-CH₂CH₃, -CH(CH₃)CH₂CH₃, -CH₂CH(CH₃)₂, -C(CH₃)₃, -CH₂CH₂CH₂-CH₂CH₃ und -CH₂CH₂-CH₂CH₂CH₂CH₃; aber auch -CH=CH₂, -C=CH, -CH₂CH=CH₂, -CH=CH-CH₃, -CH₂C≡CH, -C≡CCH₃ und -CH=CHCH=CH₂. Bevorzugte unsubstituierte zweibindige Aliphaten umfassen -CH₂-, -CH₂CH₂-, -CH₂CH(CH₃)-, -CH(CH₃)-CH₂-, -CH₂CH₂CH₂-, -CH(CH₃)CH₂CH₂-, -CH₂CH(CH₃)-CH₂-, -CH₂CH₂CH(CH₃)-, -CH-(CH₂CH₃)CH₂- und -CH₂CH₂-CH₂CH₂-; aber auch -CH=CH-, -C≡C-, -CH₂CH=CH-, -CH=CHCH₂-, -CH₂C≡C- und -C≡C-CH₂-. Bevorzugte substituierte einbindige Aliphaten umfassen -CH₂F, -CHF₂, -CF₃, -CH₂CF₃, -CF₂CF₃, -CH₂OH, -CH₂CH₂OH, -CH₂CHOHCH₃, -CH₂OCH₃ und CH₂CH₂OCH₃. Bevorzugte substituierte zweibindige Aliphaten umfassen -CF₂-, -CF₂CF₂-, -CH₂CHOH-, -CHOHCH₂- und -CH₂CHOHCH₂-. Besonders bevorzugt sind Methyl, Ethyl, n-Propyl und n-Butyl.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono- oder multicyclischer Kohlenwasserstoffrest. Die Zahl der Ringkohlenstoffatome liegt vorzugsweise im angegebenen Bereich (d.h. ein "C₃₋₈-"Cycloaliphat weist vorzugsweise 3, 4, 5, 6, 7 oder 8 Ringkohlenstoffatome auf). Zum Zwecke der Beschreibung ist "C₃₋₈-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂. Vorteilhaft ist C₃₋₈-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I, -OH, -OC₁₋₆-Alkyl, -OC(=O)C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH verstanden. Bevorzugt sind Verbindungen, wobei "Aliphat substituiert" oder "Cycloaliphat substituiert" Aliphat oder Cycloaliphat substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von -CF₃ oder -CH₂CF₃, oder an verschiedenen Stellen, wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfasst -OAliphat u.a. auch -OCH₂CH₂O-CH₂CH₂-OH. Bevorzugt ist es, wenn Aliphat oder Cycloaliphat substituiert sind mit -F, -Cl, -Br, -I, -CN, -CH₃, -C₂H₅, -NH₂, -NO₂, -SH, -CF₃, -OH, -OCH₃, -OC₂H₅ oder -N(CH₃)₂. Ganz besonders bevorzugt ist es, wenn Aliphat oder Cycloaliphat substituiert sind mit -OH, -OCH₃ oder -OC₂H₅.

Bevorzugt steht Aryl jeweils unabhängig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein- oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)-NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NH-C(=O)R₀, -NHC(=O)OR₀, -NHC(=O)NH₂, -NHC(=O)NHR₀, -NH-C(=O)N(R₀)₂, -Si(R₀)₃, -PO(OR₀)₂.

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die Substituenten von Aryl und Heteroaryl jeweils unabhängig voneinander ausgewählt aus -F, -Cl, -Br, -I, -CN, -CHO, -CO₂H, -NH₂, -NO₂, -NHR₀, -N(R₀)₂, -N⁺(R₀)₃, -N⁺(R₀)₂O⁻, -SH, -SR₀, -OH, -OR₀, -C(=O)R₀, -CO₂R₀, -C(=O)NH₂, -C(=O)NHR₀, -C(=O)N(R₀)₂, -S(=O)₁₋₂R₀, -S(=O)₂NH₂, -SO₃H, =O oder -R₀. Bevorzugte Substituenten sind -F, -Cl, -Br, -I, -OH, -OC₁₋₆-Alkyl, -O-C(=O)-C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH. Bevorzugt sind Verbindungen, wobei "Aryl substituiert" oder "Heteroaryl substituiert" Aryl oder Heteroaryl substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet. Besonders bevorzugte Substituenten sind -F, -Cl, -OH, -SH, -NH₂ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Die erfindungsgemäßen Verbindungen können je nach Substitutionsmuster chiral oder achiral sein.

Bei den erfindungsgemäßen Verbindungen kann es sich je nach Substitution in Bezug auf den Cyclohexanring um Isomere handeln, bei denen das Substitutionsmuster in 1,4-Position (1-Position: >C(NR₁R)R₃; 4-Position: >CQ((CH₂)ₙXR_{B}) auch mit syn/anti bezeichnet werden kann. "Syn/anti-Isomere" sind eine Untergruppe der Stereoisomere (Konfigurationsisomere).

In einer bevorzugten Ausführungsform beträgt der Diastereomerenüberschuss des syn-Isomers wenigstens 50 %de, bevorzugter wenigstens 75 %de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des anti-Isomers wenigstens 50 %de, bevorzugter wenigstens 75 %de, noch bevorzugter wenigstens 90%de, am bevorzugtesten wenigstens 95%de und insbesondere wenigstens 99%de.

Geeignete Methoden zur Trennung der Isomere (Diastereomere) sind dem Fachmann bekannt. Als Beispiele können Säulenchromatographie, präparative HPLC und Kristallisationsverfahren genannt werden.

Sind die erfindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Acetylsalicylsäure, Hippursäure und/oder Asparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

Nachfolgend werden jeweils bevorzugte Ausführungsformen der erfindungsgemäßen Verbindungen erläutert. Sofern nicht ausdrücklich spezifiziert gelten alle jeweils zuvor erläuterten Definitionen der Substituenten (d.h. z.B. von R₀ bis R₄, Y₁ bis Y₄', Q etc.) und deren jeweilige bevorzugte Ausführungsformen entsprechend und werden daher nicht wiederholt.

Besonders bevorzugte Ausführungsformen der erfindungsgemäßen Verbindungen haben die allgemeine Formel (2.2), (2.4), (2.6), (2.8), (2.10), (2.12), oder (2.14): 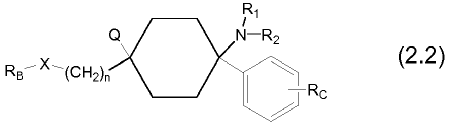 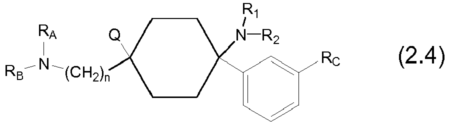 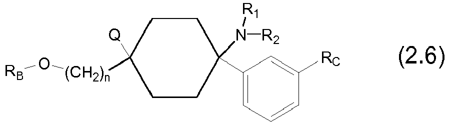 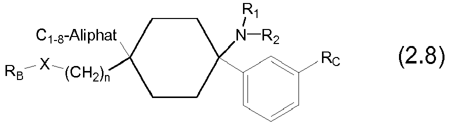 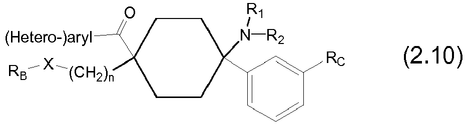 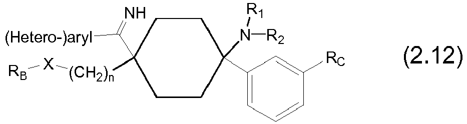 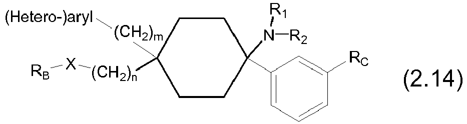 wobei
R_{C} für -H, -F, -Cl, -Br, -I, -CN, -NO₂, -CF₃, -OH oder -OCH₃ steht; und, soweit vorhanden, (Hetero-)aryl für Heteroaryl oder Aryl steht; bevorzugt für -Phenyl, -Benzyl oder -2-Indolyl; jeweils unsubstituiert oder ein- oder mehrfach substituiert, wobei die Substituenten vorzugsweise unabhängig voneinander ausgewählt sind aus der Gruppe bestehend aus -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)-NH-R₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)-OR₀, -NH-C(=O)NH₂, -NHC(=O)NHR₀ und -NHC(=O)N(R₀)₂; bevorzugter -F, -Cl, -Br, -I, -CF₃, -CN und -NO₂; und
m für 0, 1, 2, 3, 4, 5 oder 6 steht.

Bevorzugte Vertreter der Verbindungen der allgemeinen Formel (2.14) sind z.B. die Verbindungen E-10 bis E-12:

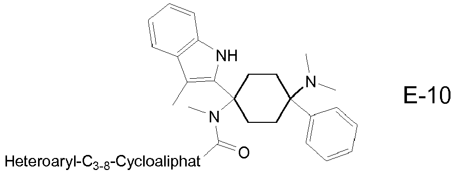

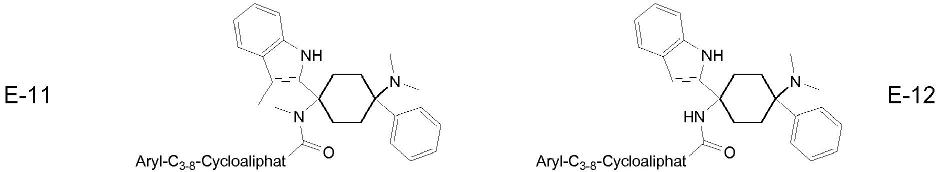

Die erfindungsgemäßen Verbindungen sind durch Substituenten definiert, beispielsweise durch R₁, R₂ und R₃ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise Y₁ = -R₀ wobei R₀ = -C₁₋₈-Aliphat (Substituent der 1. Generation), so kann -C₁₋₈-Aliphat seinerseits substituiert sein, z.B. mit -OR₀ wobei R₀ = -Aryl (Substituent der 2. Generation). Es ergibt sich daraus die funktionelle Gruppe -C₁₋₈-Aliphat-OAryl. -Aryl kann dann seinerseits erneut substituiert sein, z.B. mit -Cl (Substituent der 3. Generation). Es ergibt sich daraus dann insgesamt die funktionelle Gruppe -C₁₋₈-Aliphat-OAryl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für R₀ bis Y₄' jeweils ggf. substituiert sein, die jeweiligen Substituenten können dann ihrerseits jedoch nicht erneut substituiert sein.

In einer anderen bevorzugten Ausführungsform können bereits die Substituenten der 1. Generation nicht erneut substituiert sein, d.h. es gibt dann weder Substituenten der 2. noch Substituenten der 3. Generation. Mit anderen Worten können in dieser Ausführungsform die funktionellen Gruppen für R₀ bis Y₄' jeweils nicht substituiert sein.

Erfindungsgemäß besonders bevorzugte Ausführungsformen der Verbindungen der allgemeinen Formel (1) sind Verbindungen der allgemeinen Formel (2.2) 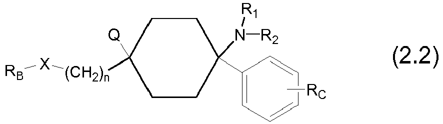 wobei
Q für -C₁₋₈-Aliphat (vorzugsweise -C₁₋₈-Alkyl), -Aryl (vorzugsweise -Phenyl), -C₁₋₈-Aliphat-Aryl (vorzugsweise -C₁₋₈-Alkyl-Phenyl), -Heteroaryl (vorzugsweise -Indolyl), -C(=O)-Heteroaryl (vorzugsweise -C(=O)-Indolyl) oder -C(=NH)-Heteroaryl (vorzugsweise -C(=NH)-Indolyl) steht;
R₁ für -CH₃ steht;
R₂ für -H oder -CH₃ steht; oder
R₁ und R₂ gemeinsam einen Ring bilden und für -(CH₂)₃₋₄- stehen;
X für -O- oder -NR_{A}- steht;
R_{A} für -H oder -C₁₋₈-Aliphat (vorzugsweise -C₁₋₈-Alkyl) steht;
R_{B} für -H, -C₁₋₈-Aliphat (vorzugsweise -C₁₋₈-Alkyl), -C₁₋₈-Aliphat-Aryl (vorzugsweise -C₁₋₈-AlkylPhenyl), -C₁₋₈-Aliphat-Heteroaryl (vorzugsweise -C₁₋₈-Alkyl-Indolyl), -C(=O)-C₁₋₈-Aliphat (vorzugsweise -C(=O)-C₁₋₈-Alkyl), -C(=O)-C₁₋₈-Aliphat-Aryl (vorzugsweise -C(=O)-Benzyl), -C(=O)-C₁₋₈-Aliphat-Heteroaryl (vorzugsweise -C(=O)-C₁₋₈-Alkyl-Indolyl), -C(=O)-C₃₋₈-Cycloaliphat-Aryl (vorzugsweise -C(=O)-Cyclopropyl-Aryl), -C(=O)-C₃₋₈-Cycloaliphat-Heteroaryl (vorzugsweise -C(=O)-Cyclopropyl-Heteroaryl), -C(=O)NH-C₁₋₈-Aliphat (vorzugsweise -C(=O)NH-C₁₋₈-Alkyl), -S(=O)₁₋₂-C₁₋₈-Aliphat (vorzugsweise -S(=O)₂-C₁₋₈-Alkyl), -S(=O)₁₋₂-Aryl (vorzugsweise -S(=O)₂-Phenyl), -S(=O)₁₋₂-Heteroaryl, -S(=O)₁₋₂-C₁₋₈-Aliphat-Aryl, -S(=O)₁₋₂-C₁₋₈-Aliphat-Heteroaryl, -S(=O)₁₋₂-C₃₋₈-Cycloaliphat-Aryl (vorzugsweise -S(=O)₂-Cyclopropyl-Aryl) oder -S(=O)₁₋₂-C₃₋₈-Cycloaliphat-Heteroaryl (vorzugsweise -S(=O)₂-Cyclopropyl-Heteroaryl) steht; oder R_{A} und R_{B} gemeinsam einen Ring bilden und für -(CH₂)₃₋₄- stehen
R_{C} für -H, -F, -Cl, -Br, -I, -CN, -NO₂, -CF₃, -OH oder -OCH₃ steht; und
n für 0, 1, 2, 3 oder 4 steht;
wobei Aliphat, Aryl und Heteroaryl jeweils unsubstituiert oder ein- oder mehrfach substituiert sind.

Ganz besonders bevorzugt sind Verbindungen aus der Gruppe:
- 4-(Dimethylamino)-4-phenyl-1-(pyrrolidin-1-yl)cyclohexyl)(1-methyl-1H-indol-2-yl)methanon;
- 4-(Dimethylamino)-4-phenyl-1-(pyrrolidin-1-yl)cyclohexyl)(1-methyl-1H-indol-2-yl)methanon;
- 4-Chloro-N-((-4-(dimethylamino)-1-methyl-4-phenylcyclohexyl)methyl)benzolsulfonamid 2-hydroxypropan-1,2,3-tricarboxylat;
- N-((1-Butyl-4-(dimethylamino)-4-phenylcyclohexyl)methyl)-4-chlorobenzol-sulfonamid 2-hydroxypropan-1,2,3-tricarboxylat;
- N-(4-Dimethylamino-1,4-diphenyl-cyclohexyl)-N-methyl-methansulfonsäureamid (polares Diastereomer);
- 3-Benzyl-1-(4-dimethylamino-1,4-diphenyl-cyclohexyl)-1-methyl-harnstoff (unpolares Diastereomer);
- 3-Benzyl-1-(4-dimethylamino-1,4-diphenyl-cyclohexyl)-1-methyl-harnstoff (polares Diastereomer);
- 1-(4-Dimethylamino-1,4-diphenyl-cyclohexyl)-3-ethyl-1-methyl-harnstoff (unpolares Diastereomer);
- 1-(4-Dimethylamino-1,4-diphenyl-cyclohexyl)-3-ethyl-1-methyl-harnstoff (polares Diastereomer);
- (E)-N-[[4-Dimethylamino-4-(3-fluorphenyl)-1-methyl-cyclohexyl]-methyl]-2-phenyl-ethensulfonsäureamid (unpolares Diastereomer);
- (E)-N-[[4-Dimethylamino-4-(3-fluorphenyl)-1-methyl-cyclohexyl]-methyl]-2-phenyl-ethensulfonsäureamid (polares Diastereomer);
- Dimethyl-[4-(3-methyl-1H-indol-2-yl)-1-phenyl-4-pyrrolidin-1-yl-cyclohexyl]-amindihydrochlorid (polares Diastereomer);
- Dimethyl-[4-(3-methyl-1H-indol-2-yl)-1-phenyl-4-pyrrolidin-1-yl-cyclohexyl]-amin;
- [4-(Azetidin-1-yl)-4-(3-methyl-1H-indol-2-yl)-1-phenyl-cyclohexyl]-dimethyl-amin (unpolares Diastereomer);
- N-(4-Dimethylamino-1,4-diphenyl-cyclohexyl)-N-methyl-methansulfonsäureamid (unpolares Diastereomer);
- 4-(Azetidin-1-yl)-1-(3-fluorophenyl)-N,N-dimethyl-4-(3-methyl-1H-indol-2-yl)cyclohexanamin (unpolares Diastereomer);
- 4-(Azetidin-1-yl)-1-(3-fluorophenyl)-N,N-dimethyl-4-(3-methyl-1H-indol-2-yl)cyclohexanamin (polares Diastereomer);
- [1-(3-Fluorphenyl)-4-(3-methyl-1H-indol-2-yl)-4-pyrrolidin-1-yl-cyclohexyl]-dimethyl-amin (unpolares Diastereomer);
- [1-(3-Fluorphenyl)-4-(3-methyl-1H-indol-2-yl)-4-pyrrolidin-1-yl-cyclohexyl]-dimethylamin (polares Diastereomer);
- [4-(5-Fluor-3-methyl-1H-indol-2-yl)-1-phenyl-4-pyrrolidin-1-yl-cyclohexyl]-dimethylamin (unpolares Diastereomer);
- [4-(5-Fluor-3-methyl-1H-indol-2-yl)-1-phenyl-4-pyrrolidin-1-yl-cyclohexyl]-dimethylamin (polares Diastereomer);
- [4-(Azetidin-1-yl)-4-(5-fluor-3-methyl-1H-indol-2-yl)-1-(3-fluorphenyl)-cyclohexyl]-dimethyl-amin (unpolares Diastereomer);
- [4-(Azetidin-1-yl)-4-(5-fluor-3-methyl-1H-indol-2-yl)-1-(3-fluorphenyl)-cyclohexyl]-dimethyl-amin (polares Diastereomer);
- (1,4-Diphenyl-4-pyrrolidin-1-yl-cyclohexyl)-methyl-amin (polares Diastereomer);
- (1,4-Diphenyl-4-pyrrolidin-1-yl-cyclohexyl)-methyl-amin (unpolares Diastereomer);
- (1,4-Diphenyl-4-pyrrolidin-1-yl-cyclohexyl)-dimethyl-amin (polares Diastereomer);
- (1,4-Diphenyl-4-pyrrolidin-1-yl-cyclohexyl)-dimethyl-amin (unpolares Diastereomer);
und deren physiologisch verträgliche Salze.

Die erfindungsgemäßen Verbindungen wirken beispielsweise auf den im Zusammenhang mit verschiedenen Erkrankungen relevanten ORL1-Rezeptor, sodass sie sich als pharmazeutischer Wirkstoff in einem Arzneimittel eignen.

Ein weiterer Gegenstand der Erfindung betrifft daher Arzneimittel, welche wenigstens eine erfindungsgemäße Verbindung enthalten, sowie ggf. geeignete Zusatz- und/oder Hilfsstoffe und/oder ggf. weitere Wirkstoffe.

Die erfindungsgemäßen Verbindungen weisen eine vergleichbare Affinität zum ORL1-Rezeptor auf wie die Verbindungen gemäß WO 03/008370. Im Vergleich zu diesen Verbindungen zeigen sie jedoch eine höhere Selektivität gegenüber dem kappa-Opioid-Rezeptor, welcher für Nebenwirkungen, wie beispielsweise Dysphorie, Sedation und Diurese verantwortlich ist. Darüber hinaus zeigen die erfindungsgemäßen Verbindungen bei günstigem Verhältnis der ORL1/µ-Affinität eine ausgewogene Affinität an den µ-Opioid-Rezeptor, welche nicht zu strak ist. Dies ist von Vorteil, da der µ-Opioid-Rezeptor mit Nebenwirkungen in Verbindung gebracht wird, insbesondere Atemdepression, Obstipation und Suchtabhängigkeit. Daher eignen sich die erfindungsgemäßen Verbindungen besonders für die Arzneimittelentwicklung.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt eine enthaltene erfindungsgemäße Verbindung als reines Diastereomer und/oder Enantiomer vor.

Der ORL1-Rezeptor wurde insbesondere im Schmerzgeschehen identifiziert. Entsprechend können erfindungsgemäße Verbindungen zur Herstellung eines Arzneimittels zur Behandlung von Schmerz, insbesondere von akutem, neuropathischem oder chronischem Schmerz, verwendet werden.

Ein weiterer Gegenstand der Erfindung betrifft daher die Verwendung einer erfindungsgemäßen Verbindung zur Herstellung eines Arzneimittels zur Behandlung von Schmerz, insbesondere von akutem, viszeralem, neuropathischem oder chronischem Schmerz.

Ein weiterer Gegenstand der Erfindung betrifft die Verwendung einer erfindungsgemäßen Verbindung zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn eine verwendete Verbindung als reines Diastereomer und/oder Enantiomer, als Razemat oder als nicht-äquimolare oder äquimolare Mischung der Diastereomere und/oder Enantiomere vorliegt. Offenbart wird ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis einer erfindungsgemäßen Verbindung, oder eines erfindungsgemäßen Arzneimittels. Beschrieben wird außerdem ein ein Verfahren zur Herstellung der erfindungsgemäßen Verbindungen wie in der folgenden Beschreibung und Beispielen ausgeführt. Im Folgenden steht/stehen, sofern nicht ausdrücklich anders angegeben,
- Y₁, Y₁', Y₂, Y₂', Y₃, Y₃', Y₄ und Y₄' für -H;
- R₁ für -CH₃;
- R₂ für -H oder -CH₃; oder
- R₁ und R₂ bilden gemeinsam einen Ring und stehen für -(CH₂)₃₋₄-;
- R₃ für -Phenyl, einfach oder mehrfach substituiert mit -H, -F, -Cl, -Br, -I, -CN, -NO₂, -CF₃, -OH oder -OCH₃;
- R₄ für -H, -R₀ oder -C(=O)R₀;
- W für -O-, -S-, -NR₁₁-, -CR₁₂=CR₁₃-, -CR₁₂=N- oder -N=CR₁₃-; bevorzugt für -O-, -S-, oder -NR₁₁-; besonders bevorzugt für -NR₁₁-;
- R₅, R₆, R₁₁, R₁₂ und R₁₃ jeweils unabhängig voneinander für -H, -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂; oder R₅ und R₆ bilden zusammen einen fünf- oder sechsgliedrigen, gesättigten, partiell ungesättigten oder aromatischen, unsubstituierten oder ein- oder mehrfach substituierten Ring, welcher ggf. ein oder zwei Heteroringatome umfasst unabhängig voneinander ausgewählt aus N, S und O;
- A₁ für -N= oder -CR₇=,
- A₂ für -N= oder -CR₈=,
- A₃ für -N= oder -CR₉=,
- A₄ für -N= oder -CR₁₀=;
   mit der Maßgabe, dass höchstens zwei der Reste A₁, A₂, A₃ und A₄, vorzugsweise 0, 1 oder 2 der Reste A₁, A₂, A₃ und A₄, für -N= stehen;
- R₇, R₈, R₉ und R₁₀ jeweils unabhängig voneinander für -H, -F, -Cl, -Br, -I, -NO₂, -CF₃, -OR₁₄, -SR₁₄, -SO₂R₁₄, -CN, -COOR₁₄, -CONR₁₄, -NR₁₅R₁₆, =O oder -R₀; vorzugsweise für -F, -Cl, -Br, -I, -CF₃, -CN oder -NO₂;
- R₁₄ jeweils unabhängig für -H oder -R₀;
- R₁₅ und R₁₆ unabhängig voneinander für -H oder -R₀; oder R₁₅ und R₁₆ stehen zusammen für -CH₂CH₂OCH₂CH₂-, -CH₂CH₂NR₄CH₂CH₂- oder -(CH₂)₃₋₆-;
- R₀ für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl.

### a) Synthese der Cyclohexan-1,4-diamine

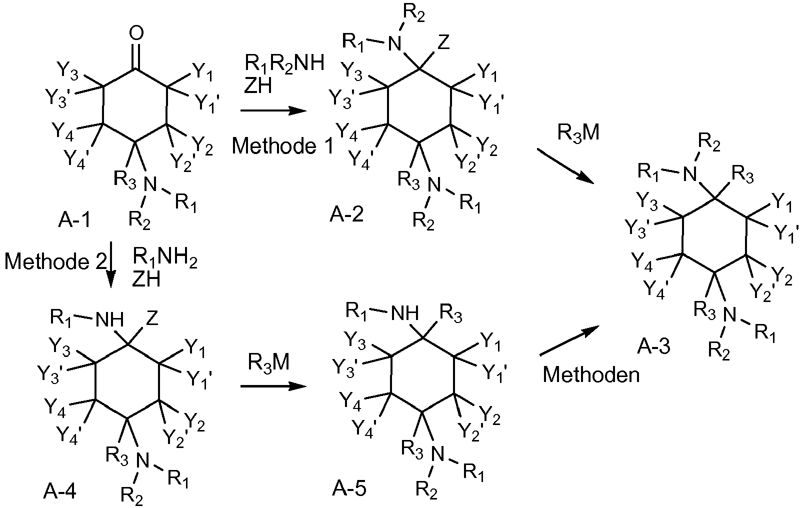

### Methode 1:

Strukturen der Formel A-2 lassen sich durch Reaktion von Ketonen A-1 mit Aminen und aciden Reaktanden Z-H herstellen. Geeignete Reaktanden Z-H sind z. B. Cyanwasserstoff, 1,2,3-Triazol, Benzotriazol oder Pyrazol.

Ein besonders bevorzugter Weg zu Verbindungen der Struktur A-2 ist die Umsetzung von Ketonen mit Metallcyaniden und dem entsprechenden Amin in Gegenwart von Säure, vorzugsweise in einem Alkohol, bei Temperaturen von - 40 bis 60 °C, vorzugsweise bei Raumtemperatur mit Alkalimetallcyaniden in Methanol.

Ein weiterer besonders bevorzugter Weg zu Verbindungen der Struktur A-2 ist die Umsetzung von Ketonen mit 1,2,3-Triazol und dem entsprechenden Amin in Gegenwart unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich A-2 analoge Strukturen mit Benzotriazol- oder Pyrazol- statt Triazolgruppen einführen.

Allgemein können Cyclohexan-1,4-diamine A-3 auch durch Substitution von geeigneten Abgangsgruppen Z in Strukturen der Formel A-2 erhalten werden. Geeignete Abgangsgruppen sind bevorzugt Cyanogruppen; 1,2,3-Triazol-1-yl-Gruppen. Weitere geeignete Abgangsgruppen sind 1*H*-Benzo[d][1,2,3]triazol-1-yl-Gruppen und Pyrazol-1-yl-Gruppen (Katritzky et al., Synthesis 1989, 66-69).

Ein besonders bevorzugter Weg zu Verbindungen der Struktur A-3 ist die Umsetzung von Aminonitrilen A-2 mit entsprechenden Organometallverbindungen, vorzugsweise Grignardverbindungen, vorzugsweise in Ethern, vorzugsweise bei RT. Die Organometallverbindungen sind entweder käuflich erhältlich oder können nach bekannten Methoden hergestellt werden. Ein weiterer besonders bevorzugter Weg zu Verbindungen der Struktur A-3 ist die Umsetzung von Aminotriazolen A-2 mit entsprechenden Organometallverbindungen, vorzugsweise Grignardverbindungen, vorzugsweise in Ethern, vorzugsweise bei RT.

Die Organometallverbindungen sind entweder käuflich erhältlich oder können nach literaturbekannten Methoden hergestellt werden.

### Methode 2:

Strukturen der Formel A-4 lassen sich durch Reaktion von Ketonen A-1 mit primären Aminen und aciden Reaktanden Z-H herstellen. Geeignete Reaktanden Z-H sind z. B. Cyanwasserstoff, 1,2,3-Triazol, Benzotriazol oder Pyrazol.

Ein besonders bevorzugter Weg zu Verbindungen der Struktur A-4 ist die Umsetzung von Ketonen mit Metallcyaniden und dem entsprechenden Amin in Gegenwart von Säure, vorzugsweise in einem Alkohol, bei Temperaturen von - 40 bis 60 °C, vorzugsweise bei Raumtemperatur mit Alkalimetallcyaniden in Methanol.

Ein weiterer besonders bevorzugter Weg zu Verbindungen der Struktur A-4 ist die Umsetzung von Ketonen mit 1,2,3-Triazol und dem entsprechenden Amin in Gegenwart unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich A-4 analoge Strukturen mit Benzotriazol- oder Pyrazol- statt Triazolgruppen einführen.

Allgemein können Cyclohexan-1,4-diamine A-5 auch durch Substitution von geeigneten Abgangsgruppen Z in Strukturen der Formel A-4 erhalten werden. Geeignete Abgangsgruppen sind bevorzugt Cyanogruppen; 1,2,3-Triazol-1-yl-Gruppen. Weitere geeignete Abgangsgruppen sind 1*H*-Benzo[d][1,2,3]triazol-1-yl-Gruppen und Pyrazol-1-yl-Gruppen (Katritzky et al., Synthesis 1989, 66-69).

Ein besonders bevorzugter Weg zu Verbindungen der Struktur A-5 ist die Umsetzung von Aminonitrilen A-4 mit entsprechenden Organometallverbindungen, vorzugsweise Grignardverbindungen, vorzugsweise in Ethern, vorzugsweise bei RT. Die Organometallverbindungen sind entweder käuflich erhältlich oder können nach bekannten Methoden hergestellt werden. Ein weiterer besonders bevorzugter Weg zu Verbindungen der Struktur A-5 ist die Umsetzung von Aminotriazolen A-4 mit entsprechenden Organometallverbindungen, vorzugsweise Grignardverbindungen, vorzugsweise in Ethern, vorzugsweise bei RT.

Die Organometallverbindungen sind entweder käuflich erhältlich oder können nach literaturbekannten Methoden hergestellt werden.

Cyclohexan-1,4-diamine des Typs A-3 lassen sich durch dem Fachmann bekannte Methoden auch aus A-5 synthetisieren. Eine Einführung von (Alkyl-)Substituenten kann dann unter den Bedingungen einer reduktiven Aminierung über eine Aldehydkomponente erfolgen.
Eine solche, dem Fachmann bekannte Methode kann die Umsetzung mit einem Aldehyd unter Zugabe einer Reduktionsmittels, beispielsweise Natriumborhydrid, darstellen.

### b) Synthese der (1,4-Diaminocyclohexyl)(heteroaryl)methanone

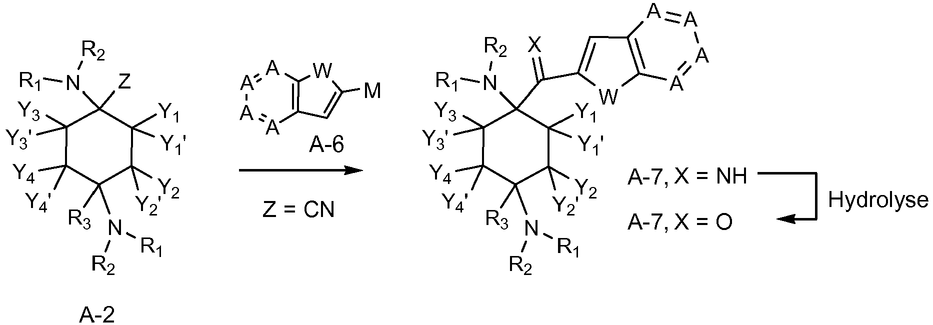

Substituierte (1,4-Diaminocyclohexyl)(heteroaryl)methanone des Typs A-7, X=O lassen sich durch dem Fachmann bekannte Methoden aus den oben beschriebenen Edukt A-2, Z = CN synthetisieren. Durch Umsetzung von metallierten Heterocyclen vom Typ A-6 mit der Dreifachbindung von A-2,Z = CN wird das Intermediat A-7, X = NH erhalten. Eine Hydrolyse unter sauren Bedingungen ergibt dann unter Iminspaltung A-7, X = O.

### c) Synthese der 4-Alkyloxycyclohexan-1-amine

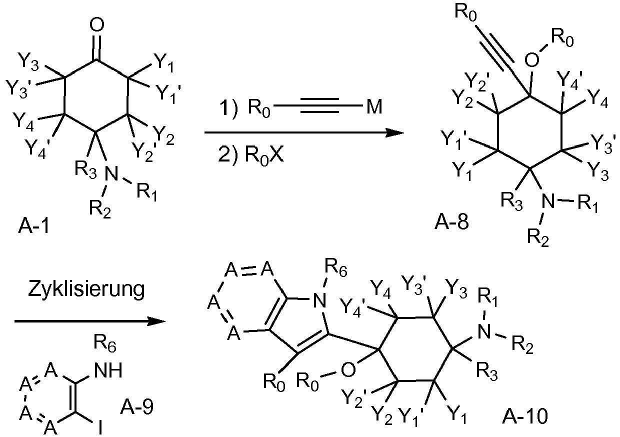

Substituierte 4-Alkyloxycyclohexan-1-amine des Typs A-10 lassen sich durch dem Fachmann bekannte Methoden aus dem Edukt A-1synthetisieren. Das durch die Reaktion von metallierten Alkinen mit A-1 erhaltene Alkoholat wird mit den entsprechenden Elektrophilen z. B. vom Typ R₀X (mit X= z. B. Br, I, OTos, OTf etc.) zu A-8 umgesetzt. Die Umsetzung der Carbinole A-9 zu den erfindungsgemäßen substituierten 4-Alkyloxycyclohexan-1-aminen des Typs A-10 kann in organischen Lösungsmitteln, beispielsweise Tetrahydrofuran, Dimethylformamid, Benzol, Toluol, Xylolen, Dimethoxyethan oder Diethylenglykol-dimethylether, in Gegenwart einer anorganischen Base, beispielsweise Natrium-, Kalium- oder Cäsiumcarbonat oder Kaliumphosphat, in Anwesenheit von PdCl₂, Pd(OAc)₂, PdCl₂(MeCN)₂, PdCl₂(PPh₃)₂ oder [1,3-Bis-(2,6-diisopropylphenyl)imidazol-2-yliden]-(3-chlorpyridyl)palladium(II)-chlorid (PEPPSI®), gegebenenfalls in Anwesenheit von zusätzlichen Liganden, beispielsweise Triphenyl-, Tri-*o*-tolyl-, Tricyclohexyl oder Tri-*t*-butylphosphin, gegebenenfalls in Anwesenheit von Phasentransferkatalysatoren, beispielsweise Tetra-n-butylammoniumchlorid, Tetra-*n-*butylammoniumhydroxid oder Tetra-n-butylammoniumiodid und bei Temperaturen zwischen 60 °C und 180 °C, auch mikrowellenunterstützt, erfolgen.

### d) Synthese der 4-aminomethyl-cyclohexyl-1-amine

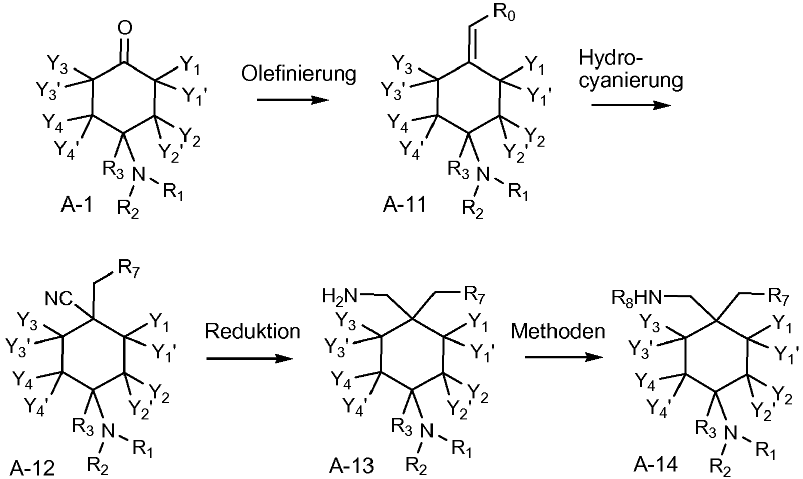

Substituierte 4-Aminomethyl-cyclohexyl-1-amine des Typs A-14 lassen sich durch dem Fachmann bekannte Methoden aus den bekannten Edukten A-1 synthetisieren. Ausgehend von Ketonen wie A-1 erhält man durch eine Wittig-Olefinierung mit Phosphoryliden die intermediären Alkenen A-11. Verbindungen der Formel A-12 können dann aus den entsprechenden Vorläufern A-11 in Gegenwart eines Cobalt-(II)-Salen-Komplex durch eine Hydrocyanierung erhalten werden (Carreira et al. Angew. Chem. Int. Ed., 46, 2006, 4519.). Eine Umsetzung der Nitrilgruppe in A-12 mit einem Reduktionsmittel, beispielsweise einem Hydrid wie Natrium- oder Lithiumborhydrid, Natriumcyanoborhydrid, Natriumtriacetoxyborhydrid, Diisobutylaluminiumhydrid, Lithium-tri-(sec.-butyl)borhydrid (L-Selectride®) oder Lithiumaluminiumhydrid, gegebenenfalls in Anwesenheit von Lewis-Säuren, beispielsweise ZnCl₂, Ni(OAc)₂ oder CoCl₂ ergibt die Amine A-13.

### Methode 1:

Amine des Typs A-13 können nach dem Fachmann bekannten Verfahren zu Verbindungen des A-14 acyliert, sulfonyliert oder carbamoyliert werden. Eine solche, dem Fachmann bekannte Methode kann die Umsetzung mit einem Anhydrid oder einem Säurechlorid unter Zugabe einer Base, beispielsweise Triethylamin, darstellen.

### Methode: 2

Amine des Typs A-13 können nach dem Fachmann bekannten Verfahren zu Verbindungen des Typs A-14 reduktiv aminiert werden. Eine solche, dem Fachmann bekannte Methode kann die Umsetzung mit einem Aldehyd unter Zugabe einer Reduktionsmittels, beispielsweise Natriumborhydrid, darstellen.

### d) Synthese der (4-Aminocyclohexyl)methanole

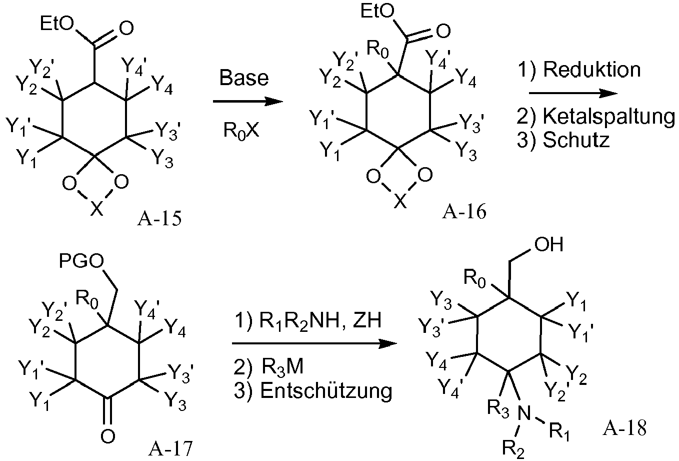

Substituierte (4-Aminocyclohexyl)methanole des Typs A-18 lassen sich durch dem Fachmann bekannte Methoden aus den bekannten Edukten A-15 synthetisieren. In der Literatur wird die Deprotonierung von Estern A-15 mit einer Base, beispielsweise Lithiumdiisopropylamid (LDA) und Umsetzung mit den entsprechenden Elektrophilen z. B. vom Typ R₀-X (mit X= z. B. Br, I, OTos, OTf etc.) zu A-16 beschrieben (Williams et al. J. Org. Chem. 1980, 45, 5082; Shiner et al. J. Am. Chem. Soc. 1981, 103, 436; Xia et al. Org. Lett. 2005, 7, 1315.). Eine Umsetzung von A-16 kann mit einem Reduktionsmittel, beispielsweise einem Hydrid wie Natrium- oder Lithiumborhydrid, Natriumcyanoborhydrid, Natriumtriacetoxyborhydrid, Diisobutylaluminiumhydrid, Lithium-tri-(sec.-butyl)borhydrid (L-Selectride®) oder Lithiumaluminiumhydrid, gegebenenfalls in Anwesenheit von Lewis-Säuren, beispielsweise ZnCl₂, Ni(OAc)₂ oder CoCl₂ und eine Ketalspaltung nach dem Fachmann bekannten Methoden durch Entschützen mittels Säuren erfolgen. Dabei ist X aus der Gruppe Alkyl, Alkyl/ Alkyliden/ mit Aryl oder Alkyl (gesättigt/ungesättigt) substituiertem Alkyliden ausgewählt. Eine Schützung der Hydroxylgruppe nach dem Fachmann bekannten Methoden, beispielsweise durch Umsetzung mit Alkylvinylethern, führt zu den entsprechenden α-Alkyloxyethylethern A-17.

Strukturen der Formel A-18 lassen sich durch Reaktion von Ketonen A-17 mit Aminen und aciden Reaktanden Z-H herstellen. Geeignete Reaktanden Z-H sind z. B. Cyanwasserstoff, 1,2,3-Triazol, Benzotriazol oder Pyrazol.

Ein besonders bevorzugter Weg zu solchen Aminonitrilen ist die Umsetzung von Ketonen mit Metallcyaniden und dem entsprechenden Amin in Gegenwart von Säure, vorzugsweise in einem Alkohol, bei Temperaturen von - 40 bis 60 °C, vorzugsweise bei Raumtemperatur mit Alkalimetallcyaniden in Methanol.

Ein weiterer besonders bevorzugter Weg zu solchen Aminonitrilen ist die Umsetzung von Ketonen mit 1,2,3-Triazol und dem entsprechenden Amin in Gegenwart unter wasserentziehenden Bedingungen, vorzugsweise unter Verwendung eines Wasserabscheiders bei erhöhter Temperatur in einem inerten Löungsmittel oder unter Verwendung von Molsieb oder einem anderen Trockenmittel. Analog lassen sich analoge Strukturen mit Benzotriazol- oder Pyrazol- statt Triazolgruppen einführen.

Die Einführung des Restes R₃ kann durch Substitution von geeigneten Abgangsgruppen Z, wie beispielweise schon für die Umsetzung von A-2 in A-3 beschrieben, erfolgen.

Verbindungen der Formel A-18 können aus entsprechenden Acetalen, oder aus deren Salzen, nach dem Fachmann bekannten Methoden durch Entschützen mittels Säuren freigesetzt werden. Dabei ist PG aus der Gruppe von dem Fachmann bekannten acetalischen Schutzgruppen für Hydroxylgruppen ausgewählt, beispielweise einer α-Alkyloxyethylether-Schutzgruppe.

### e) Synthese der 4-Indolylcyclohexan-1,4-diamine

### Methode 1:

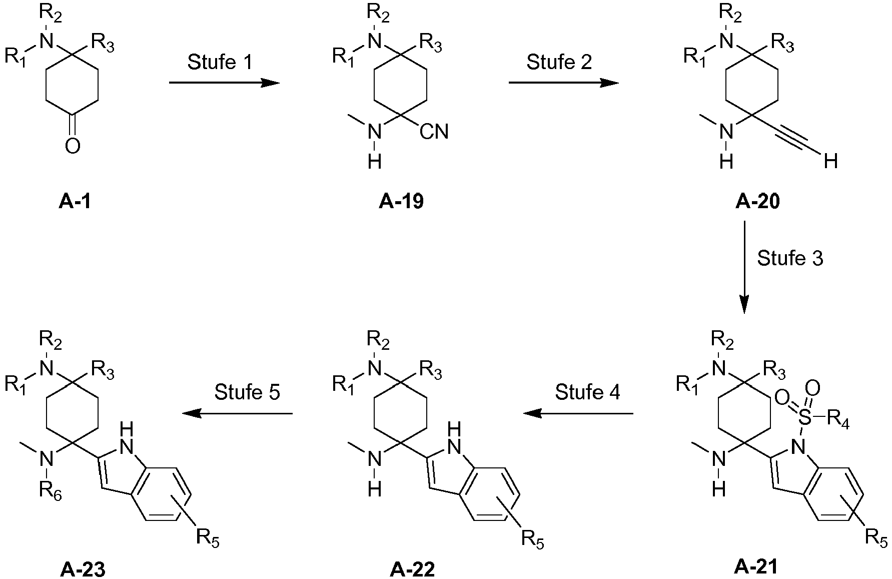

### Stufe 1:

Die Keto-Gruppe lässt sich nach literaturbekannten Methoden, insbesondere unter Verwendung der für die Synthese von A-1 relevanten Literaturstellen, in das Monomethylaminonitril überführen.

### Stufe 2:

Aminonitrile A-19 lassen sich mit entsprechenden Organometallverbindungen, vorzugsweise Organolithium und Grignardverbindungen, vorzugsweise in Ethern, vorzugsweise bei RT in Alkinderivate A-20 überführen. Die Organometallverbindungen sind entweder käuflich erhältlich oder können nach bekannten Methoden hergestellt werden.

### Stufe 3:

Alkinderivate A-20 lassen sich nach F. Messina et al. / Tetrahedron: Asymmetry 11 (2000) 1681-1685 in die geschützten Indolderivate A-21 überführen.

### Stufe 4:

Indolderivate A-21 lassen sich nach literaturbekannten Methoden (vgl. Protective Groups in Organic Synthesis by Peter G. M. Wuts, Theodora W. Greene, WileyBlackwell; 4th Edition) zu Indolderivaten A-22 umsetzen.

### Stufe 5:

Indolderivate A-22 lassen sich durch, dem Fachmann bekannte Methoden in Amide A-23 überführen. Eine solche, dem Fachmann bekannte Methode kann beispielsweise die Umsetzung mit von A-22 mit einer Carbonsäure unter Zugabe eines Kupplungsreagenzes beispielsweise Carbonyldiimidazol sein.

Indolderivate A-22 lassen sich durch, dem Fachmann bekannte Methoden in Sulfonamide A-23 überführen. Eine solche, dem Fachmann bekannte Methode kann beispielsweise die Umsetzung mit von A-22 mit einem Sulfonylchlorid unter Zugabe einer Base, beispielsweise Triethylamin sein.

Indolderivate A-22 lassen sich durch, dem Fachmann bekannte Methoden in Amine A-23 überführen. Eine solche, dem Fachmann bekannte Methode kann beispielsweise die Umsetzung mit von A-22 mit einem Aldehyd unter Zugabe eines Reduktionsmittels, beispielsweise Natriumborhydrid sein.

### Methode 2:

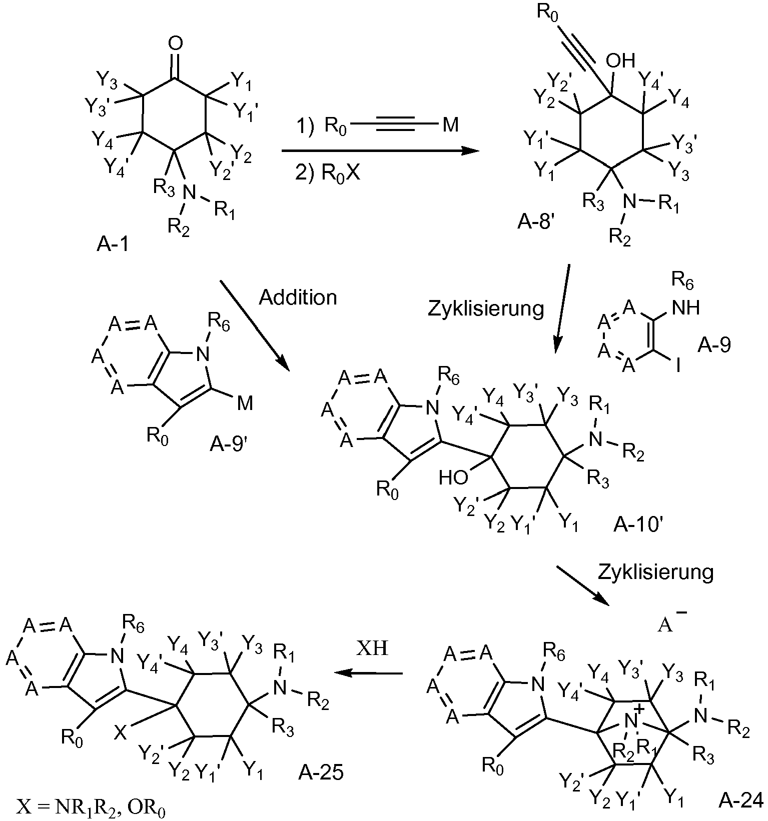

Substituierte Cyclohexanamine des Typs A-25 lassen sich aus dem Edukt A-1 durch die Reaktion mit metallierten Alkinen synthetisieren. Die Umsetzung der Carbinole A-8' und A-9 zu substituierten 4-Alkyloxycyclohexan-1-aminen des Typs A-10' kann in organischen Lösungsmitteln, beispielsweise Tetrahydrofuran, Dimethylformamid, Benzol, Toluol, Xylolen, Dimethoxyethan oder Diethylenglykol-dimethylether, in Gegenwart einer anorganischen Base, beispielsweise Natrium-, Kalium- oder Cäsiumcarbonat oder Kaliumphosphat, in Anwesenheit von PdCl2, Pd(OAc)₂, PdCl₂(MeCN)₂, PdCl₂(PPh₃)₂ oder [1,3-Bis-(2,6-diisopropylphenyl)imidazol-2-yliden]-(3-chlorpyridyl)palladium(II)-chlorid (PEPPSI®), gegebenenfalls in Anwesenheit von zusätzlichen Liganden, beispielsweise Triphenyl-, Tri-o-tolyl-, Tricyclohexyl oder Tri-*t*-butylphosphin, gegebenenfalls in Anwesenheit von Phasentransferkatalysatoren, beispielsweise Tetra-n-butylammoniumchlorid, Tetra-n-butylammoniumhydroxid oder Tetra-n-butylammoniumiodid und bei Temperaturen zwischen 60 °C und 180 °C, auch mikrowellenunterstützt, erfolgen. Alternativ dazu kann auch eine Umsetzung von metallierten Heterocyclen A-9' mit Edukt A-1 in organischen Lösungsmitteln bei Temperaturen zwischen 25 °C und -100 °C zu den Carbinolen des Typs A-10' führen. Die anschließende Zyklisierung von A-10' zum Ammoniumsalz A-24 kann in organischen Lösungsmitteln in Gegenwart von Fluorierungsmitteln bei Temperaturen zwischen 25 °C und -100 °C erfolgen. Die Öffnung des Salzes A-24 zu den erfindungsgemäßen substituierten Cyclohexanaminen des Typs A-25 kann mit geeigneten Nukleophilen ohne oder auch in Gegenwart organischen Lösungsmittel bei Temperaturen zwischen 0°C und 180 °C, auch mikrowellenunterstützt, erfolgen.

### f) Synthese der N-heteroaryl-1,4-diamine

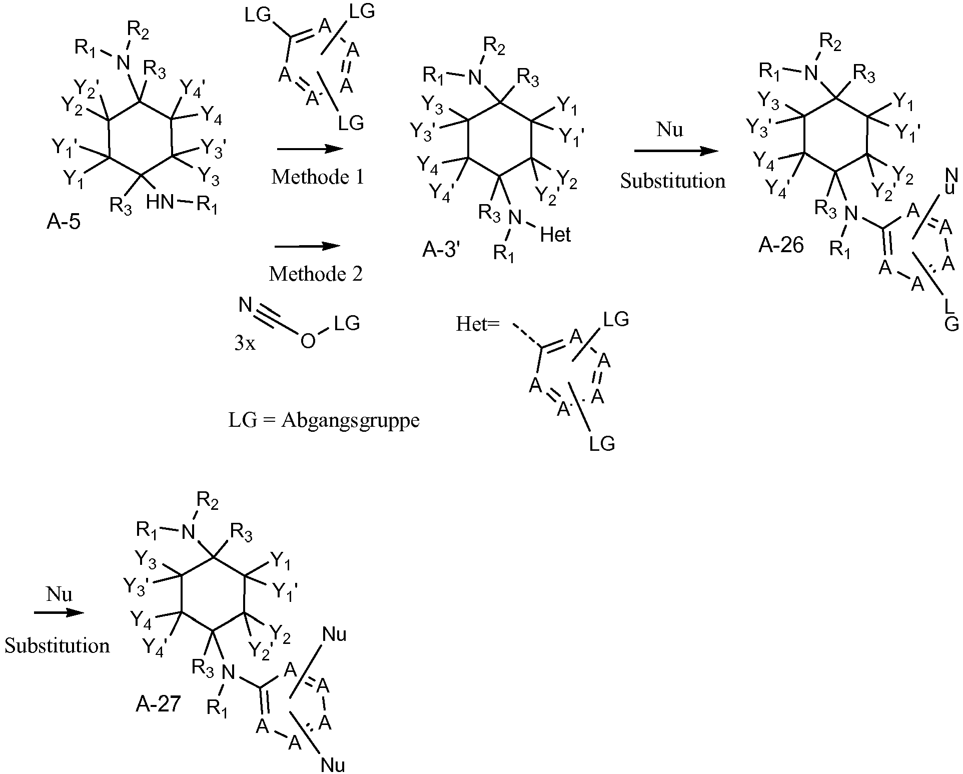

Substituierte Cyclohexanamine des Typs A-26 und A-27 lassen sich aus dem Edukt A-5 synthetisiert. Durch die Reaktion von Heterocyclen (Methode 1) oder Cyanaten (Methode 2) mit geeigneten Abgangsgruppen (z.B. mit LG = Cl oder 4-OMe-C₆H₄) wird das Intermediat A-3' erhalten. Die restlichen Abgangsgruppen können sukzessiv durch geeignete Nukleophile (Nu) ersetzt werden und man erhält die erfindungsgemäßen substituierten Cyclohexanamine des Typs A-26 und A-27.

### g) Vorstufen

Verbindungen der allgemeinen Formeln A-1 und A-15 sind entweder kommerziell erhältlich, oder ihre Herstellung ist aus dem Stand der Technik bekannt oder in für den Fachmann offensichtlicher Weise aus dem Stand der Technik ableitbar. Insbesondere relevant sind hierfür die folgenden Zitate: Jirkovsky et al., J. Heterocycl. Chem., 12, 1975, 937-940; Beck et al., J. Chem. Soc. Perkin 1, 1992, 813-822; Shinada et al., Tetrahedron Lett., 39, 1996, 7099-7102; Garden et al., Tetrahedron, 58, 2002, 8399-8412; Lednicer et al., J. Med. Chem., 23, 1980, 424-430; Bandini et al. J. Org. Chem. 67, 15; 2002, 5386 - 5389; Davis et al., J.Med.Chem. 35, 1, 1992, 177-184; Yamagishi et al., J.Med.Chem. 35, 11, 1992, 2085-2094; Gleave et al.; Bioorg.Med.Chem.Lett. 8, 10, 1998, 1231-1236; Sandmeyer, Helv.Chim.Acta; 2; 1919; 239; Katz et al.; J. Med. Chem. 31, 6, 1988; 1244-1250; Bac et al. Tetrahedron Lett. 1988, 29, 2819; Ma et al. J. Org. Chem. 2001, 66, 4525; Kato et al. J. Fluorine Chem. 99, 1, 1999, 5-8.

Bezüglich weiterer Einzelheiten zur Synthese der erfindungsgemäßen Verbindungen kann vollumfänglich verweisen werden auf WO2002/090317, WO2002/90330, WO2003/008370, WO2003/008371, WO2003/080557, WO2004/043899, WO2004/043900, WO2004/043902, WO2004/043909, WO2004/043949, WO2004/043967, WO2005/063769, WO2005/066183, WO2005/110970, WO2005/110971, WO2005/110973, WO2005/110974, WO2005/110975, WO2005/110976, WO2005/110977, WO2006/018184, WO2006/108565, WO2007/079927, WO2007/079928, WO2007/079930, WO2007/079931, WO2007/124903, WO2008/009415 und WO2008/009416.

### Beispiele

Die folgenden Beispiele dienen zur näheren Erläuterung der Erfindung, sind jedoch nicht einschränkend auszulegen. Beispiele 2, 7, 18, 19, 26, 29, 30, 31, 32, 44, 45, 53, 54, 56, 57, 95, 96, 108, 109, 126, 132, 134, 135, 147, 148, 151 und 152 sind erfindungsgemäße Beispiele, die übrigen Beispiele sind Referenzbeispiele.

Die Ausbeuten der hergestellten Verbindungen sind nicht optimiert. Alle Temperaturen sind unkorrigiert. Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei),"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt. Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Laufmitteln für chromatographische Untersuchungen sind stets in Volumen/Volumen angegeben.

### Beispiel 1:

### 1-(Imino(1-methyl-1H-indol-2-yl)methyl)-N1,N1,N4,N4-tetramethyl-4-phenylcyclohexan-1,4-diamin bis(2-hydroxypropan-1,2,3-tricarboxylat)

### a) 1,4-Bis-dimethylamino-4-phenylcyclohexancarbonitril

Eine Mischung aus Methanol (50 ml) und Wasser (50 ml) wurde mit Salzsäure (37 %, 0,2 ml) angesäuert und unter Eiskühlung und Rühren mit einer wäßrigen Dimethylaminlösung (40 %, 11,5 ml, 91 mmol) versetzt. Anschließend wurde zu der Lösung 4-Dimethylamino-4-phenyl-cyclohexanon (2,17 g, 10 mmol) und KCN (1,6 g, 24,6 mmol) gegeben. Nach 15 min war eine klare Lösung entstanden. Die Eiskühlung wurde entfernt und der Ansatz 2,5 h bei RT gerührt, wobei nach ca. 1 h ein weißer Feststoff auszufallen begann. Zur Vervollständigung der Fällung wurde der Ansatz mittels Eiskühlung für 1 h erneut auf ca. 0°C gebracht. Anschließend wurde der Niederschlag mittels einer Fritte abgetrennt und im Vakuum bei einer Badtemperatur von 40 °C getrocknet. Es wurde ein Diastereoisomerengemisch der Titelverbindung in einer Ausbeute von 1,83 g (67 %) und mit einem Schmelzpunkt von 82-92 °C erhalten.
13C NMR (101 MHz, CDCl3) δ ppm: 29.3*, 30.2, 31.2, 37.7, 38.2, 39.9, 58.4*, 60.2, 62.4*, 118.7, 119.0, 126.8, 127.4, 127.7, 128.0, 136.2*, 137.7*
*verbreiterte Signale

### b) 1-[Imino-(1-methyl-1H-indol-2-yl)methyl]-N,N,N',N'-tetramethyl-4-phenyl-cyclohexan-1,4-diamin [unpolareres Diastereoisomer und polareres Diastereoisomer]

Unter Feuchtigkeitsausschluß wurde bei 0 °C zu einer Lösung von n-Butyllithium (2,5N in n-Hexan, 4 ml, 10 mmol) in trockenem THF (10 ml) N-Methylindol [1,31 g, 10 mmol, gelöst in trockenem THF (10 ml)] gegeben. Der Ansatz wurde 60 min unter Beibehaltung der Kühlung gerührt, wobei nach ca. 10 min ein Feststoff auszufallen begann. Anschließend erfolgte die Zugabe des Diastereoisomerengemisches aus der vorhergehenden Stufe [1,33 g, 5 mmol, gelöst in trockenem THF(10 ml)] innerhalb von 10 min. Nach beendeter Zugabe wurde die Kühlung entfernt und der Ansatz nach Erreichen der RT weitere 18 h gerührt. - Zur Aufarbeitung wurde der Ansatz vorsichtig mit einem Gemisch aus THF (5 ml) und Wasser (1 ml) versetzt. Anschließend wurde zu der Mischung gesättigte NaCl-Lösung (30 ml) gegeben. Die organische Phase wurde abgetrennt, die wäßrige Phase wurde mit Ethylacetat (4 x 20 ml) extrahiert. Die vereinigten organischen Extrakte wurden über MgSO4 getrocknet und anschließend eingeengt. Der erhaltene Rückstand (2,7 g) wurde chromatographisch gereinigt [Kieselgel 60 G (10 g); Ethylacetat (100 ml), Ethylacetat/Ethanol 1 : 1 (100 ml), EtOH 50 ml]. Das unpolarere Diastereoisomer wurde so in einer Ausbeute von 26 % (526 mg), das polarere Diastereoisomer in einer Ausbeute von 32 % (650 mg) erhalten.

### c) 1-(Imino(1-methyl-1H-indol-2-yl)methyl)-N1,N1,N4,N4-tetramethyl-4-phenylcyclohexan-1,4-diamin bis(2-hydroxypropan-1,2,3-tricarboxylat)

Das unpolarere Diastereoisomer (230 mg, 0,61 mmol) wurde in Propan-2-ol (5 ml) in der Siedehitze gelöst und mit einer heißen Lösung von Citronensäure [382 mg, 2 mmol, in Propan-2-ol (4 ml)] versetzt. Beim Abkühlen der Lösung auf RT fiel ein Niederschlag aus. Der Ansatz wurde zur Vervollständigung der Fällung 20 h bei 5 °C belassen, dann wurde der Feststoff mittels einer Fritte abgetrennt und getrocknet. Das Biscitrat wurde so als glasiger Feststoff in einer Ausbeute von 310 mg (64 %) erhalten.
13C NMR (101 MHz, DMSO-D6) δ□ppm: 25.5 (Propan-2-ol), 26.9, 27.4, 30.9, 37.5, 38.1, 43.3, 62.0 (Propan-2-ol?), 62.7, 66.1*, 71.9, 101.3*, 110.1, 119.7, 120.7, 122.1, 126.3, 128.6, 128.8, 132.1*, 137.0, 137.3, 171.2, 173.5, 175.5
*stark verbreiterte Signale

### Beispiel 2:

### (4-Dimethylamino-4-phenyl-1-(pyrrolidin-1-yl)cyclohexyl)-(1-methyl-1H-indol-2-yl)methanon (unpolareres Diastereoisomer)

Während der Synthese der Beispielverbindung 6, Schritt b) fiel auch die analoge unpolare Verbindung als Gemisch an, dieses (680 mg) wurde mit 2N HCl (20 ml) versetzt und 18 h bei RT gerührt. Es fiel ein Feststoff aus. Zur Aufarbeitung wurde die Reaktionsmischung bei Raumtemperatur mit 2N NaOH (30 ml) basisch gestellt. Die wäßrige Phase wurde mit Ethylacetat (3 x 10 ml) extrahiert. Die vereinigten organischen Extrakte wurden über MgSO4 getrocknet und anschließend eingeengt. Versuche zur Umkristallisation des angefallenen Rohproduktes (aus Ethylacetat und DMSO) führten zu keiner Abtrennung der Verunreinigung. Ein Teil des erhaltenen Rohproduktes wurde chromatographisch [Kieselgel 60 G (10 g); Cyclohexan/Ethylacetat 2 : 8 (100 ml)] gereinigt. Die Titelverbindung wurde so mit einem Schmelzpunkt von 212-218 °C in einer Menge von 59 mg isoliert.
13C NMR (101 MHz, CDCl₃) δ ppm: 24.6, 26.4*, 29.9, 32.3, 37.9, 45.6, 59.9* 67.6, 110.1, 110.6, 120.3, 122.7, 124.9, 125.8, 126.5, 127.3, 127.6, 134.4, 138.2*, 138.9, 198.2
*verbreiterte Signale

### Beispiel 3:

### 1-(Imino(1-methyl-1H-indol-2-yl)methyl)-N1,N1,N4,N4-tetramethyl-4-phenylcyclohexan-1,4-diamin bis(2-hydroxypropan-1,2,3-tricarboxylat)(polareres Diastereoisomer)

Das polarere Diastereoisomer aus Beispiel 1, Schritt b) (248 mg, 0,66 mmol) wurde in Propan-2-ol (5 ml) in der Siedehitze gelöst und mit einer heißen Lösung von Citronensäure [382 mg, 2 mmol, in heißem Propan-2-ol (4 ml)] versetzt. Beim Abkühlen der Lösung auf RT fiel ein Niederschlag aus. Der Ansatz wurde zur Vervollständigung der Fällung 20 h bei 5 °C belassen, dann wurde der Feststoff mittels einer Fritte abgetrennt und getrocknet. Das Citrat wurde so in einer Ausbeute von 380 mg (73 %, Schmelzpunkt ab 80 °C) als Biscitrat erhalten.
13C NMR (101 MHz, DMSO-D6) δ ppm: 24.0, 25.4 (Propan-2-ol), 28.1, 31.4, 37.4, 37.5, 43.4, 62.0, 64.4 (Propan-2-ol), 67.8, 71.8, 103.4, 110.2, 119.7, 121.0, 122.4, 126.2, 128.8, 129.2, 129.3, 130.2, 136.2, 137.8, 170.9, 171.2, 175.6

### Beispiel 4:

### (1,4-Bis-dimethylamino-4-phenylcyclohexyl)-(1-methyl-1H-indol-2-yl)-methanon (unpolareres Diastereoisomer)

Die Beispielverbindung 1 (250 mg, 0,62 mmol) wurde mit 2N HCl (10 ml) versetzt und 3 h bei RT sowie 1 h bei 50 °C (Badtemperatur) gerührt. Während der Reaktionszeit fiel ein Niederschlag aus. - Zur Aufarbeitung wurde das Reaktionsgemisch bei Raumtemperatur zunächst mit K₂CO₃ neutralisiert und dann mit 2N NaOH (1 ml) streng basisch gemacht. Die wäßrige Phase wurde mit Ethylacetat (3 x 10 ml) extrahiert. Die vereinigten organischen Extrakte wurden über MgSO4 getrocknet und anschließend eingeengt. Der erhaltene Rückstand (240 mg) wurde chromatographisch gereinigt [Kieselgel 60 G (10 g); Cyclohexan/Ethylacetat 1 : 1, (100 ml)]. Die Titelverbindung wurde so von noch vorhandenem Ausgangsprodukt abgetrennt und in einer Ausbeute von 120 mg (48 %) mit einem Schmelzpunkt von 165-169 °C (nach Umkristallisation aus Ethanol) erhalten.
13C NMR (101 MHz, CDCl3) δ ppm: 24.2, 30.2, 32.3, 37.9, 38.8, 59.0, 69.6, 110.1, 111.5, 120.3, 122.9, 125.0, 125.8, 126.3, 126.8, 127.4, 134.8, 139.0, 139.3, 198.9

### Beispiel 5:

### (1,4-Bis-dimethylamino-4-phenylcyclohexyl)-(1-methyl-1H-indol-2-yl)-methanon (polareres Diastereoisomer)

Die Beispielverbindung 3 (360 mg, 0,9 mmol) wurde mit 2N HCl (10 ml) versetzt und 4 h bei 70 °C (Badtemperatur) gerührt. - Zur Aufarbeitung wurde das Reaktionsgemisch bei Raumtemperatur zunächst mit K₂CO₃ neutralisiert und mit 2N NaOH (1 ml) streng basisch gemacht. Die wäßrige Phase wurde mit Ethylacetat (3 x 10 ml) extrahiert. Die vereinigten organischen Extrakte wurden über MgSO4 getrocknet und anschließend eingeengt. Der erhaltene Rückstand (240 mg) wurde chromatographisch gereinigt [Kieselgel 60 G (10 g); Cyclohexan/Ethylacetat 1 : 1, (150 ml), Ethylacetat (50 ml)]. Die Titelverbindung wurde so in einer Ausbeute von 234 mg (65 %) mit einem Schmelzpunkt von 109-111 °C (nach Umkristallisation aus Propan-2-ol) isoliert.
13C NMR (101 MHz, CDCl3) δ ppm: 25.2, 29.8, 32.5, 38.3, 38.5, 61.8, 69.7, 110.2, 111.7, 120.3, 122.9, 125.1, 125.8, 126.5, 127.8, 127.9, 133.7, 136.8, 139.2, 198.4

### Beispiel 6:

### 4-(Imino(1-methyl-1H-indol-2-yl)methyl)-N,N-dimethyl-1-phenyl-4-(pyrrolidin-1-yl)cyclohexanamin (polareres Diastereoisomer)

### a) 4-Dimethylamino-4-phenyl-1-(pyrrolidin-1-yl)cyclohexancarbonitril

Eine Mischung aus Methanol (50 ml) und Wasser (50 ml) wurde mit Salzsäure (37 %, 0,2 ml) angesäuert und unter Eiskühlung und Rühren mit Pyrrolidin (7,5 ml, 91 mmol) versetzt. Anschließend wurde zu der Lösung 4-Dimethylamino-4-phenylcyclohexanon (2,17 g, 10 mmol) gegeben. Um eine möglichst vollständige Lösung des Ketons zu erreichen, wurde der Ansatz 10 min gerührt. Dann erfolgte die Zugabe von KCN (1,6 g, 24,6 mmol). Die Eiskühlung wurde entfernt und der Ansatz 2 d bei RT gerührt, wobei weißer Feststoff ausfiel. Zur Vervollständigung der Fällung wurde der Ansatz 1 h mittels Eiskühlung erneut auf ca. 0 °C gebracht. Anschließend wurde der Niederschlag mittels einer Fritte abgetrennt und im Vakuum bei einer Badtemperatur von 40 °C getrocknet. Es wurde ein Diastereoisomerengemisch der Titelverbindung in einer Ausbeute von 2,7 g (90 %) und mit einem Schmelzpunkt von 136-142 °C erhalten.
AS 09460: 13C NMR (101 MHz, CDCl3) δ □ppm: 23.4, 23.5, 29.1*, 31.4, 32.3*, 37.7, 38.2, 48.0, 48.1, 58.8*, 60.3*, 61.8*. 62.2*, 119.7, 120.0, 126.7, 126.8, 127.4, 127.7, 127.9, 136.4*, 137.5*
*verbreiterte Signale

### b) 4-(Imino(1-methyl-1H-indol-2-yl)methyl)-N,N-dimethyl-1-phenyl-4-(pyrrolidin-1-yl)cyclohexanamin(polareres Diastereoisomer)

Unter Feuchtigkeitsausschluß wurde bei 0 °C zu einer Lösung von n-Butyllithium (2,5N in n-Hexan, 4 ml, 10 mmol) in trockenem THF (10 ml) N-Methylindol [1,31 g, 10 mmol, gelöst in trockenem THF (10 ml)] gegeben. Der Ansatz wurde 60 min unter Beibehaltung der Kühlung gerührt, wobei nach ca. 10 min ein Feststoff auszufallen begann. Anschließend erfolgte die Zugabe des Diastereoisomerengemisches aus der vorhergehenden Stufe [1,49 g, 5 mmol, gelöst in trockenem THF (20 ml)] innerhalb von 20 min. Nach beendeter Zugabe wurde die Kühlung entfernt und der Ansatz nach Erreichen von RT weitere 18 h gerührt. - Zur Aufarbeitung wurde der Ansatz vorsichtig mit einem Gemisch aus THF (5 ml) und Wasser (1 ml) versetzt. Anschließend wurde zu der Mischung gesättigte NaCl-Lösung (30 ml) gegeben. Die organische Phase wurde abgetrennt, die wäßrige Phase wurde mit Ethylacetat (4 x 20 ml) extrahiert. Die vereinigten organischen Extrakte wurden über MgSO4 getrocknet und anschließend eingeengt. Der erhaltene Rückstand (2,78 g) wurde chromatographisch gereinigt [Kieselgel 60 G (10 g); Ethylacetat (200 ml), Ethylacetat/Ethanol 1 : 1 (50 ml)]. Das polarere Diastereoisomer konnte so in einer Ausbeute von 6 % (140 mg) als viskose Masse isoliert werden. Das unpolare Diastereomer wurde als Gemisch erhalten.
13C NMR (101 MHz, CDCl3) □δ□ppm: 24.2, 26.0, 29.8, 31.7, 38.2, 44.9, 61.3, 64.2, 104.9, 109.8, 119.9, 121.4, 122.7, 126.5, 127.0, 127.6, 127.7, 137.0, 137.3, 138.3, 175.2

### Beispiel 7:

### (4-Dimethylamino-4-phenyl-1-(pyrrolidin-1-yl)cyclohexyl)-(1-methyl-1H-indol-2-yl)methanon (polareres Diastereoisomer)

Die Beispielverbindung 6, Schritt b) (99 mg, 0,23 mmol) wurde mit 2N HCl (3 ml) versetzt und 18 h bei RT gerührt. Sofort nach Zugabe der Säure setzte eine Orangefärbung der Lösung ein. - Zur Aufarbeitung wurde das Reaktionsgemisch bei Raumtemperatur mit 2N NaOH (5 ml) basisch gemacht. Die wäßrige Phase wurde mit Dichlormethan (3 x 10 ml) extrahiert. Die vereinigten organischen Extrakte wurden über MgSO4 getrocknet und anschließend eingeengt. Der erhaltene Rückstand (84 mg) wurde chromatographisch gereinigt [Kieselgel 60 G (10 g); Ethylacetat (120 ml)]. Die Titelverbindung wurde so in einer Ausbeute von 68 mg (68 %) mit einem Schmelzpunkt ab 134 °C isoliert.
13C NMR (101 MHz, CDCl3) δ ppm: 24.1, 26.3*, 29.9, 32.4, 38.3, 45.3, 61.6*, 67.9, 110.2, 111.1, 120.3, 122.9, 125.0, 125.9, 126.5, 127.8, 127.9, 134.1, 136.9*, 139.1, 198.1
*verbreiterte Signale

### Beispiel 8:

### N,N,N'-Trimethyl-1,4-diphenyl-cyclohexan-1,4-diamin (unpolares Diastereomer)

### a) 4-Dimethylamino-1-methylamino-4-phenyl-cyclohexancarbonitril

Zu einer auf 0 °C gekühlten Lösung von 4N Salzsäure (3.75 mL) und Methanol (2.25 mL) wurde 40 %-ige wässrige Methylaminlösung (8.7 mL, 69 mmol) und 4-Dimethylamino-4-phenylcyclohexanon (3.13 g, 14.4 mmol), gelöst in Methanol (15 mL), gegeben. Anschließend wurde die Reaktionsmischung mit Kaliumcyanid (2.25 g, 34 mmol) versetzt und 5 d bei RT gerührt. Zur Aufarbeitung wurde das Gemisch mit Wasser (60 mL) versetzt und mit Ether (3x 50 mL) extrahiert. Die vereinigten organischen Phasen wurden mit Natriumsulfat getrocknet und i. Vak. eingeengt.
Ausbeute: 3.48 g (94 %), Diastereomerengemisch
1H-NMR (DMSO-d6): 1.31 (1 H, m); 1.64 (1 H, m); 1.79 (2 H, m); 1.93 (6 H, d); 2.03 (2 H, m); 2.22 und 2.34 (3 H, dd); 2.77 (1 H, m); 2.63 und 2.77 (1 H, m); 7.33 (5 H, m).

### b) N,N,N'-Trimethyl-1,4-diphenyl-cyclohexan-1,4-diamin (unpolares Diastereomer)

Phenyllithium (8.4 mL, 15 mmol, 1.8 M Lösung in Dibutylether) wurde unter Argon vorgelegt und bei RT tropfenweise mit einer Lösung des Diastereoisomerengemisches aus der vorhergehenden Stufe (1.29 g, 5 mmol) in Diethylether (15 mL) versetzt. Dabei erwärmte sich die Reaktionslösung auf 35 °C und ein Feststoff fiel aus. Das Reaktionsgemisch wurde 30 min unter Rückfluss (Bad 50 °C) gekocht, anschließend im Eisbad (0-10 °C) mit 20 %-iger NH4CI-Lösung (10 mL) hydrolysiert und die organische Phase abgetrennt. Die wässrige Phase wurde mit Ether (2x 30 mL) extrahiert. Die vereinigten organischen Lösungen wurden über Na₂SO₄ getrocknet und i. Vak. eingeengt.
Der Rückstand wurde durch Flash-Chromatographie (50 g Kieselgel) mit Chloroform/Methanol (20:1□9:1□1:1 + 1 % TEA) getrennt.
Ausbeute: 283 mg (18 %) unpolares Diastereomer, Öl
1H-NMR (DMSO-d6): 1.64 (2 H, m); 1.86 (3 H, s); 1.92 (6 H, s); 2.09 (6 H, m); 7.25 (2 H, m); 7.35 (6 H, m); 7.49 (2 H, m).

### Beispiel 9:

### N,N,N'-Trimethyl-1,4-diphenyl-cyclohexan-1,4-diamin (polares Diastereomer)

Bei der Aufreinigung der Beispielverbindung 8 Schritt b) konnte auch das analoge polare Diastereomer isoliert werden.
Ausbeute: 306 mg (20 %) polares Diastereomer.
1H-NMR (DMSO-d6): 1.47 (2 H, m); 1.87 (5 H, m); 1.95 (6 H, s); 2.13 (4 H, m); 7.10 (1 H, m); 7.23 (5 H, m); 7.34 (4 H, m).

### Beispiel 10:

### N,N,N',N'-Tetramethyl-1,4-diphenyl-cyclohexan-1,4-diamin (unpolares Diastereomer)

Eine Lösung der Beispielverbindung 8 (242 mg, 0.78 mmol) und Formalin (1.1 mL, 37 %-ige wässrige Lösung) in Acetonitril (10 mL) wurde portionsweise mit Natriumcyanoborhydrid (200 mg, 3.2 mmol) versetzt und 45 min bei RT gerührt. Anschließend wurde konz. Essigsäure bis zur neutralen Reaktion zugegeben und 45 min bei RT gerührt.
Zur Aufarbeitung wurde das Lösungsmittel i. Vak. entfernt, der Rückstand in 2N NaOH (10 mL) aufgenommen und anschließend mit Ether (3x 10 mL) extrahiert. Die organische Lösung wurde über Na₂SO₄ getrocknet und i. Vak. eingeengt. Der verbliebene Rückstand wurde durch Flash-Chromatographie mit CHCl₃/Me0H (1:1) gereinigt.
Ausbeute: 230 mg (92 %)
Schmelzpunkt: 117-118 °C
1H-NMR (DMSO-d6): 1.76 (4 H, breit); 1.96 (12 H, s); 2.28 (4 H, breit); 7.15 (2 H, m); 7.27 (8 H, m).

### Beispiel 11:

### N,N,N',N'-Tetramethyl-1,4-diphenyl-cyclohexan-1,4-diamin (polares Diastereomer)

Eine Lösung der Beispielverbindung 9, Schritt b) (223 mg, 0.72 mmol) und Formalin (1.0 mL, 37 %-ige wässrige Lösung) in Acetonitril (10 mL) wurde portionsweise mit Natriumcyanoborhydrid (182 mg, 2.9 mmol) versetzt und 45 min bei RT gerührt. Anschließend wurde konz. Essigsäure bis zur neutralen Reaktion addiert und 45 min bei RT gerührt.
Zur Aufarbeitung wurde das Lösungsmittel i. Vak. entfernt, der Rückstand in 2N NaOH (10 mL) aufgenommen und anschließend mit Ether (3x 10 mL) extrahiert. Die organische Lösung wurde über Na₂SO₄ getrocknet und i. Vak. eingeengt. Der verbliebene Rückstand wurde durch Flash-Chromatographie mit CHCl₃/MeOH (9:1) gereinigt.
Ausbeute: 160 mg (69 %)
Schmelzpunkt: 197-198 °C
1H-NMR (CD3OD): 1.47 (4 H, d); 1.91 (12 H, s); 2.75 (4 H, d); 7.32 (2 H, m); 7.46 (8 H, m).

### Beispiel 12:

### 1-Benzyl-N,N,N',N'-tetramethyl-4-phenyl-cyclohexan-1,4-diamine (unpolares Diastereomer)

### a) 1,4-Bis-dimethylamino-4-phenyl-cyclohexancarbonitril

Zu einem Gemisch aus 4N Salzsäure (14 mL) und Methanol (5 mL) wurde unter Eiskühlung 40% wässrige Dimethylamin-Lösung (14 mL, 110.5 mmol), 4-Dimethylamino-4-phenylcyclohexanon (5.00 g, 23.04 mmol) und Kaliumcyanid (3.60 g, 55.3 mmol) hinzugefügt. Die Mischung wurde 2 d bei Raumtemperatur gerührt und anschließend nach der Zugabe von Wasser (200 mL) mit Ether (4 x 150 mL) extrahiert. Nach dem Einengen der Lösung wurde der Rückstand in Dichlormethan (200 mL) aufgenommen und mit Magnesiumsulfat über Nacht getrocknet, filtriert und das Lösungsmittel i. Vak. entfernt. Das Nitril wurde als Öl erhalten, welches durchkristallisierte.
Ausbeute: 5.87 g (90 %)
1H-NMR (DMSO-d6): 1.36 (1H, m); 1.61 (1 H, m); 1.61 (2 H, m); 1.92 (8 H, m); 2.16 (4 H, m); 2.28 (3 H, s); 2.44 (1 H, m); 2.59 (1 H, m); 7.35 (5 H, m).

### b) 1-Benzyl-N,N,N',N'-tetramethyl-4-phenyl-cyclohexan-1,4-diamine (unpolares Diastereomer)

Zur der Titelverbindung der vorhergehenden Stufe (5.84 g, 20.5 mmol) wurde in THF (115 mL) gelöst und unter Eiskühlung tropfenweise mit Benzylmagnesiumchlorid 2M (36 mL, 71.57 mmol) versetzt. Die Reaktionsmischung rührte bei Raumtemperatur über Nacht. Die Reaktionsmischung wurde mit 20%-iger Ammoniumchlorid-Lösung (15 mL) und Wasser (10 mL) versetzt und mit Diethylether (3 x 50 mL) extrahiert. Die vereinten organischen Phasen wurden mit Wasser (50 mL) und ges. NaCl-Lösung (50 mL) gewaschen, über Na₂SO₄ getrocknet, filtiert und i. Vak. eingeengt.
Der Rückstand wurde durch Flash-Chromatographie mit Cyclohexan/Ethylacetat (1:1) gereinigt.
Ausbeute: 770 mg (11 %) unpolares Diastereomer
1H-NMR (DMSO-d6): 1.57 (4 H, m); 1.72 (2 H, m); 1.79 (6 H, s); 2.19 (6 H, s); 2.23 (2 H, m); 2.63 (2 H, s); 7.26 (10 H, m).

### Beispiel 13:

### 1-Benzyl-N,N,N',N'-tetramethyl-4-phenyl-cyclohexan-1,4-diamine (polares Diastereomer)

Bei der Aufreinigung der Beispielverbindung 12 Schritt b) konnte auch das analoge polare Diastereomer isoliert werden.
Der Rückstand wurde durch Flash-Chromatographie mit Cyclohexan/Ethylacetat (1:1) gereinigt. Das unpolare Diastereomer wurde sauber erhalten. Das polare Diastereomer wurde verunreinigt isoliert und nochmals durch Flash-Chromatographie mit Acetonitril/ Methanol/1N NH₄Cl (9:1:1) gereinigt.
Ausbeute: 600 mg (9 %) polares Diastereomer
1H-NMR (DMSO-d6): 0.88 (2 H, t); 1.70 (2 H, m); 1.85 (6 H, s); 1.90 (2 H, m); 2.14 (2 H, m); 2.26 (6 H, s); 2.48 (2 H, s); 7. 00 (6 H, m); 7.18 (4 H, m).
13C-NMR (DMSO-d6): 27.1; 28.6; 36.3; 36.8; 37.8; 57.0; 60.5; 125.2; 125.8; 127.1; 127.2; 130.2; 136.9; 138.7.

### Beispiel 14:

### 4-Methoxy-4-(3-(methoxymethyl)-1H-indol-2-yl)-N,N-dimethyl-1-phenylcyclohexanamin 2-hydroxypropan-1,2,3-tricarboxylat

### a) [4-Methoxy-4-(3-methoxy-prop-1-ynyl)-1-phenyl-cyclohexyl]-dimethyl-amin

Zu einer 2.5 M Lösung von Butyllithium in Hexan (8.4 mL, 21.0 mmol) wurde bei -30 °C unter Argon Methylpropargylether (1.47 g, 21.0 mmol), gelöst in abs. THF (15 mL), getropft. Anschließend wurde bei -30 °C eine Lösung des 4-Dimethylamino-4-phenylcyclohexanon (4.34 g, 20.0 mmol) in abs. THF (20 mL) und Lithiumbromid (0.87 g, 10 mmol), gelöst in abs. THF (2.5 mL), zugegeben. Das Reaktionsgemisch wurde auf -5 °C erwärmt, tropfenweise mit einer Lösung von Methyliodid (4.25 g, 30 mmol) in abs. DMSO (25 mL) versetzt und 2 h bei 50 °C gerührt. Zur Aufarbeitung des Reaktionsgemisches wurde unter Eisbadkühlung Wasser (30 mL) zugegeben und mit Cyclohexan (4 x 50 mL) extrahiert. Die organische Phase wurde mit 20%-iger Ammoniumchloridlösung gewaschen, über Na₂SO₄ getrocknet und i. Vak. eingeengt. Der verbliebene Rückstand wurde durch Flash-Chromatographie mit CHCl3/MeOH (20:1) gereinigt.
Ausbeute: 2.34 g (39 %)
1H-NMR (DMSO-d6): 1.57 (2 H, m); 1.96 (10 H, m); 2.25 (2 H, m); 3.18 (3 H, s); 3.27 (3 H, m); 4.05 (2 H, s); 7.37 (5 H, m).

### b) [4-Methoxy-4-(3-methoxy-prop-1-ynyl)-1-phenyl-cyclohexyl]-dimethyl-amin

2-lodanilin (328 mg, 1.5 mmol), die Titelverbindung der vorhergehenden Stufe (452 mg, 1.5 mmol) und Natriumcarbonat (795 mg, 7.5 mmol) wurden in abs. DMF (10 mL) unter Argon gelöst. Anschließend wurde der Katalysator (PEPPSI®, 204 mg, 0.3 mmol) zugegeben und die Lösung bei 100 °C 24 h gerührt. Zur Aufarbeitung wurde die schwarze Reaktionslösung i. Vak. zur Trockene eingeengt, der Rückstand in CHCl₃ gelöst und mit Wasser gewaschen. Die organische Phase wurde über Na₂SO₄ getrocknet und i. Vak. eingeengt. Der verbliebene Rückstand wurde durch Flash-Chromatographie mit CHCl₃/MeOH (20:1□9:1) gereinigt.
Ausbeute: 71 mg (12 %)
1H-NMR (DMSO-d6): 1.62 (2 H, m); 2.22 (10 H, m); 2.63 (2 H, m); 3.00 (3 H, s); 3.10 (3 H, m); 4.46 (2 H, s);6.95 (2 H, m); 7.28 (1 H, d); 7.46 (6 H; m); 10.72 (1 H, s).

### c) 4-Methoxy-4-(3-(methoxymethyl)-1H-indol-2-yl)-N,N-dimethyl-1-phenylcyclohexanamin 2-hydroxypropan-1,2,3-tricarboxylat

Die Titelverbindung der vorhergehenden Stufe (217 mg, 0.55 mmol) wurde in heißem Ethanol (4 mL) gelöst und mit einer Lösung von Citronensäure (106 mg, 0.55 mmol) in heißem Ethanol (2 mL) versetzt. Nach 2-stündigem Stehen im Kühlschrank und Zugabe von Ether wurde der entstandene Feststoff abgesaugt und i. Vak. getrocknet.
Ausbeute: 165 mg (51 %)
Schmelzpunkt: 184-186 °C
1H-NMR (DMSO-d6): 1.61 (2 H, m); 2.22 (4 H, m); 2.37 (6 H, s); 2.52 (4 H, m); 3.01 (3 H, s); 3.08 (3 H, m); 4.45 (2 H, s); 6.99 (2 H, m); 7.25 (1 H, d); 7.50 (4 H, m); 7.65 (2 H, m); 10.73 (1 H, s).

### Beispiel 15:

### 4-(Benzyloxy)-4-(3-(methoxymethyl)-1H-indol-2-yl)-N,N-dimethyl-1-phenylcyclohexanamin a) [4-Benzyloxy-4-(3-methoxy-prop-1-ynyl)-1-phenyl-cyclohexyl]-dimethyl-amin

Zu einer 2.5 M Lösung von Butyllithium in Hexan (2.1 mL, 5.2 mmol) wurde bei -30 °C unter Argon Methylpropargylether (0.36 g, 5.2 mmol), gelöst in abs. THF (5 mL), getropft. Anschließend wurde bei -30 °C eine Lösung des 4-Dimethylamino-4-phenylcyclohexanons (1.08 g, 5.0 mmol) in abs. THF (5 mL) und Lithiumbromid (0.22 g, 2.5 mmol), gelöst in abs. THF (2.0 mL), zugegeben. Das Reaktionsgemisch wurde auf -5 °C erwärmt, tropfenweise mit einer Lösung von Benzylbromid (1.28 g, 7.5 mmol) in abs. DMSO (10 mL) versetzt und 2 h bei 50 °C gerührt. Zur Aufarbeitung des Reaktionsgemisches wurde unter Eisbadkühlung Wasser (10 mL) zugegeben und mit Cyclohexan (4 x 20 mL) extrahiert. Die organische Phase wurde mit 20%-iger Ammoniumchloridlösung gewaschen, über Na₂SO₄ getrocknet und i. Vak. eingeengt. Der verbliebene Rückstand wurde durch Flash-Chromatographie mit CHCl₃/MeOH (40:1) gereinigt.
Ausbeute: 541 mg (29 %), unpolare Verbindung
1H-NMR (DMSO-d6): 1.67 (2 H, m); 1.94 (6 H, s); 2.04 (4 H, m); 2.30 (2 H, m); 3.19 (3 H, s); 4.09 (2 H, s); 4.60 (2 H, s); 7.31 (10 H, m).

### b) 4-(Benzyloxy)-4-(3-(methoxymethyl)-1 H-indol-2-yl)-N,N-dimethyl-1-phenylcyclohexanamin

2-Acetylamino-iodanilin (359 mg, 1.37 mmol), die Titelverbindung der vorhergehenden Stufe (519 mg, 1.37 mmol) und Natriumcarbonat (726 mg, 6.85 mmol) wurden in abs. DMF (10 mL) unter Argon gelöst. Anschließend wurde der Katalysator (PEPPSI, 190 mg, 0.28 mmol) zugegeben und die Lösung bei 100 °C 24 h gerührt. Zur Aufarbeitung wurde die schwarze Reaktionslösung i. Vak. zur Trockene eingeengt, der Rückstand in CHCl₃ gelöst und mit Wasser gewaschen. Die organische Phase wurde über Na₂SO₄ getrocknet und i. Vak. eingeengt. Der verbliebene Rückstand wurde durch Flash-Chromatographie mit CHCl₃/ MeOH (50:1) gereinigt.
Ausbeute: 210 mg (33 %)
1H-NMR (DMSO-d6): 1.67 (2 H, m); 1.61 (2 H, m); 2.10 (6 H, bs); 2.38 (2 H, m); 2.70 (2 H, m); 3.11 (3 H, s); 4.13 (2 H, s); 4.57 (2 H, s); 7.02 (2 H, m); 7.30 (12 H, m); 10.78 (1 H, s).

### Beispiel 16:

### 4-Ethoxy-4-(3-(methoxymethyl)-1H-indol-2-yl)-N,N-dimethyl-1-phenylcyclohexanamin 2-hydroxypropan-1,2,3-tricarboxylat

### a) [4-Ethoxy-4-(3-methoxy-prop-1-ynyl)-1-phenyl-cyclohexyl]-dimethyl-amin

Zu einer 2.5 M Lösung von Butyllithium in Hexan (8.4 mL, 21.0 mmol) wurde bei -30 °C unter Argon Methylpropargylether (1.47 g, 21.0 mmol), gelöst in abs. THF (15 mL), getropft. Anschließend wurde bei -30 °C eine Lösung des 4-Dimethylamino-4-phenylcyclohexanons (4.34 g, 20.0 mmol) in abs. THF (20 mL) und Lithiumbromid (0.87 g, 10 mmol), gelöst in abs. THF (2.5 mL), zugegeben. Das Reaktionsgemisch wurde auf -5 °C erwärmt, tropfenweise mit einer Lösung von Ethyliodid (4.68 g, 30 mmol) in abs. DMSO (30 mL) versetzt und 2 h bei 50 °C gerührt. Zur Aufarbeitung des Reaktionsgemisches wurde unter Eisbadkühlung Wasser (30 mL) zugegeben und mit Cyclohexan (4 x 50 mL) extrahiert. Die organische Phase wurde mit 20 %-iger Ammoniumchloridlösung gewaschen, über Na₂SO₄ getrocknet und i. Vak. eingeengt. Der verbliebene Rückstand wurde durch Flash-Chromatographie mit CHCl₃/MeOH (20:1) gereinigt.
Ausbeute: 3.92 g (62 %)
1H-NMR (DMSO-d6): 1.12 (3 H, t); 1.58 (2 H, m); 1.96 (10 H, m); 2.25 (2 H, m); 3.17 (3 H, s); 3.51 (2 H, q); 4.04 (2 H, s); 7.37 (5 H, m).

### b) [4-Ethoxy-4-(3-methoxymethyl-1H-indol-2-yl)-1-phenyl-cyclohexyl]-dimethyl-amin

N-(2-lod-phenyl)-acetamid (522 mg, 2.0 mmol), die Titelverbindung der vorhergehenden Stufe (631 mg, 2.0 mmol) und Natriumcarbonat (1.06 g, 10.0 mmol) wurden in abs. DMF (10 mL) unter Argon gelöst. Anschließend wurde der Katalysator (PEPPSI, 272 mg, 0.4 mmol) zugegeben und die Lösung bei 100 °C 24 h gerührt. Zur Aufarbeitung wurde die schwarze Reak-tionslösung i. Vak. zur Trockene eingeengt, der Rückstand in CHCl₃ gelöst und mit Wasser gewaschen. Die organische Phase wurde über Na₂SO₄ getrocknet und i. Vak. eingeengt. Der verbliebene Rückstand wurde durch Flash-Chromatographie mit CHCl₃/MeOH (50:1) gereinigt.
Ausbeute: 249 mg (31 %)
1H-NMR (DMSO-d6): 1.11 (3 H, t); 1.61 (2 H, m); 1.99 (8 H, m); 2.19 (2 H, m); 2.48 (2 H, m); 3.12 (5 H, m); 4.53 (2 H, s); 6.99 (2 H, m); 7.27 (2 H, d); 7.47 (5 H, m); 10.61 (1 H, s).

### c) 4-Ethoxy-4-(3-(methoxymethyl)-1H-indol-2-yl)-N,N-dimethyl-1-phenylcyclohexanamin 2-hydroxypropan-1,2,3-tricarboxylat

Die Titelverbindung der vorhergehenden Stufe (188 mg, 0.462 mmol) wurde in heißem Ethanol (4 mL) gelöst und mit einer Lösung von Citronensäure (89 mg, 0.462 mmol) in heißem Ethanol (2 mL) versetzt. Nach 2-stündigem Stehen im Kühlschrank und Zugabe von Ether wurde der entstandene Feststoff abgesaugt und i. Vak. getrocknet.
Ausbeute: 152 mg (55 %)
Schmelzpunkt: 166-167 °C
1H-NMR (DMSO-d6): 1.12 (3 H, t); 1.57 (2 H, m); 2.17-2.35 (10 H, m); 2.58 (4 H, m); 2.70 (2 H, m); 3.11 (3 H, m); 4.51 (2 H, s); 6.98 (2 H, m); 7.24 (2 H, d); 7.43 (4 H, m); 7.62 (2 H, m); 10.67 (1 H, s).

### Beispiel 17:

### N-((-4-(Dimethylamino)-1-methyl-4-phenylcyclohexyl)methyl)acetamid 2-hydroxypropan-1,2,3-tricarboxylat

### a) Dimethyl-(4-methylen-1-phenyl-cyclohexyl)-amin

Unter Argon wurde tert-BuOK (0.550 g, 4.74 mmol) in abs. Ether (10 mL) vorgelegt und Methyl-triphenylphosphoniumbromid (1.89 g, 4.74 mmol) zugegeben. Anschließend wurde auf 40 °C für 30 min erwärmt. Nach dieser Reaktionszeit wurde 4-Dimethylamino-4-phenyl-cyclohexanons (1.00 g, 4.60 mmol), gelöst in abs. THF (10 mL), vorsichtig zugetropft und die Reaktionslösung auf 50 °C für 5 h erwärmt. Der Reaktionsansatz wurde über Nacht bei Raumtemperatur gerührt und i. Vak. zur Trockene eingeengt. Der Rückstand wurde in Dioxan (50 mL) aufgenommen und mit HCl/Dioxan (5 mL) versetzt. Der ausgefallene Feststoff wurde abgesaugt und mit Ether gewaschen. Das isolierte Hydrochlorid wurde mit 2N NaOH basisch eingestellt und mit Dichlormethan (2 x 80 mL) extrahiert. Die organische Phase wurde über Na₂SO₄ getrocknet, filtriert und i. Vak. eingeengt.
Ausbeute: 0.86 g (61 %)
1H-NMR (DMSO-d6): 1.82 (2 H, m); 1.99 (2 H, m); 2.30 (2 H, m); 2.43 (6 H, d); 3.01 (2 H, m); 4.67 (2 H, s); 7.55 (3 H, m); 7.72 (2 H, m).

### b) 4-Dimethylamino-1-methyl-4-phenyl-cyclohexancarbonitril

R-R-Cobalt-(II)-Salen-Komplex (Jacobsens Ligand, 26.0 mg, 0.04 mmol) wurde in Dichlormethan (5 mL) gelöst, mit Essigsäure (29 µL, 0.08 mmol, 2 eq.) versetzt und 30 min im offenen Kolben gerührt. Anschließend wurde der Ansatz i. Vak. eingeengt und mit Toluol die überschüssige Essigsäure azeotrop entfernt. Der hergestellte Cobalt-(III)-Katalysator wurde in abs. Ethanol (5 mL) unter Argon vorgelegt. Es erfolgte nach 2 min die Zugabe der Titelverbindung der vorhergehenden Stufe (0.860 g, 3.99 mmol) gelöst in Ethanol (8 mL), p-Toluolsulfonylcyanid (714 mg, 5.58 mmol) gefolgt von Phenylsilan (0.49 mL, 3.99 mmol). Anschließend wurde nochmals Ethanol (5 mL) zugegeben und die Reaktionslösung 3 d bei Raumtemperatur gerührt. Der Ansatz wurde i. Vak. bis zur Trockene eingeengt und der Rückstand durch Flash-Chromatographie (2 x an normalem Kieselgel und 1 x an ultrafeinem Kieselgel) mit Essigester gereinigt. Weitere Untersuchungen haben ergeben, dass eine einmalige säulenchromatographische Aufreinigung an ultrafeinem Kieselgel mit Chloroform/Methanol (20:1) ausreicht.
Ausbeute: 0.130 g (13 %)
1H-NMR (DMSO-d6): 1.09 (2 H, m); 1.16 (3 H, s); 1.78 (2 H, m); 1.87 (2 H, m); 1.92 (6 H, s); 2.56 (2 H, m); 7.32 (5 H, m).
13C-NMR (DMSO-d6): 25.4; 29.8; 33.1; 33.7; 37.8; 60.3; 124.5; 126.5; 127.5; 127.7; 135.4.

### c) (4-Aminomethyl-4-methyl-1-phenyl-cyclohexyl)-dimethyl-amin

LiAlH4 (38.0 mg, 0.81 mmol) wurde unter Argon in abs. THF (5 mL) vorgelegt, langsam mit der Titelverbindung der vorhergehenden Stufe (0.130 g, 0.54 mmol), gelöst in abs. THF (5 mL), versetzt und das Reaktionsgemisch 3 h unter Rückfluss gerührt. Anschließend wurde unter Eiskühlung THF (10 mL) und Wasser (4 mL) zugegeben und 30 min nachgerührt. Der Niederschlag wurde über Celite abfiltriert und mit Dichlormethan (50 mL) gewaschen. Das Filtrat wurde i. Vak. zur Trockene eingeengt.
Ausbeute: 0.12 g (90 %)
1H-NMR (DMSO-d6): 0.72 (3 H, s); 0.99 (2 H, m); 1.13 (2 H, t, NH2); 1.50 (2 H, m); 1.84 (2 H, m); 1.90 (6 H, s); 2.04 (2 H, m); 2.39 (2 H, t); 7.24 (5 H, m).

### d) N-(4-Dimethylamino-1-methyl-4-phenyl-cyclohexylmethyl)-acetamid

Die Titelverbindung der vorhergehenden Stufe (0.120 g, 0.48 mmol) wurde in abs. THF (2.5 mL) gelöst und mit Triethylamin (72.0 µL, 0.53 mmol) und Acetylchlorid (42.0 mg, 38.0 µl, 0.53 mmol) versetzt. Das Reaktionsgemisch rührte 16 h bei Raumtemperatur. Der Ansatz wurde i. Vak. zur Trockene eingeengt, der Rückstand in Essigester (10 mL) aufgenommen und mit gesättigter NaHCO₃-Lösung (2 x 10 mL) und mit gesättigter NaCl-Lösung gewaschen. Die organische Phase wurde über Na₂SO₄ getrocknet, filtriert und i. Vak. eingeengt.
Ausbeute: 109 mg (77 %)
1H-NMR (DMSO-d6): 0.71 (3 H, s); 0.96 (2 H, m); 1.17 (2 H, m); 1.47 (2 H, m); 1.84 (3 H, s); 1.91 (6 H, s); 2.11 (2 H, m); 3.02 (2 H, d) 7.30 (5 H, m); 7.69 (1 H, t).

### e) N-((-4-(Dimethylamino)-1-methyl-4-phenylcyclohexyl)methyl)acetamid 2-hydroxypropan-1,2,3-tricarboxylat

Die Titelverbindung der vorhergehenden Stufe (102 mg, 0.35 mmol) wurde in heißem Ethanol (4 mL) gelöst. Citronensäure (67.0 mg, 0.35 mmol) wurde in heißem Ethanol (1.0 mL) gelöst und zugegeben. Der Ansatz wurde bei Raumtemperatur 2 h nachgerührt. Da kein Niederschlag ausfiel wurde die Lösung i. Vak. eingeengt. Der Rückstand wurde mit Ether durchgerührt, erneut i. Vak. eingeengt und anschließend i. Vak. getrocknet. Das gewünschte Citrat wurde als poröser Feststoff erhalten.
Ausbeute: 167 mg (98 %)
1H-NMR (DMSO-d6): 0.62 (3 H, s); 0.92 (2 H, m); 1.45 (2 H, m); 1.83 (3 H, s); 2.07-2.60 (14 H, m); 3.07 (2 H, d) 7.46 (5 H, m); 7.72 (1 H, t).

### Beispiel 18:

### 4-Chloro-N-((-4-(dimethylamino)-1-methyl-4-phenylcyclohexyl)methyl)benzolsulfonamid 2-hydroxypropan-1,2,3-tricarboxylat

### a) 4-Chlor-N-(4-dimethylamino-1-methyl-4-phenyl-cyclohexylmethyl)-benzolsulfonamid

Die Titelverbindung aus Beispiel 17, Schritt c) (0.160 g, 0.65 mmol) wurde in abs. THF (3.4 mL) gelöst, mit Triethylamin (97 µL, 0.714 mmol) und 4-Chlorbenzolsulfonsäurechlorid (151 mg, 0.71 mmol) versetzt und 1 d bei Raumtemperatur gerührt. Der Ansatz wurde i. Vak. zur Trockene eingeengt und der Rückstand durch Flash-Chromatographie: 1. Säule mit Essigester/Ethanol (9:1) und 2. Säule mit Essigester, gereinigt.
Ausbeute: 70 mg (26 %)
1H-NMR (DMSO-d6): sehr schlecht aufgelöstes Spektrum.

### b) 4-Chloro-N-((-4-(dimethylamino)-1-methyl-4-phenylcyclohexyl)methyl)benzolsulfonamid 2-hydroxypropan-1,2,3-tricarboxylat

Die Titelverbindung der vorhergehenden Stufe (0.070 g, 0.17 mmol) wurde in heißem Isopropanol (4 mL) gelöst. Citronensäure (32.0 mg, 0.17 mmol) wurde in heißem Isopropanol (1.0 mL) gelöst und zugegeben. Der Ansatz wurde 2 h bei Raumtemperatur gerührt. Da kein Niederschlag ausfiel wurde die Lösung i. Vak. eingeengt. Der Rückstand wurde mit Ether durchgerührt, erneut i. Vak. eingeengt und anschließend i. Vak. getrocknet. Das gewünschte Citrat wurde als poröser Feststoff erhalten.
Ausbeute: 58 mg (57 %)
1H-NMR (DMSO-d6): 0.64 (3 H, m); 0.90-1.04 (6 H, m); 1.54 (2 H, m); 1.92 (2 H, m); 2.31 (6 H, s); 2.73 (4 H, m); 7.47-7.84 (5 H, m); 10.8 (2 H, breit).

### Beispiel 19:

### N-((1-Butyl-4-(dimethylamino)-4-phenylcyclohexyl)methyl)-4-chlorobenzolsulfonamid 2-hydroxypropan-1,2,3-tricarboxylatt

### a) (4-Butyliden-1-phenyl-cyclohexyl)-dimethyl-amin

Kalium-tert-butylat (2.75 g, 23.7 mmol) wurde unter Argon in abs. Ether (50 mL) vorgelegt und mit Butyl-triphenylphosphoniumbromid (9.45 g, 23.7 mmol) versetzt. Der Ansatz wurde 30 min auf 40 °C erwärmt. Dann wurde das 4-Dimethylamino-4-phenylcyclohexanon (5.00 g, 23.0 mmol), gelöst in abs. THF (50 mL), vorsichtig zugetropft (exotherme Reaktion). Der Ansatz wurde 6.5 h auf 50 °C erwärmt und über Nacht bei Raumtemperatur gerührt. Dann wurde der Ansatz zur Trockene i. Vak. eingeengt, in Dioxan (20 mL) aufgenommen und mit HCl/ Dioxan (5 mL) versetzt. Dabei fiel ein Niederschlag aus. Dieser wurde abfiltriert, mit Ether (10 mL) gewaschen, anschließend mit 2N NaOH basisch gestellt und mit Dichlormethan (2 x 40 mL) extrahiert. Die organische Phase wurde über Na₂SO₄ getrocknet, filtriert und i. Vak. zur Trockene eingeengt.
Ausbeute: 4.60 g (77 %)
1H-NMR (DMSO-d6): 0.83 (3 H, t); 1.27 (2 H, m); 1.94 (9 H, m); 2.09 (5 H, m); 2.29 (2 H, m); 5.04 (1 H, t); 7.23 (1 H, m); 7.36 (4 H, m).

### b) 1-Butyl-4-dimethylamino-4-phenyl-cyclohexancarbonitril

Der Cobalt-III-Katalysator (297 mg, 0.456 mmol) wurde in abs. Ethanol (100 mL) unter Argon vorgelegt. Anschließend wurde die Titelverbindung der vorhergehenden Stufe (11.6 g, 45.3 mmol), gelöst in Ethanol (40 mL), zugegeben und anschließend p-Toluolsulfonylcyanid (13.0 g, 68.0 mmol), Phenylsilan (5.6 mL, 45.3 mmol) und Ethanol (10 mL) addiert. Die Temperatur stieg auf 35 °C, deshalb wurde mit Eiswasser gekühlt. Der Ansatz wurde 72 h bei Raumtemperatur gerührt und anschließend i. Vak. zur Trockene eingeengt. Der Rückstand wurde durch Flash-Chromatographie mit Chloroform/Methanol (20:1) gereinigt. Die vorgereinigte Substanz wurde nochmals durch MPLC-Säulenchromatographie mit Chloroform/Methanol (50:1, 20:1) gereinigt.
Ausbeute: 0.233 g (1.8 %)
1H-NMR (DMSO-d6): 0.83 (3 H, t); 1.08 (2 H, m); 1.27 (6 H, m); 1.75 (2 H, m); 1.93 (8 H, m); 2.63 (2 H, m); 7.36 (5 H, m).

### c) (4-Aminomethyl-4-butyl-1-phenyl-cyclohexyl)-d imethyl-amin

Die Titelverbindung der vorhergehenden Stufe (247 mg, 0.856 mmol) wurde in abs. THF (5 mL) gelöst. Anschließend wurde unter Argon LiAlH4 (64 mg, 1.71 mmol) zugegeben und der Ansatz 5.5 h zum Sieden erhitzt. Zur Aufarbeitung wurde zum Ansatz unter Eiskühlung THF (12 mL) und H2O (5 mL) gegeben und 30 min nachgerührt. Der Ansatz wurde über eine Fritte mit Kieselgur (2 cm) filtriert, mit Dichlormethan (50 mL) und Chloroform (50 mL) nachgespült und i. Vak. eingeengt. Der Rückstand wurde durch Flash-Chromatographie mit Chloroform/Methanol (20:1, 9:1, Methanol) gereinigt.
Ausbeute: 70 mg (28 %)
1H-NMR (DMSO-d6): 0.80 (3 H, t); 1.08 (9 H, m); 1.45 (2 H, m); 1.93 (10 H, m); 2.45 (2 H, m); 7.31 (5 H, m).

### d) N-(1-Butyl-4-dimetaushylamino-4-phenyl-cyclohexylmethyl)-4-chlor-benzolsulfonamid

Die Titelverbindung der vorhergehenden Stufe (65.0 mg, 0.225 mmol) wurde unter Argon in abs. THF (5 mL) gelöst und mit Triethylamin (33.5 µL, 0.247 mmol) versetzt. Anschließend wurde zum Ansatz 4-Chlorbenzolsulfonsäurechlorid (52.0 mg, 0.247 mmol) gegeben. Der Ansatz wurde über Nacht bei Raumtemperatur gerührt. Dann wurde er i. Vak. zur Trockene eingeengt. Der Rückstand wurde in Essigester (10 mL) aufgenommen und mit gesättigter NaHCO3-Lösung (2 x 10 mL) und gesättigter NaCl-Lösung (2 x 10 mL) gewaschen. Die organische Phase wurde über Na2SO4 getrocknet, filtriert und i. Vak. zur Trockene eingeengt.
Ausbeute: 103 mg (98 %)
1H-NMR (DMSO-d6): 0.76 (3 H, t); 0.97 (8 H, m); 1.44 (2 H, m); 1.87 (10 H, m); 2.63 (2 H, m); 7.35 (5H, m); 7.52 (1 H, t); 7.68 (2 H, m); 7.85 (2 H, m).

### e) N-((1-Butyl-4-(dimethylamino)-4-phenylcyclohexyl)methyl)-4-chlorobenzolsulfonamid 2-hydroxypropan-1,2,3-tricarboxylat

Die Titelverbindung der vorhergehenden Stufe (103 mg, 0.22 mmol) wurde in heißem Ethanol (3 mL) gelöst. Die Citronensäure (42 mg, 0.22 mmol) wurde in heißem Ethanol (1 mL) gelöst und zugegeben. Der Ansatz wurde auf Raumtemperatur abgekühlt und dann i. Vak. zur Trockene eingeengt. Ausbeute: 128 mg (88 %)
Schmelzpunkt: poröser Feststoff
1H-NMR (DMSO-d6): 0.86 (3 H, t); 0.94 (6 H, m); 1.10 (2 H, m); 1.50 (2 H, m); 1.86 (2 H, m); 2.28 (6 H, s); 2.51-2.64 (6 H, m); 7.52 (6 H, m); 7.72 (2 H, t); 7.87 (2 H, m).

### Beispiel 20:

### (-4-(Dimethylamino)-4-phenyl-1-(4-phenylbutyl)cyclohexyl)methanol(unpolares Diastereoisomer)

### a) 1,4-Dioxaspiro[4,5]decan-8-carbonsäureethylester

Eine Lösung von Ethyl-4-oxocyclohexancarboxylat (28.9 g, 169 mmol), Ethylengly¬kol (36.7 g, 33.0 mL, 592 mmol) und p-Toluolsulfonsäure (380 mg, 2.0 mmol) in Toluol (90 mL) wurde über Nacht bei Raumtemperatur gerührt. Die Reaktionslösung wurde in Ether gegossen (150 mL) und mit Wasser und 5 % Natriumhydrogencar¬bonat-Lösung (je 150 mL) gewaschen. Die organische Phase wurde mit Natrium¬sulfat getrocknet und i. Vak. eingeengt. Da das Rohprodukt (26.8 g) rein anfiel, konnte es direkt weiter umgesetzt werden.
Ausbeute: 26.8 g (74 %), farbloses Öl

### b) 8-(4-Phenylbutyl)-1,4-dioxaspiro[4,5]decan-8-carbonsäureethylester

Eine 2.5 M Lösung von n-Butyllithium (2.5 g, 15.7 mL, 39.2 mmol) wurde bei -78 °C unter Argon langsam zu einer Lösung von Diisopropylamin (3.96 g, 5.50 mL, 39.2 mmol) in absolutem Tetrahydrofuran (50 mL) getropft. Hierzu wurden nacheinander 1,3-Dimethyl-3,4,5,6-tetrahydro-2(1H)pyrimidon (DMPU, 10.0 g, 9.42 mL, 78.2 mmol) und eine Lösung der Titelverbindung der vorhergehenden Stufe (8.40 g, 39.2 mmol) in absolutem Tetrahydrofuran (30 mL) getropft. Die Reaktionslösung wurde 2 h bei dieser Temperatur weiter gerührt, bevor eine Lösung von 1-Brom-4-phenylbutan (10.0 g, 47.0 mmol) in absolutem Tetrahydrofuran (50 mL) zugetropft wurde. Die resultierende Lösung wurde über Nacht bei Raumtemperatur gerührt. Anschließend wurde gesättigte Ammoniumchlorid-Lösung (50 mL) zugegeben und mit Ether (2 x 50 mL) extrahiert. Die vereinigten organischen Phasen wurden mit gesättigter Natriumchlorid-Lösung (50 mL) gewaschen, mit Natriumsulfat getrocknet und i. Vak. eingeengt. Das Roh¬produkt (17.7 g) wurde mittels Flashchromatographie (400 g, 20 x 7.5 cm) mit Cyclohexan / Ethylacetat (9:1) gereinigt.
Ausbeute: 10.3 g (76 %), farbloses Öl
1H-NMR (DMSO-d6): 1.12 (t, 3H, J = 7.1 Hz); 1.39-1.62 (m, 12H); 1.91-2.03 (m, 2H); 2.54 (t, 2H, J = 7.4 Hz); 3.82 (s, 4H); 4.05 (q, 2H, J = 7.1 Hz); 7.12-7.17 (m, 3H); 7.22-7.28 (m, 2H).

### c) [8-(4-Phenylbutyl)-1,4-dioxaspiro[4.5]dec-8-yl]methanol

Zu einer Aufschlämmung von Lithiumaluminiumhydrid (2.50 g, 67.0 mmol) in abso¬lutem Tetrahydrofuran (100 mL) wurde unter Argon bei 65 °C eine Lösung der Titelverbindung der vorhergehenden Stufe (10.2 g, 33.5 mmol) in absolutem Tetrahydrofuran (50 mL) getropft und 3 h bei dieser Temperatur gerührt, wonach die Umsetzung vollständig war. Die Reaktionsmischung wurde nach dem Abkühlen mit Wasser (4.5 mL) und 4 N Natronlauge (1.1 mL) versetzt und vom entstandenen Niederschlag abfiltriert. Der Rückstand wurde mit Tetrahydrofuran (2 x 60 mL) gewaschen und das Filtrat i. Vak. eingeengt. Da das Produkt rein anfiel, konnte es direkt weiter umgesetzt werden.
Ausbeute: 9.44 g (93 %), hellgelbes Öl
1H-NMR (DMSO-d6): 1.14-1.32 (m, 6H); 1.34-1.39 (m, 2H); 1.40-1.57 (m, 6H); 2.57 (t, 2H, J = 7.4 Hz); 3.17 (d, 2H, J = 5.2 Hz); 3.82 (s, 4H); 4.36 (t, 1H, J = 5.2 Hz); 7.13-7.19 (m, 3H); 7.24-7.29 (m, 2H).

### d) 4-Hydroxymethyl-4-(4-phenylbutyl)cyclohexanon

Eine Lösung der Titelverbindung der vorhergehenden Stufe (9.40 g, 30.9 mmol) in Aceton (150 mL) wurde mit 1 N Salzsäure (32 mL) versetzt und über Nacht bei Raumtemperatur gerührt. Die Reak¬tionslösung wurde mit 1 N Natronlauge auf pH 8 eingestellt und i. Vak. eingeengt. Der Rückstand wurde mit Wasser (50 mL) versetzt und anschließend mit Dichlormethan (3 × 50 mL) extrahiert. Die vereinigten organischen Phasen wurden mit gesättigter Natriumchlorid-Lösung (30 mL) gewaschen, mit Natriumsulfat getrocknet und i. Vak. eingeengt. Da das Produkt rein anfiel, konnte es direkt weiter umgesetzt werden.
Ausbeute: 7.96 g (99 %), hellgelbes Öl
1H-NMR (DMSO-d6): 1.19-1.67 (m, 10H); 2.22 (t, 4H, J = 6.8 Hz); 2.59 (t, 2H, J = 7.5 Hz); 3.30 (d, 2H, J = 5.2 Hz); 4.54 (t, 1H, J = 5.1 Hz); 7.13-7.21 (m, 3H); 7.24-7.29 (m, 2H).

### e) 4-(1-Ethoxy-ethoxymethyl)-4-(4-phenylbutyl)cyclohexanon

Eine Lösung der Titelverbindung der vorhergehenden Stufe (7.95 g, 30.5 mmol) in absolutem Dichlormethan (100 mL) wurde mit Pyridiniumtosylat (100 mg) und Ethylvinylether (2.64 g, 3.51 mL, 36.6 mmol) versetzt und über Nacht bei Raumtemperatur gerührt. Die Reaktionslösung wurde anschließend nacheinander mit 5 % Natriumhydrogencarbonat-Lösung, Wasser (je 2 × 50 mL) und gesättigter Natriumchlorid-Lösung (50 mL) gewaschen, mit Natriumsulfat getrocknet und i. Vak. eingeengt. Das Rohprodukt (8.87 g) wurde mittels Flashchromatographie (400 g, 20 × 7.5 cm) mit Cyclohexan / Ethylacetat (9:1) gereinigt.
Ausbeute: 6.97 g (69 %), farbloses Öl
1H-NMR (DMSO-d6): 1.09 (t, 3H, J = 7.0 Hz); 1.17 (d, 3H, J = 5.3 Hz); 1.21-1.32 (m, 2H); 1.43-1.50 (m, 2H); 1.52-1.70 (m, 6H); 2.20-2.26 (m, 4H); 2.59 (t, 2H, J = 7.5 Hz); 3.24 (d, 1H, J = 9.4 Hz); 3.34-3.42 (m, 2H); 3.49-3.59 (m, 1H); 4.62 (q, 1H, J = 5.3 Hz); 7.14-7.29 (m, 5H).

### f) 1-Dimethylamino-4-(1-ethoxyethoxymethyl)-4-(4-phenylbutyl)cyclohexancarbonitril

Zu einer eisgekühlten Mischung von 4 N Salzsäure (1.52 mL, 6.1 mmol) und Metha¬nol (1.7 mL) wurde zuerst 40 % Dimethylaminlösung (3.74 mL, 24.2 mmol), dann die Titelverbindung der vorhergehenden Stufe (2.04 g, 6.1 mmol) und Kaliumcyanid (953 mg, 14.6 mmol) gegeben. Die entstandene Suspension wurde 4 h bei Raumtemperatur gerührt. Die Suspension wurde mit Wasser (100 mL) versetzt und anschließend mit Diethylether (3 × 100 mL) extrahiert. Die vereinigten organischen Phasen wurden mit Natriumsulfat getrocknet und i. Vak. eingeengt.
Ausbeute: 2.30 g (97 %), hellgelbes Öl
1H-NMR (DMSO-d6): 1.08 (dt, 3H, J = 2.5, 7.0 Hz, 1.075 (t, 1.5 H, J = 7.0 Hz); 1.085 (t, 1.5H, J = 7.0Hz); 1.14-1.18 (m, 3H); 1.19-1.39 (m, 6H); 1.41-1.68 (m, 6H); 1.89-2.00 (m, 2H); 2.22 (s, 2.6H); 2.23 (s, 3.4H); 2.53-2.62 (m, 2H); 3.10 (d, 0.5H, J = 9.3 Hz); 3.13 (d, 0.5H, J = 9.2 Hz); 3.22-3.29 (m, 1H); 3.33-3.39 (m, 1H); 3.47-3.59 (m, 1H); 4.56-4.63 (m, 1H); 7.13-7.29 (m, 5H).

### g) [4-(1-Ethoxyethoxymethyl)-1-phenyl-4-(4-phenylbutyl)cyclohexyl]dimethylamin

Zu einer eisgekühlten 2 M Lösung von Phenylmagnesiumchlorid (2.03 g, 7.4 mL, 14.8 mmol) in Tetrahydrofuran wurde unter Argon langsam eine Lösung der Titelverbindung der vorhergehenden Stufe (Diastereoisomerengemisch, 2.30 g, 5.9 mmol) in absolutem Tetrahydrofuran (30 mL) getropft und anschließend über Nacht bei Raumtemperatur gerührt. Die Reaktionslösung wurde mit gesättigter Ammoniumchlorid-Lösung und Wasser (je 20 mL) versetzt, die Phasen getrennt und die wässrige Phase mit Diethylether (3 × 30 mL) extrahiert. Die vereinigten organischen Phasen wurden mit gesättigter Natriumchlorid-Lösung (30 mL) gewaschen, mit Natriumsulfat getrocknet und i. Vak. eingeengt. Es fielen 2.73 g Rohprodukt als Diastereoisomerengemisch an, welches mittels MPLC (LiChroprep Si60 15-25 µm, 230 g, 3.6 × 46 cm) mit Ethylacetat / Methanol (9:1) vollständig getrennt wurde.
unpolares Diastereoisomer:
Ausbeute: 918 mg (36 %), hellgelbes Öl
1H-NMR (DMSO-d6): 1.00 (t, 3H, J = 7.1 Hz); 1.06 (d, 3H, J = 5.3 Hz); 1.10-1.20 (m, 2H); 1.21-1.29 (m, 2H); 1.32-1.48 (m, 2H); 1.51-1.62 (m, 2H); 1.90 (s, 6H); 1.92-1.99 (m, 4H); 2.59 (t, 2H, J = 7.6 Hz); 2.98 (d, 1H, J = 9.3 Hz); 3.11 (d, 1H, J = 9.3 Hz); 3.21-3.29 (m, 2H); 3.39-3.50 (m, 2H); 4.48 (q, 1H, J = 5.3 Hz); 7.13-7.37 (m, 10H).

### h) (-4-(Dimethylamino)-4-phenyl-1-(4-phenylbutyl)cyclohexyl)methanol(unpolares Diastereoisomer)

Eine Lösung der Titelverbindung (unpolares Diastereoisomer) der vorhergehenden Stufe (469 mg, 1.1 mmol) in Aceton (50 mL) wurde mit 2 N Salzsäure (2 mL) versetzt und 18 h bei Raumtempe¬ratur gerührt. Die Reaktionslösung wurde mit 1 N Natronlauge auf pH 8 eingestellt, i. Vak. eingeengt, der Rückstand in Wasser (50 mL) aufgenommen und mit Dichlor¬methan (3 × 30 mL) extrahiert. Die vereinigten organischen Phasen wurden mit gesättigter Natriumchlorid-Lösung (30 mL) gewaschen, mit Natriumsulfat getrocknet und i. Vak. eingeengt. Das Rohprodukt (381 mg) wurde mittels Flashchroma¬tographie (18 g, 20 × 2.0 cm) mit Ethylacetat / Methanol (4:1) gereinigt.
Ausbeute: 325 mg (81 %), weißer Feststoff
Schmelzpunkt: 105-106 °C
1H-NMR (DMSO-d6): 1.00-1.14 (m, 2H); 1.18-1.28 (m, 2H); 1.29-1.41 (m, 4H); 1.49-1.60 (m, 2H); 1.81-1.85 (m, 1H); 1.90 (s, 6H); 1.94-2.06 (m, 3H); 2.59 (t, 2H, J = 7.7 Hz); 3.02 (d, 2H, J = 5.2 Hz); 4.21 (t, 1H, J = 5.2 Hz); 7.13-7.37 (m, 10H).
13C-NMR (DMSO-d6): 22.4; 28.1; 28.2; 32.2; 34.1; 34.3; 35.9; 36.7; 37.9; 60.1; 66.2; 125.5; 125.7; 126.0; 127.2; 127.3; 128.1; 128.2; 137.4; 142.4.
LC-MS (Methode: ASCA-7MIN-80GRAD.M): m/z: [M+1]+ = 366.6, Rt 2.38 min.

### Beispiel 21:

### (-4-(Dimethylamino)-4-phenyl-1-(4-phenylbutyl)cyclohexyl)methanol (polares Diastereoisomer)

### a) [4-(1-Ethoxyethoxymethyl)-1-phenyl-4-(4-phenylbutyl)cyclohexyl]dimethylamin

Bei dem Syntheseschritt zu Beispiel 20, Schritt g) fiel bei der chromatographischen Trennung auch das polare Diastereoisomer rein an.
polares Diastereoisomer: 700 mg (27 %), hellgelbes Öl
1H-NMR (DMSO-d6): 0.96-1.23 (m, 14H); 1.36-1.57 (m, 4H); 1.90 (s, 6H); 1.92-2.00 (m, 4H); 2.45-2.47 (m, 1H); 3.16-3.21 (m, 1H); 3.35-3.43 (m, 1H); 3.51-3.61 (m, 1H); 4.61 (q, 1H, J = 5.2 Hz); 7.09-7.38 (m, 10H).

### b) (-4-(Dimethylamino)-4-phenyl-1-(4-phenylbutyl)cyclohexyl)methanol(polares Diastereoisomer)

Eine Lösung der Titelverbindung der vorhergehenden Stufe (622 mg, 1.42 mmol) in Tetrahydrofuran (5 mL) wurde mit Eisessig (3.0 mL) und Wasser (1.5 mL) versetzt und zunächst 8 h unter Rückfluss gerührt. Da die Reaktion noch nicht vollständig war, wurde über Nacht bei 50 °C und anschließend nochmals 8 h unter Rückfluss gerührt. Obwohl dann die Umsetzung immer noch nicht ganz vollständig war, wurde die Reaktionslösung i. Vak. eingeengt und der Rückstand mehrfach in Toluol (3 × 10 mL) aufgenommen und jeweils wieder i. Vak. eingeengt. Der Rückstand wurde in 5 % Natriumhydrogencarbonat-Lösung aufgenommen (30 mL) und mit Ethylacetat (3 × 20 mL) extrahiert. Die vereinigten organischen Phasen wurden mit gesättigter Natriumchlorid-Lösung (30 mL) gewaschen, mit Natriumsulfat getrocknet und i. Vak. eingeengt. Da das Rohprodukt noch Ausgangsstoff enthielt, wurde dieser mittels Flashchromatographie (400 g, 20 × 7.5 cm) mit Ethylacetat / Methanol (4:1) abge¬trennt (77 mg). Zunächst wurden nur 245 mg der sehr polaren Zielverbindung erhalten. Durch Waschen der Säule mit Methanol (500 mL) wurde weiteres Produkt (220 mg) isoliert.
Ausbeute: 465 mg (86 %), weißer Feststoff
Schmelzpunkt: 123 °C
1H-NMR (DMSO-d6): 0.90-1.03 (m, 2H); 1.11 (br s, 4H); 1.35-1.53 (m, 4H); 1.92 (br s, 10H); 2.47 (d, 2H, J = 8.2 Hz); 3.25 (d, 2H, J = 4.9 Hz); 4.35 (t, 1H, J = 4.7 Hz); 7.09-7.15 (m, 3H); 7.18-7.24 (m, 3H); 7.28-7.39 (m, 4H).
13C-NMR (DMSO-d6): 22.3; 28.1; 28.2; 35.2; 35.7; 37.6; 38.9; 59.9; 65.3; 125.4; 126.1; 127.4; 128.0; 128.1; 142.3.

### Beispiel 22:

### [4-Benzyl-4-(dimethylaminomethyl)-1-phenyl-cyclohexyl]-dimethyl-amin

### Stufe 1:

### 4-Benzyl-1-(dimethylamino)-4-((dimethylamino)methyl)cyclohexancarbonitril

Zu einem Gemisch aus 4N Salzsäure (3 mL) und Methanol (1.05 mL) wurde unter Eiskühlung 40%-ige wässrige Dimethylamin-Lösung (2.8 mL, 22.1 mmol), 4-Benzyl-4-((dimethylamino)methyl)cyclohexanon (1.13 g, 4.60 mmol) und Kaliumcyanid (0.70 g, 11.0 mmol) hinzugefügt. Die Mischung wurde 2 d bei Raumtemperatur gerührt und anschließend nach der Zugabe von Wasser (200 mL) mit Ether (4 x 150 mL) extrahiert. Nach dem Einengen der Lösung wurde der Rückstand in Dichlormethan (200 mL) aufgenommen und mit Magnesiumsulfat über Nacht getrocknet, filtriert und das Lösungsmittel i. Vak. entfernt. Das Nitril wurde als Öl erhalten, welches durchkristallisierte.
Ausbeute: 1.06 g (77 %)
¹H-NMR (DMSO-d₆): 1.23 (2 H, m); 1.74 (2 H, m); 2.16 (6 H, s); 2.24 (6 H, s); 2.32 (2 H, m); 2.68 (2 H, s); 7.16 (5 H, m).

### Stufe 2:

### [4-Benzyl-4-(dimethylaminomethyl)-1-phenyl-cyclohexyl]-dimethyl-amin

Die Titelverbindung der Stufe 1 (0.88 g, 2.94 mmol) wurde in THF (35 mL) gelöst und unter Eiskühlung mit 2M Phenylmagnesiumchlorid-Lösung (5.1 mL, 10.2 mmol) tropfenweise versetzt. Die Reaktionslösung wurde 8 h zum Sieden erhitzt. Zur Aufarbeitung wurde die Lösung unter Eiskühlung mit 20%-iger NH₄Cl-Lösung (0.6 mL) und Wasser (0.4 mL) versetzt, mit Ether (3 x 25 mL) extrahiert, die etherische Lösung mit Wasser gewaschen, getrocknet (Na₂SO₄) und i. Vak. eingeengt. Der Rückstand wurde durch Säulenchromatographie mit EtOH/EE (1:20) gereinigt. Es wurden 2 Fraktionen erhalten, wobei die polarere Fraktion (300 mg) It. LCMS u.a. die gewünschte Substanz enthielt.
Ausbeute: 90 mg (9 %)
¹H-NMR (DMSO-d₆): 1.23 (4 H, m); 1.39 (2 H, m); 1.82 (2H, s); 1.89 (6 H, s); 2.09 (6 H, s); 2.10 (2 H, m); 2.73 (2 H, s); 7.25 (10 H, m).

### Beispiel 23:

### (4-Dimethylamino-1,4-diphenyl-cyclohexyl)-methyl-dimethyl-amin

### Stufe 1:

### 5-Cyano-2-oxo-5-phenyl-cyclohexancarbonsäure-ethylester

Zu einer Lösung von Benzylcyanid (35.6 g, 304 mmol) und Brompropionsäure-ethylester (126 g, 694 mmol) in trockenem Toluol (1070 mL) wurde bei 0 bis 5 °C NaNH₂ (100 g, 2560 mmol) unter Rühren portionsweise über 3 h zugesetzt. Anschließend wurde 6 h zum Sieden erhitzt (die Reaktion war zunächst exotherm, so dass zeitweilig das Heizbad entfernt werden musste). Anschließend wurde das Gemisch auf 0 °C gekühlt und mit einem Gemisch aus Essigsäure (240 mL) und Wasser (120 mL) gequencht. Die Toluol-Phase wurde abgetrennt, die wässrige Phase mit Toluol (2 x 200 mL) extrahiert und die vereinigte org. Phase mit NaHCO₃-Lösung (2 x 200 mL) und Wasser (2 x 200 mL) gewaschen und mit Na₂SO₄ getrocknet.
Anschließend wurde das Lösungsmittel i. Vak. entfernt.
Ausbeute: 70,8 g (86%), brauner Feststoff
¹H-NMR (DMSO-d₆): 1.25 (3 H, t); 2.24 - 2.88 (6 H, m); 4.19 (2 H, q); 7.50 (5 H, m).

### Stufe 2:

### 4-Oxo-1 -phenyl-cyclohexancarbonitril

Die Titelverbindung der Stufe 1 (70.8 g, 261 mmol) wurde in einem Gemisch aus Essigsäure (810 mL) und konzentrierter Salzsäure (354 mL) 3.5 h unter DC-Kontrolle zum Sieden erhitzt. Anschließend wurde das Gemisch auf 0 bis 5°C gekühlt, mit Wasser (1 L) verdünnt, mit NaCl gesättigt und kalt mit Essigester (3 x 300 mL) extrahiert. Die Essigester-Phase wurde mit Wasser gewaschen und i. Vak. eingeengt. Der feste Rückstand wurde nochmals in Essigester gelöst, mit NaHCO₃-Lösung gewaschen und zur Trockene eingeengt.
Ausbeute 43.3 g (83%), gelber Feststoff
Der Rückstand wurde ohne weitere Reinigung zur Umsetzung mit Ethylenglykol eingesetzt.
¹H-NMR (DMSO-d₆): 2.41 (6 H, m); 2.71 (2 H, m); 7.40 (3 H, m); 7.60 (2 H, m).
¹³C-NMR (DMSO-d₆): 35.3; 38.1; 42.3; 121.7; 125.6; 128.2; 129.0; 139.2; 206.7.

### Stufe 3:

### 8-Phenyl-1,4-dioxa-spiro[4.5]decan-8-carbonitril

Die Titelverbindung der Stufe 2 (43.3 g, 217 mmol) und Ethylenglykol (27.4 g, 435 mmol) wurden in Toluol (430 mL) unter Zusatz von *p*-Toluolsulfonsäure (1.87 g, 10.9 mmol) am Wasserabscheider 3 h unter Rückfluss gekocht.
Nach Ende der Reaktion wurde abgekühlt, mit NaHCO₃-Lösung und ges. NaCl-Lösung gewaschen, mit Na₂SO₄ getrocknet und i. Vak. eingeengt.
Ausbeute: 48.8 g (96%), Feststoff
¹H-NMR (DMSO-d₆): 1.85 (4 H, m); 2.13 (4 H, m); 3.92 (4 H, s); 7.44 (5 H, m).
¹³ C-NMR (DMSO-d₆): 32.1; 34.0; 42.5; 63.8; 106.1; 122.1; 125.5; 128.0; 128.9; 139.9.

### Stufe 4:

### C-(8-Phenyl-1,4-dioxa-spiro[4.5]dec-8-yl)-methylamin

Zu einem Gemisch von LiAlH₄ (1.87 g, 49.3 mmol) in trockenem THF (25 mL) wurde unter Schutzgas langsam eine Lösung der Titelverbindung der Stufe 3 (10.0 g, 41.1 mmol) in trockenem THF (70 mL) zugetropft. Anschließend wurde 3 h unter Rückfluss gerührt. Nach dem Abkühlen des Reaktionsgemisches wurde unter Eiskühlung eine Lösung von Wasser (1.87 mL, 104 mmol), verdünnt mit etwas THF zugetropft und 10 min nachgerührt. Danach wurde 15 %-ige wässrige NaOH (1.87 mL, 8.17 mmol), mit etwas THF verdünnt zugetropft und anschließend wieder Wasser (5.6 mL) zugegeben.
Der gebildete Niederschlag wurde über Kieselgur filtriert und das Lösungsmittel i. Vak. entfernt. Als Rückstand verblieb das Amin.
Ausbeute: 7.96 g, (78%), gelbes Öl
¹³C-NMR (DMSO-d₆): 29.9; 31.0; 42.7; 53.9; 63.5; 108.3; 125.5; 126.8; 128.2; 143.8.

### Stufe 5:

### Dimethyl-(8-phenyl-1,4-dioxa-spiro[4.5]dec-8-ylmethyl)-amin

Die Titelverbindung der Stufe 4 (5.40 g, 21.8 mmol) wurde in Acetonitril (150 mL) gelöst, wobei eine trübe Lösung entstand. Wässrige, 37 %-ige Formalin-Lösung (30.6 mL, 407 mmol) wurde zugesetzt. Der Ansatz wurde bei RT 20 min gerührt und anschließend mit Natriumcyanoborhydrid (5.76 g, 91.7 mmol) versetzt. Die Reaktion wurde durch DC in Chloroform/Methanol (9:1) verfolgt. Nach 4 h wurde die Lösung mit Essigsäure auf pH 7 eingestellt und i. Vak. eingeengt. Der Rückstand wurde in Chloroform aufgenommen, mit NaHCO₃-Lösung gewaschen und die wässrige Phase mit Ether extrahiert. Die vereinigten organischen Phasen wurden über Na₂SO₄ getrocknet und i. Vak. eingeengt. Das Rohprodukt wurde durch Flash-Chromatographie mit Chloroform/Methanol (50:1→20:1→9:1) gereinigt.
Ausbeute: 5.40 g (67 %)
¹H-NMR (DMSO-d₆): 1.32 (2 H, m); 1.56 (2 H, m); 1.77 (2 H, m); 1.91 (6 H, s); 2.14 (2 H, m); 2.28 (2 H, s); 3.80 (4 H, m); 7.16 - 7.39 (5 H, m).

### Stufe 6:

### 4-Dimethylaminomethyl-4-phenyl-cyclohexanon

Die Titelverbindung der Stufe 5 (5.40 g, 19.6 mmol) wurde in 5 %-iger H₂SO₄ (300 mL) gelöst und 1 d bei RT gerührt. Anschließend wurde die Lösung dreimal mit Ether gewaschen und die Etherphase verworfen. Die wässrige Phase wurde unter Eiskühlung mit 5N NaOH alkalisch gestellt und dreimal mit Dichlormethan extrahiert. Die organische Phase wurde mit wenig Wasser gewaschen, über Na₂SO₄ getrocknet und i. Vak. eingeengt.
Ausbeute: 4.89 g (100 %)
¹H-NMR (DMSO-d₆): 1.92 (6 H, s); 1.94 - 2.00 (2 H, m); 2.07 - 2.25 (4 H, m); 2.39 - 2.46 (4 H, m); 7.23 (1 H, m); 7.37 (2 H, m); 7.48 (2 H, m).

### Stufe 7:

### 1-Dimethylamino-4-dimethylaminomethyl-4-phenyl-cyclohexancarbonitril

Zu einem Gemisch aus 4N Salzsäure (5 mL) und Methanol (3 mL) wurde unter Eiskühlung 40 %-ige, wässrige Dimethylaminlösung (12.8 mL 21.1 mmol) getropft. Anschließend wurden nacheinander die Titelverbindung der Stufe 6 (4.89 g, 21.1 mmol) und KCN (3.30 g, 50.7 mmol) zugegeben. Die Mischung wurde 3 d bei RT gerührt. Zur Aufarbeitung wurde der Ansatz mit Wasser (10 mL) versetzt und mit Diethylether (3 x 20 mL) extrahiert. Die Etherphase wurde i. Vak. eingeengt, der Rückstand in CH₂Cl₂ (20 mL) aufgenommen, über Na₂SO₄ getrocknet und i. Vak. eingeengt.
Ausbeute: 5.16 g (86 %)
¹H-NMR (DMSO-d₆): 1.28 (2 H, m); 1.69 (2 H, m); 1.94 (6 H, s); 2.05 (2 H, m); 2.15 (6 H, s); 2.26 (2 H, m); 2.37 (2 H, s); 7.19 (1 H, m); 7.35 (4 H, m).

### Stufe 8:

### (4-Dimethylaminomethyl-1,4-diphenyl-cyclohexyl)-dimethyl-amin

Zu einer Lösung der Titelverbindung der Stufe 7 (1.00 g, 3.5 mmol) in abs. THF (10 mL) wurde unter Stickstoffatmosphäre und Eiskühlung bei 10 °C langsam 2M Phenylmagnesiumchlorid-Lösung in THF (3.5 mL, 7.0 mmol) getropft. Die Lösung wurde 20 h bei RT gerührt. Anschließend wurden 20 %-ige NH₄Cl-Lösung (5 mL) und Wasser (2 mL) zugegeben und die Lösung mit Ether (3 x 5 mL) extrahiert. Die vereinigten organischen Phasen wurden mit Wasser (2 mL) und gesättigter NaCl-Lösung (2 mL) gewaschen, über Na₂SO₄ getrocknet und i. Vak. eingeengt. Durch Flash-Chromatographie des Rückstandes mit Chloroform/Methanol (20:1) wurde ein Salz des Produktes erhalten, welches mit 1N NaOH freigesetzt, mit Chloroform extrahiert, über Na₂SO₄ getrocknet und i. Vak. vom Lösungsmittel befreit wurde.
Ausbeute: 336 mg (28 %) unpolares Diastereoisomer, poröser Feststoff¹H-NMR (CDCl₃): 1.39 (2 H, m); 1.65 - 1.78 (2 H, m); 1.82 (6 H, s); 1.96 (6 H, s); 2.20 (2 H, s); 2.28 - 2.41 (4 H, m); 7.15 - 7.44 (10 H, m).

### Beispiel 24:

### (E)-N-(4-Dimethylamino-1,4-diphenyl-cyclohexyl)-N-methyl-3-phenyl-acrylamid (unpolares Diastereomer)

Die Titelverbindung aus Beispiel 8 (202 mg, 0.656 mmol) wurde in trock. THF (20 mL) vorgelegt und mit TEA (97 µL, 0.702 mmol) versetzt. Anschließend wurde Zimtsäurechlorid (116 mg, 0.702 mmol) zugegeben. Der Ansatz wurde 20 h bei Raumtemperatur gerührt. Nach dieser Reaktionszeit wurde der Ansatz i. Vak. bis zur Trockene eingeengt. Der Rückstand wurde in Essigester (20 mL) aufgenommen und mit gesättigter NaHCO₃-Lösung (2 x 20 mL) und gesättigter NaCl-Lösung (2 x 20 mL) gewaschen. Die organische Phase wurde über Na₂SO₄ getrocknet und i. Vak. eingeengt. Der Rückstand wurde durch Flash-Chromatographie mit Chloroform/Methanol (20:1) gereinigt.
Ausbeute: 95 mg (33 %)
¹H-NMR (DMSO-d₆): 1.61 (2 H, bs); 1.97 (5 H, bs); 2.45 (1 H, m); 2.49 (4 H, m); 2.94 (3 H, bs); 3.23 (1 H, s); 3.35 (1 H, s); 3.35 (2 H, m); 7.02 (1 H, m); 7.25 (2 H, m); 7.34 (9 H, m); 7.46 (2 H, m); 7.57 (2 H, m).

### Beispiel 33:

### (4-Benzyl-4-((dimethylamino)methyl)-N-methyl-1-phenylcyclohexanamin (polares Diastereomer)

### Stufe 1:

### 4-Benzyl-4-dimethylaminomethyl-1-methylamino-cyclohexancarbonitril

Zu einer auf 0 °C gekühlten Lösung von 4N Salzsäure (6.6 mL) und Methanol (4.00 mL) wurde 40 %-ige wässrige Methylaminlösung (15.3 mL, 121 mmol) und 4-Benzyl-4-((dimethylamino)methyl)cyclohexanon(6.20 g, 25.3 mmol), gelöst in Methanol (25 mL), gegeben. Anschließend wurde die Reaktionsmischung mit Kaliumcyanid (4.00 g, 60 mmol) versetzt und 5 d bei Raumtemperatur gerührt. Zur Aufarbeitung wurde das Gemisch mit Wasser (180 mL) verdünnt und mit Ether (3 x 100 mL) extrahiert. Die vereinigten organischen Phasen wurden über Na₂SO₄ getrocknet, filtriert und i. Vak. zur Trockene eingeengt.
Ausbeute: 5.80 g (81 %)
¹H-NMR (DMSO-d₆): 1.35 (5 H, m); 1.58 (8 H, m); 2.25 (6 H, m); 2.65 (4 H, m); 4.35 (1 H, m); 7.14 (3 H, m); 7.28 (2 H, m).

### Stufe 2:

### (4-Benzyl-4-((dimethylamino)methyl)-N-methyl-1-phenylcyclohexanamin (polares Diastereomer)

Phenyllithium (33 mL, 60 mmol, 1.8 M Lösung in Dibutylether) wurde unter Argon und bei Raumtemperatur tropfenweise mit einer Lösung der Titelverbindung aus Stufe 1 (5.70 g, 20 mmol) in Diethylether (60 mL) versetzt. Dabei erwärmte sich die Reaktionslösung auf 35 °C und ein Feststoff fiel aus. Das Reaktionsgemisch wurde 30 min unter Rückfluss gerührt, anschließend im Eisbad mit 20 %-iger NH₄Cl-Lösung (40 mL) hydrolysiert und die organische Phase abgetrennt. Die wässrige Phase wurde mit Ether (3 x 100 mL) extrahiert. Die vereinigten organischen Phasen wurden über Na₂SO₄ getrocknet und i. Vak. eingeengt. Der Rückstand wurde durch Flash-Chromatographie mit Chloro
form/Methanol (20:1→9:1→1:1 →1 % TEA) gereinigt.
Das polare Diastereomer wurde sauber erhalten. Das unpolare Diastereomer wurde verunreinigt isoliert. Ausbeute: 1.40 g (21 %), polares Diastereomer
¹H-NMR (DMSO-d₆): 1.13 (2 H, m); 1.74 (4 H, m); 1.89 (3 H, m); 1.96 (4 H, m); 2.23 (6 H, s); 2.68 (2 H, s); 7.15 (4 H, m); 7.26 (2 H, m); 7.33 (2 H, m); 7.48 (2 H, m).

### Beispiel 34:

### (1-Benzyl-4-dimethylamino-4-phenyl-cyclohexyl)-methyl-dimethyl-amin (polares Diastereomer)

Eine Lösung der Titelverbindung aus Beispiel 33 (1.40 g, 4.16 mmol) und Formalin (5.8 mL, 37 %-ige wässrige Lösung) in Acetonitril (40 mL) wurde portionsweise mit Natriumcyanoborhydrid (1.03 g, 16.6 mmol) versetzt und 45 min bei Raumtemperatur gerührt. Anschließend wurde konz. Essigsäure bis zur neutralen Reaktion zugegeben und 45 min bei Raumtemperatur gerührt. Zur Aufarbeitung wurde das Lösungsmittel i. Vak. entfernt, der Rückstand in 2N NaOH (40 mL) aufgenommen und anschließend mit Ether (3 x 40 mL) extrahiert. Die organische Phase wurde über Na₂SO₄ getrocknet und i. Vak. eingeengt. Der verbliebene Rückstand wurde durch Flash-Chromatographie mit Ethylacetat (Methanol→Methanol + 2% TEA) gereinigt.
Ausbeute: 200 mg (14 %)
¹H-NMR (DMSO-d₆): 1.10 (2 H, m); 1.56 (2 H, m); 1.89 (6 H, s); 2.00 (2 H, m); 2.04 (2 H, s); 2.11 (2 H, m); 2.25 (6 H, s); 2.58 (2 H, m); 7.19 (10 H, m).

### Beispiel 37:

### [4-(Dimethylaminomethyl)-1,4-diphenyl-cyclohexyl]-dimethyl-amin (polares Diastereomer)

Zu einer Lösung der Titelverbindung Beispiel 23, Stufe 7 (1.00 g, 3.5 mmol) in abs. THF (10 mL) wurde unter Stickstoffatmosphäre und Eiskühlung bei 0 - 10 °C langsam 2M Phenylmagnesium-chlorid-Lösung in THF (3.5 mL, 7.0 mmol) getropft. Die Lösung wurde 20 h bei RT gerührt. Anschließend wurden 20 %-ige NH₄Cl-Lösung (5 mL) und Wasser (2 mL) zugegeben und die Lösung mit Ether (3 x 5 mL) extrahiert. Die vereinigten organischen Phasen wurden mit Wasser (2 mL) und gesättigter NaCl-Lösung (2 mL) gewaschen, über Na₂SO₄ getrocknet und i. Vak. eingeengt. Flash-Chromatographie des Rückstandes mit Chloroform/Methanol (20:1→9:1→4:1→1 :1→1:1 + 1 % NH₃-MeOH + 1 % NH₃) ergab mit 20:1 das Hydrochlorid des unpolaren Diastereoisomers, welches mit 1N NaOH freigesetzt, mit Chloroform extrahiert, über Na₂SO₄ getrocknet und i. Vak. eingeengt wurde. Mit MeOH + 1 % NH₃ wurde das polare Diastereoisomer erhalten. Da das erste Spektrum hier ebenfalls auf ein Salz hindeutete, wurde auch das polare Diastereoisomer mit 1N NaOH freigesetzt, mit Chloroform extrahiert, über Na₂SO₄ getrocknet und i. Vak. eingeengt.
Ausbeute: 81 mg (7 %), polares Diastereoisomer, poröser Feststoff
¹H-NMR (DMSO-d₆): 1.59 (2 H, breit); 1.77 - 1.86 (2 H, m); 1.89 (6 H, s); 1.95 (6 H, s); 1.97 - 2.05 (2 H, m); 2.25 (2 H, m); 2.39 (2 H, s); 7.07 - 7.37 (10 H, m).

### Beispiel 42:

### (E)-N-[[4-Dimethylamino-4-(3-fluorphenyl)-1-methyl-cyclohexyl]-methyl]-3-phenyl-acrylamid (polares Diastereomer)

### Stufe 1:

### 4-Dimethylamino-4-(3-fluor-phenyl)-cyclohexancarbaldehyd

Zu einer Lösung von (Methoxymethyl)triphenylphosphoniumchlorid (6.58 g, 19.2 mmol) in abs. THF (25 mL) wurde bei 0 °C unter Argon KOtBu (2.15 g, 19.2 mmol), gelöst in abs. THF (25 mL), getropft. Die entstandene rote Lösung wurde nach 30 min bei 0 °C mit einer Lösung von 4-Dimethyl-amino-4-(3-fluor-phenyl)-cyclohexanon (3.0 g, 12.76 mmol) in abs. THF (25 mL) versetzt und über Nacht bei RT gerührt. Das Lösungsmittel wurde i. Vak. entfernt, der Rückstand mit 1M Schwefelsäure (50 mL) versetzt und 2 h gerührt. Der dabei ausfallende Niederschlag wurde abgetrennt und das Filtrat (pH 1) mit Ether (6x 30 mL) gewaschen. Die wässrige Lösung wurde mit 5N NaOH auf pH 11 eingestellt und mit Essigester extrahiert (3x 50 mL). Die vereinigten organischen Phasen wurden über Na₂SO₄ getrocknet und i. Vak. eingeengt.
Ausbeute: 3.20 g (100 %), braunes Öl
Diastereomerengemisch 1:1
¹H-NMR (DMSO-d₆): 1.20 (1 H, m); 1.62 (2 H, m); 1.75 (3 H, m); 1.93 (6 H, s); 2.37 (3 H, m); 7.12 (3 H, m); 7.40 (1 H, m); 9.50 (0.5 H, s); 9.62 (0.5 H, s).

### Stufe 2:

### 4-Dimethylamino-4-(3-fluor-phenyl)-1-methyl-cyclohexancarbaldehyd

Eine Lösung der Titelverbindung der Stufe 1 (2.73 g, 10.95 mmol) in abs. Dichlormethan (50 mL) wurde bei 0 °C unter Argon mit *tert*-BuOK (1.47 g, 13.14 mmol) und Methyliodid (747 µl, 12 mmol) versetzt. Nach 30 min wurde der Ansatz auf RT erwärmt und anschließend über Nacht gerührt (Feststoff fiel aus). Das Reaktionsgemisch wurde mit gesättigter NaCl-Lösung (50 mL) versetzt und mit Dichlormethan (3x 30 mL) extrahiert. Die vereinigten organischen Phasen wurden über Na₂SO₄ getrocknet, i. Vak. eingeengt und der verbliebene Rückstand durch Flash-Chromatographie mit Essigester/MeOH 20:1 gereinigt.
Ausbeute: 1.39 g (51 %)
¹H-NMR (DMSO-d₆): 0.85 (1.5 H, s); 1.00 (1.5 H, s); 1.50 (1 H, m); 1.54-1.77 (4 H, m); 1.89-1.95 (7 H, m); 2.11-2.31 (2 H, m); 7.11 (3 H, m); 7.38 (1 H, m); 9.36 (0.5 H, s); 9.44 (0.5 H, s).

### Stufe 3:

### 4-Dimethylamino-4-(3-fluor-phenyl)-1-methyl-cyclohexancarbaldehydoxim

Eine Lösung der Titelverbindung der Stufe 2 (1.38 g, 5.53 mmol) und Hydroxylaminhydrochlorid (576 mg, 8.3 mmol) in abs. Ethanol (20 mL) wurden mit Amberlyst A 21 (3.9 g) versetzt und 16 h bei RT gerührt. Der Ionenaustauscher wurde abfiltriert, die Lösung eingeengt und der Rückstand mit 1N NaOH alkalisch gestellt. Die wässrige Phase wurde mit Essigester extrahiert, über Na₂SO₄ getrocknet und i. Vak. eingeengt.
Ausbeute: 1.54 g (100 %)

### Stufe 4:

### [4-Aminomethyl-1-(3-fluor-phenyl)-4-methyl-cyclohexyl]-dimethyl-amin

Lithiumaluminiumhydrid (440 mg, 11.6 mmol) wurde unter Argon in abs. THF (50 mL) suspendiert, tropfenweise mit einer Lösung der Titelverbindung der Stufe 3 (1.54 g, 5.53 mmol) in abs. THF (20 mL) versetzt und 4 h unter Rückfluss gekocht. Anschließend wurde der Ansatz bei 10 °C mit Wasser (10 mL) hydrolysiert und über Kieselgur abfiltriert. Das THF wurde i. Vak. entfernt, der Rückstand mit 1 N NaOH auf pH 11 eingestellt und mit Essigester extrahiert. Die vereinigten organischen Phasen wurden über Na₂SO₄ getrocknet, i. Vak. eingeengt und der verbliebene Rückstand durch Flash-Chromatographie mit MeOH + 2 % NH₃ getrennt.
Ausbeute: 435 mg (30 %, unpolares Diastereomer)
¹H-NMR (DMSO-d₆): 0.85 (3 H, s); 1.03 (2 H, m); 1.29 (2 H, m); 1.83 (2 H, m); 1.91 (6 H, s); 2.08 (2 H, m); 2.17 (2 H, s); 7.09 (3 H, m); 7.38 (1 H, m).
Ausbeute: 510 mg (35 %, polares Diastereomer)
¹H-NMR (DMSO-d₆): 0.72 (3 H, s); 1.00 (2 H, m); 1.49 (2 H, m); 1.78 (2 H, m); 1.91 (6 H, s); 2.07 (2 H, m); 2.38 (2 H, s); 7.09 (3 H, m); 7.39 (1 H, m).

### Stufe 5:

### (E)-N-[[4-Dimethylamino-4-(3-fluorphenyl)-1-methyl-cyclohexyl]-methyl]-3-phenyl-acrylamid (polares Diastereomer)

Eine Lösung der Titelverbindung der Stufe 4 (polares Diastereomer) (250 mg, 0.94 mmol) und Hünigbase (169 µl, 1.0 mmol) in abs. Dichlormethan (10 mL) wurde mit Zimtsäurechlorid (166 mg 1.0 mmol) versetzt und 24 h bei RT gerührt. Die organische Lösung wurde mit gesättigter NaHCO₃-Lösung und gesättigter NaCl-Lösung gewaschen, über Na₂SO₄ getrocknet, i. Vak. eingeengt und der verbliebene Rückstand durch Flash-Chromatographie mit Essigester/MeOH 4:1 gereinigt.
Ausbeute: 295 mg (80 %), poröser Feststoff
¹H-NMR (DMSO-d₆): 0.77 (3 H, s); 1.03 (2 H, m); 1.53 (2 H, m);1.87 (2 H, m); 1.93 (6 H, s); 2.12 (2 H, m); 3.17 (2 H, d); 6.76 (1 H, d); 7.07 (3 H, m); 7.37 (5 H, m); 7.56 (2 H, m); 7.96 (1 H, t).

### Beispiel 48:

### [4-(Butyl-methyl-amino)-1,4-diphenyl-cyclohexyl]-dimethyl-amin (unpolares Diastereomer)

### Stufe 1:

### N-(4-Dimethylamino-1,4-diphenyl-cyclohexyl)-N-methyl-butyramid

Die Titelverbindung aus Beispiel 8 (308 mg, 1.0 mmol) wurde in abs. THF (15 mL) vorgelegt und mit TEA (165 µL, 1.2 mmol) und Butyrylchlorid (103 mg, 1.2 mmol, V= 124 µL) versetzt. Der Ansatz wurde 20 h bei Raumtemperatur gerührt und anschließend i. Vak. zur Trockene eingeengt. Der Rückstand wurde in Essigester (20 mL) aufgenommen und mit gesättigter NaHCO₃-Lösung (2x 20 mL) und mit gesättigter NaCl-Lösung (2x 20 mL) gewaschen. Die organische Phase wurde über Na₂SO₄ getrocknet und i. Vak. eingeengt. Der Rückstand wurde mittels Flash-Chromatographie mit Chloroform/Methanol (50:1) gereinigt.
Ausbeute: 206 mg (53 %)
¹H-NMR (DMSO-d₆): 0.76 (3 H, t); 1.41 (2 H, q); 1.60 (2 H, m); 1.95 (6 H, s); 2.22 (4 H, t); 2.33 (2 H, m); 2.82 (3 H, s); 7.20-7.39 (10 H, m).

### Stufe 2:

### [4-(Butyl-methyl-amino)-1,4-diphenyl-cyclohexyl]-dimethyl-amin (unpolares Diastereomer)

Die Titelverbindung aus Stufe 1 (200 mg, 0.528 mmol) wurde in abs. THF (15 mL) gelöst. LiAlH₄ (39 mg, 1.06 mmol) wurde unter Argon zugegeben. Der Ansatz wurde 7 h unter Rückfluss gekocht. Anschließend wurde der Ansatz auf Raumtemperatur abgekühlt, unter Eiskühlung mit THF (12 mL) und H₂O (5 mL) versetzt und 30 min nachgerührt. Der Ansatz wurde über eine Fritte mit Kieselgur filtriert und mit Dichlormethan (50 mL) nachgespült. Die vereinigten Filtrate wurde i. Vak. eingeengt.
Ausbeute: 194 mg (100 %), Öl
¹H-NMR (DMSO-d₆): 0.74 (3 H, t); 1.12 (4 H, m); 1.73 (4 H, breit); 1.83 (6 H, s); 1.90 (3 H, s); 1.92 (1 H, s); 1.96 (2 H, breit); 2.25 (4 H, breit); 7.25 (2 H, m); 7.38 (8 H, m).

### Beispiel 49

### [4-(Butyl-methyl-amino)-1,4-diphenyl-cyclohexyl]-dimethyl-amin (polares Diastereomer)

Die Titelverbindung aus Beispiel 9 (308 mg, 1.0 mmol) und Butyraldehyd (72 mg, 1.0 mmol, V= 89 µL) wurden in abs. Acetonitril (30 mL) vorgelegt und mit Natriumcyanoborhydrid (250 mg, 4.0 mmol) versetzt. Der Ansatz wurde 45 min bei Raumtemperatur gerührt, anschließend mit konz. Essigsäure (ca. 500 µL) versetzt und weitere 45 min bei Raumtemperatur gerührt. Zur Aufarbeitung wurde der Ansatz i. Vak. zur Trockene eingeengt. Der Rückstand wurde mit 2N NaOH versetzt und mit Ether (3x 20 mL) extrahiert. Die vereinigten organischen Phasen wurde über Na₂SO₄ getrocknet und i. Vak. eingeengt. Der Rückstand wurde mittels Flash-Chromatographie mit Chloroform/Methanol (9:1) gereinigt.
Ausbeute: 111 mg (30 %), Öl
¹H-NMR (DMSO-d₆): 0.86 (3 H, t); 1.30 (6 H, m); 2.05 (12 H, m); 2.35 (5 H, m); 7.29 (10 H, m).

### Beispiel 50:

### [4-(Benzyl-methyl-amino)-1,4-diphenyl-cyclohexyl]-dimethyl-amin (unpolares Diastereomer)

### Stufe 1:

### N-(4-Dimethylamino-1,4-diphenyl-cyclohexyl)-N-methyl-benzamid

Die Titelverbindung aus Beispiel 8 (308 mg, 1.0 mmol) wurde in abs. THF (15 mL) vorgelegt und mit TEA (165 µL, 1.2 mmol) und Benzoylchlorid (168 mg, 1.2 mmol, V= 147 µL) versetzt. Der Ansatz wurde 16 h bei Raumtemperatur gerührt und anschließend i. Vak. zur Trockene eingeengt. Der Rückstand wurde in Essigester (20 mL) aufgenommen, mit gesättigter NaHCO₃-Lösung(2x 20 mL) und mit gesättigter NaCl-Lösung (2x 20 mL) gewaschen. Die organische Phase wurde über Na₂SO₄ getrocknet und i. Vak. eingeengt. Der Rückstand wurde mittels Flash-Chromatographie mit Chloroform/Methanol (20:1) gereinigt.
Ausbeute: 169 mg (41 %)
¹H-NMR (DMSO-d₆): 1.75 (2 H, m); 1.98 (6 H, s); 2.38 (3 H, m); 2.55 (2 H, m); 2.69 (4 H, s); 7.24-7.41 (13 H, m); 7.54 (2 H, d).

### Stufe 2:

[4-(Benzyl-methyl-amino)-1,4-diphenyl-cyclohexyl]-dimethyl-amin (unpolares Diastereomer) Die Titelverbindung aus Stufe 1 (160 mg, 0.387 mmol) wurde in abs. THF (15 mL) gelöst und mit LiAlH₄ (29 mg, 0.775 mmol) unter Argon versetzt. Der Ansatz wurde 7 h unter Rückfluss gekocht und anschließend auf Raumtemperatur abgekühlt. Zu dem Ansatz wurde unter Eiskühlung THF (5 mL) und H₂O (5 mL) gegeben und 30 min nachgerührt. Der Ansatz wurde über eine Fritte mit Kieselgur filtriert und mit Dichlormethan (50 mL) nachgespült. Die vereinigten Filtrate wurde i. Vak. eingeengt.
Ausbeute: 149 mg (97 %)
¹H-NMR (DMSO-d₆): 1.78 (3 H, s); 1.85 (10 H, s); 2.33 (4 H, m); 3.14 (2 H, bs); 7.04-7.20 (4 H, m); 7.31 (2 H, m); 7.40 (9 H, m).

### Beispiel 51:

### [4-(Benzyl-methyl-amino)-1,4-diphenyl-cyclohexyl]-dimethyl-amin (polares Diastereomer)

### Stufe 1:

### N-(4-Dimethylamino-1,4-diphenyl-cyclohexyl)-N-methyl-benzamid

Die Titelverbindung aus Beispiel 9 (308 mg, 1.0 mmol) wurde in abs. THF (15 mL) vorgelegt und mit TEA (165 µL, 1.2 mmol) und Benzoylchlorid (168 mg, 1.2 mmol, V= 147 µL) versetzt. Der Ansatz wurde 16 h bei Raumtemperatur gerührt und anschließend i. Vak. bis zur Trockene eingeengt. Der Rückstand wurde in Essigester (20 mL) aufgenommen, mit gesättigter NaHCO₃-Lösung(2x 20 mL) und mit gesättigter NaCl-Lösung (2x 20 mL) gewaschen. Die organische Phase wurde über Na₂SO₄ getrocknet und i. Vak. eingeengt. Der Rückstand wurde mittels Flash-Chromatographie mit Chloroform/Methanol (20:1) gereinigt. Ausbeute: 304 mg (74 %)
¹H-NMR (DMSO-d₆): 1.63 (2 H, m); 1.92-2.00 (10 H, m); 2.52 (1 H, m); 2.76 (3 H, s); 7.16 (1 H, m); 7.28 (4 H, m); 7.39-7.49 (10 H, m).

### Stufe 2:

### [4-(Benzyl-methyl-amino)-1,4-diphenyl-cyclohexyl]-dimethyl-amin (polares Diastereomer)

Die Titelverbindung aus Stufe 1 (290 mg, 0.702 mmol) wurde in abs. THF (15 mL) gelöst und mit LiAlH₄ (52 mg, 1.40 mmol) unter Argon versetzt. Der Ansatz wurde 7 h unter Rückfluss gekocht und anschließend auf Raumtemperatur abgekühlt. Der Ansatz wurde über eine Fritte mit Kieselgur filtriert und mit Dichlormethan (50 mL) nachgespült. Die vereinigten Filtrate wurde i. Vak. eingeengt.
Ausbeute: 250 mg (89 %)
¹H-NMR (DMSO-d₆): 1.43 (1 H, m); 1.72-1.76 (1 H, m); 1.89 (3 H, s); 1.99 (6 H, s); 2.42 (3 H, breit); 3.25 (2 H, bs); 7.16-7.39 (15 H, m).

### Beispiel 66:

### 2-[(4-Dimethylamino-1,4-diphenyl-cyclohexyl)-methyl-amino]-essigsäure (polares Diastereomer)

### Stufe 1:

### [(4-Dimethylamino-1,4-diphenyl-cyclohexyl)-methyl-amino]-essigsäure-tert-butyl ester

Eine Lösung der Titelverbindung aus Beispiel 9 (246 mg, 0.8 mmol) und Bromessigsäure*tert*-butylester (132 µl, 0.9 mmol) in abs. DMF (10 mL) wurde mit Kaliumcarbonat (124 mg, 0.9 mmol) versetzt und 20 h bei RT gerührt. Anschließend wurde i. Vak. das Lösungsmittel entfernt, der Rückstand in Dichlormethan (20 mL) gelöst, mit Wasser (2x 10 mL) und gesättigter NaCl-Lösung (2x 10 mL) gewaschen und über Na₂SO₄ getrocknet. Die organische Lösung wurde i. Vak. eingeengt und der verbliebene Rückstand durch Flash-Chromatographie mit Essigester/MeOH 20:1 gereinigt.
Ausbeute: 133 mg (39 %)
¹H-NMR (CDCl₃): 1.44 (9 H, s); 1.78 (2 H, bs); 1.95 (2 H, bs); 2.09 (6 H, s); 2.21 (3 H, s); 2.43 (4 H, m); 2.92 (2 H, s); 7.16 - 7.31 (10 H, m).

### Stufe 2:

### 2-[(4-Dimethylamino-1,4-diphenyl-cyclohexyl)-methyl-amino]-essigsäure (polares Diastereomer)

Die Titelverbindung aus Stufe 1(130 mg, 0.3 mmol) wurde in Anisol (0.5 mL) und Trifluoressigsäure (2.5 mL) gelöst und 20 h bei RT gerührt. Anschließend wurde i. Vak. zur Trockene eingeengt, der feste Rückstand mit 1N NaOH durchgerührt, der Feststoff filtriert, mit Wasser gewaschen und i. Vak. getrocknet.
Ausbeute: 69 mg (63 %)
Schmelzpunkt: 270-273 °C
¹H-NMR (DMSO-d₆): 1.70 (3 H, br); 1.96 (6 H, s); 2.00 (3 H, s); 2.25 (2 H, m); 2.45 (4 H, m); 3.32 (2 H, s); 7.14 (2 H, m); 7.25 (8 H, m).

In Anlehnung an eine Vorschrift wie im Beispiel 66 beschrieben wurden mit der Ausnahme, daß die in der Tabelle 1-1 angeführten Edukte verwendet werden, folgende Verbindung erhalten.

**Tabelle 1-1:**

| **BSP.- Nr.** | **Edukt** | **Produkt** | **Cy (%) / MS (m/z)** |
|---|---|---|---|
| 67 | Bsp. 8 | 2-[(4-Dimethylamino-1,4-diphenyl-cyclohexyl)-methyl-amino]-essigsäure (unpolares Diastereomer) | 63 (367) |

### Beispiel 68

### [1-(4-Methoxyphenyl)-4-methylamino-4-phenyl-cyclohexyl]-dimethyl-amin (unpolares Diastereomer)

### Stufe 1:

### [8-(4-Methoxy-phenyl)-1,4-dioxa-spiro[4.5]dec-8-yl]-dimethyl-amin

Magnesium (3.65 g, 150 mmol) und ein Jodkristall wurden unter Stickstoffatmosphäre vorgelegt und erhitzt. Anschließend wurde abs. Ether (10 mL) zugegeben und eine Lösung von 4-Bromanisol (18.8 mL, 150 mmol) in abs. Ether (150 mL) so zugetropft, dass der Ether leicht siedete. Die entstandene Lösung wurde 1 h bei RT nachgerührt und anschließend bei RT tropfenweise mit einer Lösung von 8-Dimethylamino-1,4-dioxa-spiro[4.5]decan-8-carbonitril (10.5 g, 50.0 mmol) in abs. THF (100 mL) versetzt, wobei sich die Lösung während der Zugabe bis zum Sieden auf 37-40 °C erhitzte. Ein Niederschlag fiel aus der Lösung aus und der Ansatz wurde über Nacht bei RT gerührt. Die Lösung wurde unter Eiskühlung mit NH₄Cl-Lösung (150 mL) versetzt, die Phasen getrennt, die wässrige Phase dreimal mit Ether extrahiert, die vereinigten organischen Phasen mit gesättigter NaCl-Lösung und Wasser gewaschen, über Na₂SO₄ getrocknet und i. Vak. eingeengt. Flash-Chromatographie des Rückstandes mit Ethylacetat/Methanol (20:1→9:1→4:1→1:4→MeOH) ergab das gewünschte Produkt.
Ausbeute: 6.80 g (47 %)

### Stufe 2:

### 4-Dimethylamino-4-(4-methoxy-phenyl)-cyclohexanon

Die Titelverbindung aus Stufe 1 (6.80 g, 23 mmol) wurde in Ether (100 mL) gelöst, mit 5 %-iger H₂SO₄ (100 mL) versetzt und die Lösung 2 d bei RT kräftig gerührt. Die Phasen wurden getrennt und die Etherphase verworfen. Die wässrige Phase wurde unter Eiskühlung mit 5 N NaOH alkalisch gestellt und mit Ether dreimal extrahiert, die vereinigten organischen Phasen anschließend mit Wasser gewaschen, über Na₂SO₄ getrocknet und i. Vak. eingeengt.
Ausbeute: 4.20 g (73 %)
¹H-NMR (DMSO-d₆): 2.00 (6 H, s); 2.01-2.14 (4 H, m); 2.42-2.48 (2 H, m); 2.53-2.63 (2 H, m); 3.76 (3 H, s); 6.93 (2H, d); 7.34 (2 H, d).

### Stufe 3:

### 4-Dimethylamino-4-(4-methoxy-phenyl)-1-methylamino-cyclohexancarbonitril

Zu einem Gemisch aus 4N Salzsäure (1.98 mL) und Methanol (2.3 mL) wurde unter Eiskühlung 40 %-ige wässrige Methylamin-Lösung (3.50 mL, 40.1 mmol) getropft. Anschließend wurden eine Lösung der Titelverbindung aus Stufe 2 (2.00 mg, 8.09 mmol) in Methanol (30 mL) und Kaliumcyanid (1.32 g, 20.3 mmol) zugegeben. Die Mischung wurde 3 d bei RT gerührt und anschließend nach der Zugabe von Wasser (10 mL) mit Ether 4x extrahiert. Die vereinigten organischen Phasen wurden über Na₂SO₄ getrocknet und i. Vak. eingeengt.
Ausbeute: 2.23 g (96 %), verunreinigtes Produkt, wurde roh weiter umgesetzt
¹H-NMR (DMSO-d₆): 1.29 (1 H, m); 1.61 (1 H, m); 1.69-1.86 (4 H, m); 1.90 (6 H, d); 1.93-2.04 (2 H, m); 2.28 (3 H, dd); 2.75 (1 H, dq); 3.75 (3 H, d); 6.90 (2 H, d); 7.23 (2 H, dd).

### Stufe 4:

### [1-(4-Methoxyphenyl)-4-methylamino-4-phenyl-cyclohexyl]-dimethyl-amin (unpolares Diastereomer)

Phenyllithium (12.9 mL, 23.3 mmol, 1.8 M Lösung in Dibutylether) wurde unter Argon vorgelegt und bei RT tropfenweise mit einer Lösung der Titelverbindung der Stufe 3 (2.23 g, 7.76 mmol) in abs. Diethylether (30 mL) versetzt. Dabei erwärmte sich die Reaktionslösung auf 35°C und ein Feststoff fiel aus. Das Reaktionsgemisch wurde 1 h unter Rückfluss (Bad 50 °C) gerührt, anschließend im Eisbad (0-10 °C) mit 20 %-iger NH₄Cl-Lösung (20 mL) hydrolysiert und die organische Phase abgetrennt. Die wässrige Phase wurde mit Ether (3x 50 mL) extrahiert. Die vereinigten organischen Lösungen wurden über Na₂SO₄ getrocknet und i. Vak. eingeengt. Durch Flash-Chromatographie (100 g Kieselgel) mit Essigester/Methanol (20:1→9:1→MeOH→MeOH + 2 % NH₃) wurde das unpolare Diastereoisomer in einer Mischfraktion mit Ausgangsstoff und Keton und zuletzt das polare Diastereoisomer erhalten. Die Mischfraktion mit unpolarem Diastereoisomer wurde durch erneute Flash-Chromatographie mit Dichlormethan/Methanol (50:1 →20:1 →9:1 →4:1) gereinigt.
Ausbeute: 232 mg (9 %), unpolares Diastereoisomer
¹H-NMR (CDCl₃): 1.71 (2 H, m); 1.98 (4 H, m); 1.99 (6 H, s); 2.11 (1 H, m); 2.19-2.41 (5 H, m); 3.81 (3 H, s); 6.91 (2 H, m); 7.27 (3 H, m); 7.37 (2 H, m); 7.48 (2 H, m).

### Beispiel 69

### [1-(4-Methoxyphenyl)-4-methylamino-4-phenyl-cyclohexyl]-dimethyl-amin (polares Diastereomer)

Während der Synthese der Titelverbindung von Beispiel 68 konnte im Rahmen der Stufe 4 auch das polare Diastereomer isoliert werden.
Ausbeute: 177 mg (7 %), polares Diastereoisomer
¹H-NMR (CDCl₃): 1.58-1.92 (4 H, m); 2.03 (4 H, m); 2.07 (6 H, s); 2.10-2.18 (2 H, m); 2.29 (2 H, m); 3.80 (3 H, s); 6.87 (2 H, d); 7.14 (1 H, m); 7.20-7.33 (6 H, m).

In Anlehnung an eine Vorschrift wie in den Beispielen 68 und 69 beschrieben wurden mit der Ausnahme, daß die Bromide oder entsprechenden Grignardreagenzien sowie die Carbonitrile wie in der Tabelle 1-2 angeführt, verwendet werden, folgenden Verbindungen erhalten.

**Tabelle 1-2:**

| Carbonitrile: | | | | |
|---|---|---|---|---|
| 8-Dimethylamino-1,4-dioxa-spiro[4.5]decan-8-carbonitril (CN-A) | | | | |
| 8-(Pyrrolidin-1-yl)-1,4-dioxaspiro[4.5]decane-8-carbonitril (CN-B) | | | | |
| **BSP.- Nr.** | **R-Br/MgX** | **Carbonitril** | **Produkt** | **Cy (%) / MS (m/z)** |
| **38** | 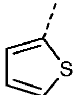 | (CN-A) | Dimethyl-(4-methylamino-4-phenyl-1-thiophen-2-yl-cyclohexyl)-amin (unpolares Diastereomer) | 20 (315) |
| **39** | 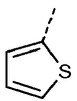 | (CN-A) | Dimethyl-(4-methylamino-4-phenyl-1-thiophen-2-yl-cyclohexyl)-amin (polares Diastereomer) | 33 (315) |
| **46** | 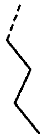 | (CN-A) | (1-Butyl-4-methylamino-4-phenyl-cyclohexyl)-dimethyl-amin (unpolares Diastereomer) | 2 (289) |
| **47** | 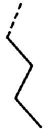 | (CN-A) | (1-Butyl-4-methylamino-4-phenyl-cyclohexyl)-dimethyl-amin (polares Diastereomer) | 28 (289) |
| **60** | 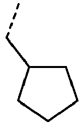 | (CN-A) | [4-(Cyclopentyl-methyl)-4-dimethylamino-1-phenyl-cyclohexyl]-methyl-amin (unpolares Diastereomer) | 8(315) |
| **61** | 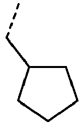 | (CN-A) | [4-(Cyclopentyl-methyl)-4-dimethylamino-1-phenyl-cyclohexyl]-methyl-amin (polares Diastereomer) | 36 (315) |
| **70** | 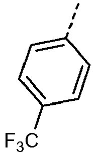 | (CN-A) | Dimethyl-[4-methylamino-4-phenyl-1-[4-(trifluormethyl)-phenyl]-cyclohexyl]-amin (unpolares Diastereomer) | 8 (377) |
| **71** | 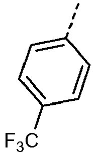 | (CN-A) | Dimethyl-[4-methylamino-4-phenyl-1-[4-(trifluormethyl)-phenyl]-cyclohexyl]-amin (polares Diastereomer) | 7 (377) |
| **147** | 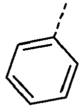 | (CN-B) | (1,4-Diphenyl-4-pyrrolidin-1-yl-cyclohexyl)-methylamin (polares Diastereomer) | 8 (335) |
| **148** | 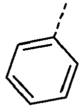 | (CN-B) | (1,4-Diphenyl-4-pyrrolidin-1-yl-cyclohexyl)-methylamin (unpolares Diastereomer) | 3 (335) |

### Beispiel 74:

### [4-Dimethylamino-1-(4-methoxyphenyl)-4-phenyl-cyclohexyl]-dimethyl-amin (polares Diastereomer)

Eine Lösung der Titelverbindung aus Beispiel 69 (111 mg, 0.33 mmol) und Formalin (0.45 mL, 37 %-ige wässrige Lösung) in Acetonitril (3 mL) wurde mit Natriumcyanoborhydrid (83 mg, 1.32 mmol) versetzt und 45 min bei RT gerührt. Anschließend wurde konz. Essigsäure bis zur neutralen Reaktion zugegeben und 45 min bei RT gerührt. Zur Aufarbeitung wurde das Lösungsmittel i. Vak. entfernt, der Rückstand in 2N NaOH (5 mL) aufgenommen und anschließend mit Ether (3 x 10 mL) extrahiert. Die organische Lösung wurde über Na₂SO₄ getrocknet und i. Vak. eingeengt. Der verbliebene Rückstand wurde durch Flash-Chromatographie mit Ethylacetat/Methanol (1:2→MeOH) gereinigt.
Ausbeute: 82 mg (71 %)
¹H-NMR (CDCl₃): 1.62-2.05 (4 H, m); 2.07 (12 H, s); 2.37 (4 H, m); 3.79 (3 H, s); (6.77 (3 H, s); 6.83 (2 H, d); 7.20 (3 H, m); 7.28 (4 H, m).

### Beispiel 75:

### [4-Dimethylamino-1-(4-methoxyphenyl)-4-phenyl-cyclohexyl]-dimethyl-amin (unpolares Diastereomer)

Eine Lösung der Titelverbindung aus Beispiel 68 (96 mg, 0.28 mmol) und Formalin (0.39 mL, 37 %-ige wässrige Lösung) in Acetonitril (3 mL) wurde mit Natriumcyanoborhydrid (72 mg, 1.15 mmol) versetzt und 45 min bei RT gerührt. Anschließend wurde konz. Essigsäure bis zur neutralen Reaktion zugegeben und 45 min bei RT gerührt. Zur Aufarbeitung wurde das Lösungsmittel i. Vak. entfernt, der Rückstand in 2N NaOH (5 mL) aufgenommen und anschließend mit Ether (3x 10 mL) extrahiert. Die organische Lösung wurde über Na₂SO₄ getrocknet und i. Vak. eingeengt. Der verbliebene Rückstand wurde durch Flash-Chromatographie mit Ethylacetat/Methanol (2:1→1 :1→1:1 + 2 % NH₃) gereinigt.
Ausbeute: 62 mg (62 %)
¹H-NMR (CDCl₃): 1.59 (4 H, m); 1.92 (6 H, s); 1.93 (6 H, s); 2.48 (4 H, m); 3.81 (3 H, s); 6.90 (2 H, m); 7.20-7.41 (7 H, m).

In Anlehnung an eine Vorschrift wie in den Beispielen 74 und 75 beschrieben wurden mit der Ausnahme, daß die in der Tabelle 1-3 angeführten Edukte verwendet werden, folgenden Verbindungen erhalten.

**Tabelle 1-3:**

| **BSP.- Nr.** | **Edukt (BSP.-Nr.)** | **Produkt** | **Cy (%) / MS (m/z)** |
|---|---|---|---|
| **40** | **39** | [4-(Dimethyl-amino)-4-phenyl-1-thiophen-2-yl-cyclohexyl]-dimethyl-amin (polares Diastereomer) | 73 (329) |
| **41** | **38** | (4-Dimethylamino-4-phenyl-1-thiophen-2-yl-cyclohexyl)-dimethyl-amin (unpolares Diastereomer) | 69 (329) |
| **58** | **46** | (4-Butyl-4-dimethylamino-1-phenyl-cyclohexyl)-dimethyl-amin (unpolares Diastereomer) | 27(303) |
| **59** | **47** | (4-Butyl-4-dimethylamino-1-phenyl-cyclohexyl)-dimethyl-amin (polares Diastereomer) | 79 (303) |
| **62** | **60** | [4-(Cyclopentyl-methyl)-4-dimethylamino-1-phenyl-cyclohexyl]-dimethyl-amin (unpolares Diastereomer) | 51 (329) |
| **63** | **61** | [4-(Cyclopentyl-methyl)-4-dimethylamino-1-phenyl-cyclohexyl]-dimethyl-amin (polares Diastereomer) | 86 (329) |
| **72** | **71** | [4-(Dimethyl-amino)-4-phenyl-1-[4-(trifluormethyl)-phenyl]-cyclohexyl]-dimethyl-amin (polares Diastereomer) | 36 (391) |
| **73** | **70** | [4-Dimethylamino-4-phenyl-1-[4-(trifluormethyl)-phenyl]-cyclohexyl]-dimethyl-amin (unpolares Diastereomer) | 68 (391) |
| **151** | **147** | (1,4-Diphenyl-4-pyrrolidin-1-yl-cyclohexyl)-dimethyl-amin (polares Diastereomer) | 34 (349) |
| **152** | **148** | (1,4-Diphenyl-4-pyrrolidin-1-yl-cyclohexyl)-dimethyl-amin (unpolares Diastereomer) | 45 (349) |

### Beispiel 76:

### [4-[(1H-Indol-3-yl-methylamino)-methyl]-4-methyl-1-phenyl-cyclohexyl]-dimethyl-amin (unpolares Diastereomer)

### Stufe 1:

### 4-Dimethylamino-4-phenyl-cyclohexancarbaldehyd

Zu einer Lösung von (Methoxymethyl)triphenylphosphoniumchlorid (25.7 g, 75.0 mmol) in abs. THF (100 mL) wurde bei 0 °C unter Argon *tert*-BuOK (8.41 g, 75 mmol), gelöst in abs. THF (100 mL), getropft. Die entstandene rote Lösung wurde nach 30 min bei 0 °C mit einer Lösung von 4-Dimethylamino-4-phenyl)-cyclohexanon (10.9 g, 50.0 mmol) in abs. THF (100 mL) versetzt und über Nacht bei RT gerührt. Das Lösungsmittel wurde i. Vak. entfernt, der Rückstand mit 1N Schwefelsäure (150 mL) versetzt und 2 h gerührt. Der dabei ausfallende Niederschlag wurde abgetrennt und das Filtrat (pH 1) mit Ether (6x 100 mL) gewaschen. Die wässrige Lösung wurde mit 5N NaOH auf pH 11 eingestellt und mit Essigester extrahiert (3x 100 mL). Die vereinigten organischen Phasen wurden über Na₂SO₄ getrocknet und i. Vak. eingeengt.
Ausbeute: 11.6 g (100 %), braunes Öl
Diastereomerengemisch 1:1
¹H-NMR (DMSO-d₆): 1.18 (1 H, m); 1.59-1.91 (5 H, m); 1.92 (6 H, s); 2.36 (3 H, m); 7.23-7.38 (5 H, m); 9.48 (0.5 H, s); 9.62 (0.5 H, s).

### Stufe 2:

### 4-Dimethylamino-1-methyl-4-phenyl-cyclohexancarbaldehyd

Eine Lösung der Titelverbindung aus Stufe 1 (11.6 g, 50.0 mmol) in abs. Dichlormethan (200 mL) wurde bei 0 °C unter Argon mit tert-BuOK (6.50 g, 58.0 mmol) und Methyliodid (3.42 mL, 55.0 mmol) versetzt. Nach 30 min wurde der Ansatz auf RT erwärmt und anschließend über Nacht gerührt (Feststoff fiel aus). Das Reaktionsgemisch wurde mit Wasser und gesättigter NaCl-Lösung (50 mL) gewaschen, über Na₂SO₄ getrocknet, i. Vak. eingeengt und der verbliebene Rückstand durch Flash-Chromatographie mit Essigester/MeOH 20:1 gereinigt.
Ausbeute: 5.90 g (48 %)
¹H-NMR (DMSO-d₆): 0.83 (1.5 H, s); 1.00 (1.5 H, s); 1.08 (1 H, m); 1.55-1.82 (5 H, m); 1.88 (3 H, s); 1.92 (3 H, s); 2.14-2.32 (2 H, m); 7.27 (5 H, m); 9.36 (0.5 H, s); 9.45 (0.5 H,s).

### Stufe 3:

### 4-Dimethylamino-1-methyl-4-phenyl-cyclohexancarbaldehydoxim

Eine Lösung von der Titelverbindung aus Stufe 2 (5.90 g, 24.0 mmol) und Hydroxylaminhydrochlorid (2.50 g, 36.0 mmol) in abs. Ethanol (100 mL) wurde mit Amberlyst A 21 (17.0 g) versetzt und 20 h bei RT gerührt. Der Ionenaustauscher wurde abfiltriert, die Lösung eingeengt und der Rückstand mit 1N NaOH alkalisch gestellt. Die wässrige Phase wurde mit Essigester extrahiert, über Na₂SO₄ getrocknet und i. Vak. eingeengt.
Ausbeute: 6.25 g (100 %)

### Stufe 4

### [4-Aminomethyl-4-methyl-1-phenylcyclohexyl]-dimethyl-amin

Lithiumaluminiumhydrid (1.82 g, 48.0 mmol) wurde unter Argon in abs. THF (200 mL) suspendiert, tropfenweise mit einer Lösung der Titelverbindung aus Stufe 3 (6.25 g, 24.0 mmol) in abs. THF (20 mL) versetzt und 4 h unter Rückfluss gekocht. Anschließend wurde der Ansatz bei 10 °C mit Wasser (20 mL) hydrolysiert und über Kieselgur abfiltriert. Das THF wurde i. Vak. entfernt, der Rückstand mit 1 N NaOH auf pH 11 eingestellt und mit Essigester extrahiert. Die vereinigten organischen Phasen wurden über Na₂SO₄ getrocknet, i. Vak. eingeengt und der verbliebene Rückstand durch Flash-Chromatographie mit MeOH + 1 % NH₃ getrennt.
Ausbeute: 1.44 g (24 %, unpolares Diastereomer)
¹H-NMR (DMSO-d₆): 0.86 (3 H, s); 1.03 (2 H, m); 1.29 (2 H, m); 1.84 (2 H, m); 1.91 (6 H, s); 2.10 (2 H, m); 2.16 (2 H, s); 7.24 (1 H, m); 7.32 (4 H, m).
Ausbeute: 1.53 g (26 %, polares Diastereomer)
¹H-NMR (DMSO-d₆): 0.72 (3 H, s); 1.00 (2 H, m); 1.49 (2 H, m); 1.83 (2 H, m); 1.90 (6 H, s); 2.05 (2 H, m); 2.39 (2 H, s); 7.23 (1 H, m); 7.34 (4 H, m).

### Stufe 5:

### [4-[(1H-Indol-3-yl-methylamino)-methyl]-4-methyl-1-phenyl-cyclohexyl]-dimethyl-amin (unpolares Diastereomer)

Indol-3-aldehyd (203 mg, 1.4 mmol) und das unpolare Diastereomer aus Stufe 4 (345 mg, 1.4 mmol) wurden in abs. THF (10 mL) gelöst, mit Na₂SO₄ (2.0 g) versetzt und 24 h bei RT gerührt. Anschließend wurde Dichlorethan (10 mL) und Natriumtriacetoxyborhydrid (423 mg, 2.0 mmol) zugegeben und weitere 24 h bei RT gerührt. Zur Aufarbeitung wurde i. Vak. das Lösungsmittel entfernt, der Rückstand mit EE (20 mL), Wasser (20 mL) und 10 %-iger Schwefelsäure (bis pH 1) versetzt und die Phasen getrennt. Die wässrige Phase wurde mit 5N NaOH auf pH 11 eingestellt und dreimal mit Essigester extrahiert. Die vereinigten organischen Phasen wurden über Na₂SO₄ getrocknet, i. Vak. eingeengt und der verbliebene Rückstand durch Flash-Chromatographie mit Essigester/MeOH 1:1 + 1 % NH₃ gereinigt.
Ausbeute: 397 mg (76 %), poröser Feststoff
¹H-NMR (DMSO-d₆): 0.92 (3 H, s); 1.14 (2 H, m); 1.32 (2 H, m); 1.89 (8 H, bs); 2.05 (2 H, m); 2.22 (2 H, s); 3.75 (2 H, s); 6.9-7.54 (10 H, m); 10.75 (1 H, s).

### Beispiel 77:

### [4-[(1H-Indol-3-yl-methylamino)-methyl]-4-methyl-1-phenyl-cyclohexyl]-dimethyl-amin (polares Diastereomer)

Indol-3-aldehyd (203 mg, 1.4 mmol) und das polare Diastereomer aus Beispiel 76, Stufe 4 (345 mg, 1.4 mmol) wurden in abs. THF (10 mL) gelöst, mit Na₂SO₄ (2.0 g) versetzt und 24 h bei RT gerührt. Anschließend wurde Dichlorethan (10 mL) und Natriumtriacetoxyborhydrid (423 mg, 2.0 mmol) zugegeben und weitere 24 h bei RT gerührt. Zur Aufarbeitung wurde i. Vak. das Lösungsmittel entfernt, der Rückstand mit Essigester (20 mL), Wasser (20 mL) und 10 %-iger Schwefelsäure (bis pH 1) versetzt und die Phasen getrennt. Die wässrige Phase wurde mit 5N NaOH auf pH 11 eingestellt und dreimal mit Essigester extrahiert. Die vereinigten organischen Phasen wurden über Na₂SO₄ getrocknet, i. Vak. eingeengt und der verbliebene Rückstand durch Flash-Chromatographie mit Essigester/MeOH (1:1 + 1 % NH₃) gereinigt.
Ausbeute: 370 mg (70 %)
Schmelzpunkt: 55-56 °C
¹H-NMR (DMSO-d₆): 0.78 (3 H, s); 1.02 (2 H, m); 1.57 (3 H, m); 1.79 (2 H, m) 1.86 (6 H, s); 2.02 (2 H, m); 2.44 (2 H, s); 3.89 (2 H, s); 6.97 (1 H, t); 7.06 (1 H, t); 7.22-7.32 (7 H, m); 7.64 (1 H, d); 10.82 (1 H, s).

### Beispiel 78:

### [4-[(1H-Indol-3-yl-methyl-methyl-amino)-methyl]-4-methyl-1-phenyl-cyclohexyl]-dimethyl-amin (unpolares Diastereomer)

Eine Lösung der Titelverbindung aus Beispiel 76 (300 mg, 0.8 mmol) und Formalin (1.2 mL, 37 %-ige wässrige Lösung) in Acetonitril (10 mL) wurde portionsweise mit Natriumcyanoborhydrid (201 mg, 3.2 mmol) versetzt und 2 h bei RT gerührt. Anschließend wurde konz. Essigsäure bis zur neutralen Reaktion zugegeben und 45 min bei RT gerührt. Zur Aufarbeitung wurde das Lösungsmittel i. Vak. entfernt, der Rückstand in 2N NaOH (10 mL) aufgenommen und anschließend mit Ether (3x 20 mL) extrahiert. Die organische Lösung wurde über Na₂SO₄ getrocknet und i. Vak. eingeengt. Der verbliebene Rückstand wurde durch Flash-Chromatographie mit Essigester/MeOH (1:1) gereinigt.
Ausbeute: 189 mg (56 %)
Laut NMR und LCMS handelt es sich dabei um die Hydroxymethylverbindung, die in 1N NaOH (2 mL) und THF (2 mL) gelöst und 2 h unter Rückfluss gekocht wurde. Anschließend wurde mit Ether (2x 20 mL) extrahiert. Die organische Lösung wurde über Na₂SO₄ getrocknet und i. Vak. eingeengt. Der verbliebene Rückstand wurde durch Flash-Chromatographie mit EE/MeOH (1:1 + 1 % NH₃) gereinigt.
Ausbeute: 119 mg (38 %)
¹H-NMR (CDCl₃): 1.04 (3 H, s); 1.32 (4 H, m); 1.87 (2 H, m); 2.05 (6 H, s) 2.14 (2 H, s); 2.15 (3 H, s); 2.34 (2 H, m); 3.63 (2 H, s); 6.78 (1 H, s); 7.08 (1 H, t); 7.17 (1 H, t); 7.30-7.41 (6 H, m); 7.62 (1 H, d); 7.99 (1 H, s).

### Beispiel 79:

### [4-[(1H-Indol-3-yl-methyl-methyl-amino)-methyl]-4-methyl-1-phenyl-cyclohexyl]-dimethyl-amin (polares Diastereomer)

Eine Lösung der Titelverbindung aus Beispiel 77 (300 mg, 0.8 mmol) und Formalin (1.2 mL, 37 %-ige wässrige Lösung) in Acetonitril (10 mL) wurde portionsweise mit Natriumcyanoborhydrid (201 mg, 3.2 mmol) versetzt und 2 h bei RT gerührt. Anschließend wurde konz. Essigsäure bis zur neutralen Reaktion zugegeben und 45 min bei RT gerührt.Zur Aufarbeitung wurde das Lösungsmittel i. Vak. entfernt, der Rückstand in 2N NaOH (10 mL) aufgenommen und anschließend mit Ether (3x 20 mL) extrahiert. Die organische Lösung wurde über Na₂SO₄ getrocknet und i. Vak. eingeengt. Der verbliebene Rückstand wurde mit EE/MeOH (1:1, 5 mL) versetzt, dabei fiel ein farbloser Feststoff aus, der abgetrennt wurde. Laut NMR und LCMS handelte es sich dabei um die Hydroxymethylverbindung .
Die Mutterlauge wurde eingeengt (240 mg), es handelte auch die Hydroxymethylverbindung. Ausbeute: 299 mg (89 %)
Die Hydroxymethylverbindung (240 mg, 0.57 mmol)) wurde in 1N NaOH (2 mL) und THF (2 mL) gelöst und 2 h unter Rückfluss gekocht wurde. Anschließend wurde mit Ether (2x 20 mL) extrahiert. Die organische Lösung wurde über Na₂SO₄ getrocknet und i. Vak. eingeengt. Der verbliebene Rückstand wurde durch Flash-Chromatographie mit EE/MeOH (1:1 + 1 % NH₃) gereinigt.
Ausbeute: 181 mg (82 %)
¹H-NMR (CDCl₃): 0.88 (3 H, s); 1.13 (2 H, m); 1.74 (2 H, m); 1.80 (4 H, m) 2.10 (6 H, s); 2.29 (3 H, s); 2.44 (2 H, s); 3.78 (2 H, s); 7.10-7.40 (9 H, m); 7.85 (1 H, d); 8.24 (1 H, s).

### Beispiel 80:

### [3-[[[4-(Dimethyl-amino)-1-methyl-4-phenyl-cyclohexyl]-methyl-methyl-amino]-methyl]-1H-indol-1-yl]-methanol (polares Diastereomer)

Im Rahmen der Synthese von Beispiel 79 fiel die Hydroxymethylverbindung als Zwischenprodukt an.
Ausbeute: 299 mg (89 %)
¹H-NMR (CDCl₃): 0.41 (2 H, m); 0.62 (2 H, m); 0.65 (3 H, s); 1.27 (2 H, m) 1.61 (2 H, m); 1.75 (6 H, s); 2.28 (2 H, s); 2.47 (3 H, s); 3.64 (2 H, s); 5.63 (2 H, s); 7.01 (2 H, m); 7.14-7.40 (7 H, m); 7.76 (1 H, d)

### Beispiel 86:

### [4-[[4,6-Bis(methylamino)-[1,3,5]triazin-2-yl]-methyl-amino]-1,4-diphenyl-cyclohexyl]-dimethyl-amin (unpolares Diastereomer)

Die Titelverbindung von Beispiel 103 (200 mg, 0.31 mmol) wurde in einer 33%-igen Methylamin-Lösung in Ethanol (2 mL) gelöst und in der Mikrowelle 30 min bei 100 °C und 60 min bei 120 °C gerührt. Der ausgefallene Niederschlag wurde abgesaugt und i. Vak. getrocknet.
Ausbeute: 89 mg (64%)
Schmelzpunkt: 250-252 °C
¹H-NMR (DMSO): 1.65 (2 H, m); 1.96 (6 H, s); 2.41 (4 H, m); 2.60 (6 H, s); 3.06 (5 H, m); 6.21 (2 H, m); 7.16-7.43 (10 H, m).

### Beispiel 87:

### [4-[[4-(4-Methoxy-phenoxy)-6-methylamino-[1,3,5]triazin-2-yl]-methyl-amino]-1,4-diphenyl-cyclohexyl]-dimethyl-amin (unpolares Diastereomer)

In Rahmen der Synthese der Titelverbindung von Beispiel 86 wurde
die Mutterlauge wurde i. Vak. eingeengt und der verbliebene Rückstand durch Flash-Chromatographie mit Essigester gereinigt.
Ausbeute: 25 mg (15 %)
Schmelzpunkt: 181-182 °C
¹H-NMR (DMSO), Temp: 100 °C: 1.70 (2 H, m); 1.99 (6 H, s); 2.24 (2 H, m); 2.38 (2 H, m); 2.56 (2 H, m); 2.67 (3 H, d); 2.96 (3 H, s); 3.76 (3 H, s); 6.71 (1 H, m); 6.98 (3 H, m); 7.15-7.38 (10 H, m).

In Anlehnung an eine Vorschrift wie in den Beispielen 86 und 87 beschrieben wurden mit der Ausnahme, daß Edukte, wie in der Tabelle 1-4 angeführt, verwendet werden, folgenden Verbindung erhalten.

**Tabelle 1-4:**

| **BSP.- Nr.** | **Edukt** | **Methode nach** | **Produkt** | **Cy (%) / MS (m/z)** |
|---|---|---|---|---|
| 90 | Bsp. 100 | Bsp. 86 | [4-[[4,6-Bis(methylamino)-[1,3,5]triazin-2-yl]-methyl-amino]-1,4-diphenyl-cyclohexyl]-dimethyl-amin (polares Diastereomer) | 13 (446) |
| 91 | Bsp. 100 | Bsp. 87 | [4-[[4-(4-Methoxy-phenoxy)-6-methylamino-[1,3,5]triazin-2-yl]-methyl-amino]-1,4-diphenyl-cyclohexyl]-dimethyl-amin (polares Diastereomer) | 59 (539) |

### Beispiel 94:

### [4-(Dimethyl-amino)-1-(3-fluorphenyl)-4-(3-methyl-1H-indol-2-yl)-cyclohexyl]-dimethyl-amin (polares Diastereomer)

### Stufe 1:

### 4-(Dimethylamino)-4-(3-fluorophenyl)-1-(3-methyl-1H-indol-2-yl)cyclohexanol (unpolares und polares Diastereomer)

In einer Argon-Atmosphäre wurde zu einer Lösung von Skatol (1,00 g, 7,62 mmol) in absolutem Tetrahydrofuran (25 ml) bei -78 °C langsam n-Butyllithium (8,39 mmol, 3,35 ml, 2,5M in Hexan) gegeben. Es bildete sich ein farbloser Niederschlag. Nach 10 min wurde die Lösung auf Raumtemperatur erwärmt. Anschließend wurde in die Reaktionsmischung für ca. 3 min Kohlendioxid eingeleitet. Es bildete sich eine farblose Lösung. Nach 5 min wurden die flüchtigen Bestandteile bei Raumtemperatur im Vakuum vollständig entfernt (Wasserbadtemperatur, ≤ 30 °C). Der farblose feste Rückstand wurde erneut in absolutem Tetrahydrofuran (20 ml) gelöst. Die hellgelbe Reaktionsmischung wurde auf -78 °C gekühlt und tert-Butyllithium (8,39 mmol, 5,59 ml, 1,5M in Pentan) hinzugetropft. Es bildete sich eine orange Lösung. Diese wurde 1 h bei -20 °C gerührt und dann auf -78 °C gekühlt. Anschließend wurde 4-(Dimethylamino)-4-(3-fluorophenyl)cyclohexanon [1,97 g, 8,39 mmol, in absolutem Tetrahydrofuran (20 ml)] hinzugetropft und die entstandene Lösung 2 h gerührt. Dann wurde gesättigte wäßrige Ammoniumchlorid-Lösung (50 ml) zur Reaktionsmischung getropft, 10 min gerührt, die Mischung auf 0 °C erwärmt und 20 min gerührt. Darauf wurde 2N wäßrige Chlorwasserstoff-Lösung (50 ml) hinzugegeben und 10 min gerührt (leichte Gasentwicklung). Anschließend wurde der pH-Wert der milchigen Suspension mit gesättigter Natriumhydrogencarbonat-Lösung (50 ml) und 5N Natriumhydroxid-Lösung (20 ml) basisch gestellt. Nach 10 min wurden die Phasen getrennt. Die organische Phase enthielt einen farblosen Feststoff. Die Phasen wurden getrennt. Die wäßrige Phase wurde mit Dichlormethan/Methanol 20 : 1 (3 x 50 ml) extrahiert.
Die organischen Lösungen wurden vereinigt. Die flüchtigen Bestandteile wurden vollständig im Vakuum entfernt. Das verbleibende, hellbraune Pulver wurde mit Methanol (5 x 75 ml) extrahiert. Der Rückstand bestand ausschließlich aus dem unpolareren Diastereoisomer 6b/7b (450 mg (1,23 mmol, 16 %). Die Extrakte wurden im Vakuum zur Trockne eingeengt. Der Rückstand wurde in Methanol (ca. 30 ml) aufgenommen. Ein heller Feststoff löste sich nicht. Dieser wurde mit Hilfe einer Fritte abgetrennt und anschließend im Vakuum getrocknet. Es wurden 980 mg (2,67 mmol, 35 %) eines farblosen Pulvers erhalten. Dieses bestand aus beiden Diastereoisomeren.

Die Mutterlauge wurde chromatographisch getrennt [Kieselgel 60 (150 g); Trichlormethan/Ethanol 50 : 1 (500 ml), 19 : 1 (500 ml), 9 : 1 (300 ml), 5 : 1 (300 ml), 1 : 1 (300 ml), jeweils 0,5 % Triethylamin, besser mit Trichlormethan/Ethanol 100 : 1 beginnen]. Die erhaltenen Fraktionen der beiden Diastereoisomerenmußten aus Methanol umkristallisiert werden. Es wurden 93 mg (0,25 mmol, 3 %) des unpolareren Diastereoisomers (Smp. 197-202 °C) und 146 mg (0,40 mmol, 5 %) des polareren Diastereoisomers(179-188 °C) erhalten.
13C{1H}-NMR (101 MHz, DMSO-D6, δ ppm, unpolareres Diastereoisomer): 9.5 (1 C), 28.4 (2 C), 32.5 (2 C), 37.8 (2 C), 58.2 (1 C, br), 69.4 (1 C), 102.7 (1 C), 111.0 (1 C), 113.0 (1 C, d, J = 21 Hz), 113.4 (1 C, d, J = 21 Hz), 117.3 (1 C), 117.8 (1 C) 119.9 (1 C), 122.6 (1 C, d, J = 2 Hz), 128.9 (1 C, J = 8 Hz), 129.8 (1 C), 133.9 (1 C), 142.1 (1 C, br), 142.7 (1 C, d, J = 5 Hz), 161.9 (1 C, d, J = 242 Hz)
13C{1H}-NMR (101 MHz, DMSO-D6, δ ppm, polareres Diastereoisomer): 9.0 (1 C), 26.9 (2 C, br), 33.5 (2 C), 37.6 (2 C), 55.9 (1 C, br), 68.5 (1 C), 102.3 (1 C), 110.9 (1 C), 113.5 (1 C, sbr), 115.8 (1 C, sbr), 117.2 (1 C), 117.8 (1 C) 120.0 (1 C), 125.0 (1 C, sbr), 126.6 (1 C), 130.0 (1 C, br), 133.7 (1 C), 141.1 (1 C, br), 162.4 (1 C, d, J = 244 Hz), n.b. (1 C)

### Stufe 2:

### 1-(3-Fluorophenyl)-4-(1H-indol-2-yl)-7,7-dimethyl-7-azoniabicyclo[2.2.1]heptanfluorid

Der Alkohol aus Stufe 1 (beide Diastereoisomere, 2,20 g, 6,00 mmol) wurde bei -78 °C in absolutem Dichlormethan (50 ml) suspendiert. Es wurden nacheinander Triethylamin (3,65 g, 36,02 mmol, 4,99 ml, 0,73 g/ml), DMAP (16 mg, 0,12 mmol) und DAST (2,90 g, 18,01 mmol, 2,36 ml, 1,23 g/ml) zugegeben. Die Lösung wurde 1 h bei -78 °C gerührt. Anschließend wurde die Reaktionsmischung innerhalb von 10 h (über Nacht) auf Raumtemperatur erwärmt. Dann wurde gesättigte Natriumhydrogencarbonat-Lösung (50 ml) hinzugegeben und 15 min gerührt (- bis die Gasentwicklung beendet war -). Anschließend wurde Natriumhydroxid-Lösung (5N, 20 ml) hinzugegeben und 10 min gerührt. Die Phasen wurden getrennt.
Die rotbraune organische Phase wurde im Vakuum zur Trockne eingeengt. Anschließend wurde der erhaltene braune Feststoff in Methanol (50 ml) gelöst.
Die wäßrige Phase wurde ebenfalls im Vakuum zur Trockne eingeengt. Der helle Rückstand wurde mit Methanol (5 × 75 ml) extrahiert.

Die vereinigten Methanol-Lösungen wurden im Vakuum zur Trockne eingeengt. Der Rückstand wurde erst mit Dichlormethan (2 × 30 ml) und dann mit Methanol (5 × 75 ml) extrahiert. Es blieb ein heller Feststoff zurück.
Die Methanol-Extrakte wurden im Vakuum zur Trockne eingeengt. Es verblieben 1,20 g (3,26 mmol, 54 %) des Produktes als heller Feststoff.
Die Dichlormethan-Extrakte wurden im Vakuum zur Trockne eingeengt. Der Rückstand wurde in Methanol (5 ml) aufgenommen und stehen gelassen. Es fiel ein weißer Feststoff aus. Es wurden so weitere 0,43 g (1,16 mmol, 19 %) des Produktes (Smp. 175 °C) erhalten.
¹³C{¹H}-NMR (101 MHz, DMSO-*D*₆, δ ppm): 11.2 (1 C), 29.8 (2 C), 30.3 (2 C), 40.6 (2 C), 81.2 (1 C, d, J = 2 Hz), 83.2 (1 C), 111.5 (1 C), 114.4 (1 C), 116.7 (1 C, d, J = 23 Hz), 117.6 (1 C, d, J = 21 Hz), 119.11 (1 C), 119.13 (1 C) 121.7 (1 C), 123.4 (1 C), 125.6 (1 C, J = 3 Hz), 128.8 (1 C), 131.0 (1 C, d, J = 8 Hz), 132.2 (1 C, d, J = 7 Hz), 135.8 (1 C), 162.3 (1 C, d, J = 244 Hz)

### Stufe 3:

### [4-(Dimethyl-amino)-1-(3-fluorphenyl)-4-(3-methyl-1H-indol-2-yl)-cyclohexyl]-dimethyl-amin (polares Diastereomer)

Die Titelverbindung aus Stufe 2 (500 mg, 1,36 mmol) wurde in Acetonitril/Methanol (1 : 1, 20 ml) suspendiert. Anschließend wurde Dimethylamin (2M in Tetrahydrofuran, 14 ml, 27,15 mmol) hinzugegeben und 2 d bei Raumtemperatur gerührt. Die Lösung wurde 6 h bei 80 °C (Ölbadtemperatur) gerührt, anschließend auf grobes Kieselgel aufgezogen und flashchromatographisch getrennt [Kieselgel 60 (150 g); Trichlormethan/Ethanol 50 : 1 (1000 ml), 19 : 1 (500 ml), 9 : 1 (1000 ml), jeweils 0,5 % Triethylamin]. Es wurde zunächst das unpolarere Diastereoisomer isoliert. Darüber hinaus wurden 250 mg eines Feststoffgemisches isoliert. Das Feststoffgemisch wurde in Methanol (10 ml) gelöst, 50 mg Kaliumhydroxid hinzugegeben und 10 min gerührt. Die flüchtigen Bestandteile wurden vollständig im Vakuum entfernt. Der helle Rückstand wurde mit Essigester (3 x 20 ml) extrahiert. Die Extrakte wurden im Vakuum von flüchtigen Bestandteilen befreit. Es wurden 135 mg (0,34 mmol, 25 %) des polareren Diastereoisomers (Smp. 65-73 °C) isoliert.

¹³C{¹H}-NMR (101 MHz, DMSO-*D*₆, δ ppm, polareres Diastereoisomer): 10.7 (1 C), 28.8 (2 C, br), 29.3 (2 C, br), 37.7 (2 C), 38.7 (2 C), 58.7 (1 C, br), 60.5 (1 C, br), 107.0 (1 C, br), 110.5 (1 C), 112.9 (1 C, d, J = 21 Hz), 113.7 (1 C, d, J = 21 Hz), 117.5 (1 C), 117.7 (1 C), 120.4 (1 C), 122.9 (1 C, br), 128.9 (1 C, d, J = 8 Hz), 129.0 (1 C), 132.5 (1 C, sbr), 134.5 (1 C), 141.4 (1 C, br), 161.9 (1 C, d, J = 243 Hz)

### Beispiel 97:

### [4-(Dimethyl-amino)-1-(3-fluorphenyl)-4-(3-methyl-1H-indol-2-yl)-cyclohexyl]-dimethyl-amin (unpolares Diastereomer)

Während der Synthese der Titelverbindung des Beispiels 94 Stufe 3 fiel auch das unpolare Diastereomer an. Es wurden 152 mg (0,39 mmol, 29 %) (Smp. 126-132 °C) isoliert.

¹³C{¹H}-NMR (101 MHz, DMSO-*D*₆, δ ppm, unpolareres Diastereoisomer): 10.7 (1 C), 29.6 (2 C, br), 29.7 (2 C, br), 37.8 (2 C), 38.7 (2 C), 60.0 (1 C, br), 60.6 (1 C, br), 107.0 (1 C, br), 110.5 (1 C), 113.0 (1 C, d, J = 21 Hz), 114.2 (1 C, d, J = 21 Hz), 117.5 (1 C), 117.8 (1 C), 120.4 (1 C), 123.5 (1 C, br), 129.1 (1 C), 129.1 (1 C, d, J = 6 Hz), 132.2 (1 C, br), 134.6 (1 C), 140.4 (1 C, br), 162.2 (1 C, d, J = 242 Hz)

In Anlehnung an eine Vorschrift wie in den Beispielen 94 und 97 beschrieben wurden mit der Ausnahme, daß Edukte, wie in der Tabelle 1-5 angeführt, verwendet werden, folgenden Verbindungen erhalten.

**Tabelle 1-5:**

| Indole: | | | | | |
|---|---|---|---|---|---|
| Skatol | | | | | |
| 5-Fluoro-3-methyl-1H-indol (IN-A) | | | | | |
| Ketone: | | | | | |
| 4-Dimethylamino-4-phenylcyclohexanon (BB-A) | | | | | |
| 4-(Dimethylamino)-4-(3-fluorophenyl)cyclohexanon (BB-B) | | | | | |
| 4-(Dimethylamino)-4-(thiophen-2-yl)cyclohexanon (BB-C) | | | | | |
| **BSP.-Nr.** | **Indol** | **Keton** | **Amin** | **Produkt** | **Cy* (%) / MS (m/z)** |
| 35 | Skatol | BB-A | Dimethylamin | [4-(Dimethyl-amino)-4-(3-methyl-1H-indol-2-yl)-1-phenyl-cyclohexyl]-dimethyl-amin (polares Diastereomer) | 27(376) |
| 36 | Skatol | BB-A | Dimethylamin | [4-(Dimethyl-amino)-4-(3-methyl-1H-indol-2-yl)-1-phenyl-cyclohexyl]-dimethyl-amin (unpolares Diastereomer) | 25(376) |
| 53 | Skatol | BB-A | Pyrrolidin | Dimethyl-[4-(3-methyl-1H-indol-2-yl)-1-phenyl-4-pyrrolidin-1-yl-cyclohexyl]-amindihydrochlorid (polares Diastereomer) | 17(402) |
| 54 | Skatol | BB-A | Pyrrolidin | Dimethyl-[4-(3-methyl-1H-indol-2-yl)-1-phenyl-4-pyrrolidin-1-yl-cyclohexyl]-amin (unpolares Diastereomer) | 48(402) |
| 56 | Skatol | BB-A | Azetidin | [4-(Azetidin-1-yl)-4-(3-methyl-1H-indol-2-yl)-1-phenyl-cyclohexyl]-dimethyl-amin (unpolares Diastereomer) | 19(388) |
| 82 | Skatol | BB-A | Methylamin | [4-Dimethylamino-1-(3-methyl-1H-indol-2-yl)-4-phenyl-cyclohexyl]-methylamin (polares Diastereomer) | 26(362) |
| 83 | Skatol | BB-A | Methylamin | [4-Dimethylamino-1-(3-methyl-1H-indol-2-yl)-4-phenyl-cyclohexyl]-methylamin (unpolares Diastereomer) | 22(362) |
| 84 | Skatol | BB-A | Benzylamin | Benzyl-[4-dimethylamino-1-(3-methyl-1H-indol-2-yl)-4-phenyl-cyclohexyl]-amin; 2-Hydroxy-propan-1,2,3-tricarbonsäure | 12(438) |
| 95 | Skatol | BB-B | Azetidin | 4-(Azetidin-1-yl)-1-(3-fluorophenyl)-N, N-d imethyl-4-(3-methyl-1H-indol-2-yl)cyclohexanamin (unpolares Diastereomer) | 33(406) |
| 96 | Skatol | BB-B | Azetidin | 4-(Azetidin-1-yl)-1-(3-fluorophenyl)-N, N-d imethyl-4-(3-methyl-1H-indol-2-yl)cyclohexanamin (polares Diastereomer) | 4(406) |
| 108 | Skatol | BB-B | Pyrrolidin | [1-(3-Fluorphenyl)-4-(3-methyl-1H-indol-2-yl)-4-pyrrolidin-1-yl-cyclohexyl]-dimethyl-amin (unpolares Diastereomer) | 36(420) |
| 109 | Skatol | BB-B | Pyrrolidin | [1-(3-Fluorphenyl)-4-(3-methyl-1H-indol-2-yl)-4-pyrrolidin-1-yl-cyclohexyl]-dimethyl-amin (polares Diastereomer) | 29(420) |
| 110 | Skatol | BB-B | Methylamin | [1-(3-Fluorphenyl)-4-methylamino-4-(3-methyl-1Hindol-2-yl)-cyclohexyl]-dimethyl-amin | 10(380) |
| 111 | Skatol | BB-A | Piperidin | Dimethyl-[4-(3-methyl-1H-indol-2-yl)-1-phenyl-4-piperidin-1-yl-cyclohexyl]-amin (unpolares Diastereomer) | 23(416) |
| 112 | Skatol | BB-B | Piperidin | [1-(3-Fluorphenyl)-4-(3-methyl-1H-indol-2-yl)-4-piperidin-1-yl-cyclohexyl]-dimethyl-amin (polares Diastereomer) | 4(434) |
| 133 | Skatol | BB-B | Piperidin | [1-(3-Fluorphenyl)-4-(3-methyl-1H-indol-2-yl)-4-piperidin-1-yl-cyclohexyl]-dimethyl-amin (unpolares Diastereomer) | 35 (434) |
| 113 | IN-A | BB-A | Dimethylamin | [4-(Dimethyl-amino)-4-(5-fluor-3-methyl-1H-indol-2-yl)-1-phenyl-cyclohexyl]-dimethyl-amin (polares Diastereomer) | 41(394) |
| 125 | IN-A | BB-A | Dimethylamin | [4-(Dimethyl-amino)-4-(5-fluor-3-methyl-1H-indol-2-yl)-1-phenyl-cyclohexyl]-dimethyl-amin (unpolares Diastereomer) | 27(394) |
| 126 | IN-A | BB-A | Pyrrolidin | [4-(5-Fluor-3-methyl-1H-indol-2-yl)-1-phenyl-4-pyrrolidin-1-yl-cyclohexyl]-dimethyl-amin (unpolares Diastereomer) | 42 (420) |
| 132 | IN-A | BB-A | Pyrrolidin | [4-(5-Fluor-3-methyl-1H-indol-2-yl)-1-phenyl-4-pyrrolidin-1-yl-cyclohexyl]-dimethyl-amin (polares Diastereomer) | 38 (420) |
| 134 | IN-A | BB-B | Azetidin | [4-(Azetidin-1-yl)-4-(5-fluor-3-methyl-1H-indol-2-yl)-1-(3-fluorphenyl)-cyclohexyl]-dimethyl-amin (polares Diastereomer) | 55 (406) |
| 135 | IN-A | BB-B | Azetidin | [4-(Azetidin-1-yl)-4-(5-fluor-3-methyl-1H-indol-2-yl)-1-(3-fluorphenyl)-cyclohexyl]-dimethyl-amin (unpolares Diastereomer) | 28 (406) |
| 136 | IN-A | BB-A | Morpholin | [4-(5-Fluor-3-methyl-1H-indol-2-yl)-4-morpholin-4-yl-1-phenyl-cyclohexyl]-dimethyl-amin (polares Diastereomer) | 30 (436) |
| 140 | IN-A | BB-A | Morpholin | [4-(5-Fluor-3-methyl-1H-indol-2-yl)-4-morpholin-4-yl-1-phenyl-cyclohexyl]-dimethyl-amin (unpolares Diastereomer) | 15 (436) |
| 137 | IN-A | BB-A | Methylamin | [4-(5-Fluor-3-methyl-1H-indol-2-yl)-4-methylamino-1-phenyl-cyclohexyl]-dimethyl-amin (unpolares Diastereomer) | 11 (380) |
| 138 | IN-A | BB-A | Methylamin | [4-(5-Fluor-3-methyl-1H-indol-2-yl)-4-methylamino-1-phenyl-cyclohexyl]-dimethyl-amin (polares Diastereomer) | 11 (380) |
| 139 | IN-A | BB-C | Methylamin | Dimethyl-[4-methylamino-4-(3-methyl-1H-indol-2-yl)-1-thiophen-2-yl-cyclohexyl]-amin (polares Diastereomer) | 21 (368) |
| 150 | IN-A | BB-C | Methylamin | Dimethyl-[4-methylamino-4-(3-methyl-1H-indol-2-yl)-1-thiophen-2-yl-cyclohexyl]-amin (unpolares Diastereomer) | 46(368) |
| 159 | IN-A | BB-B | Cyclohexylmethylamin | [4-(Cyclohexyl-methylamino)-1-(3-fluorphenyl)-4-(3-methyl-1H-indol-2-yl)-cyclohexyl]-dimethyl-amin (unpolares Diastereomer) | 43(462) |
| 160 | IN-A | BB-B | Cyclopentylamin | [4-(Cyclopentylamino)-1-(3-fluorphenyl)-4-(3-methyl-1Hindol-2-yl)-cyclohexyl]-dimethyl-amin (unpolares Diastereomer) | 36(434) |
| 161 | IN-A | BB-B | Anilin | [4-Anilino-1-(3-fluorphenyl)-4-(3-methyl-1H-indol-2-yl)-cyclohexyl]-dimethyl-amin | 28 (381; M+1-NMe₂-Me) |
| 162 | IN-A | BB-B | 4-Aminopyridin | [1-(3-fluorphenyl)-4-(3-methyl-1H-indol-2-yl)-4-(pyridin-4-ylamino)-cyclohexyl]-dimethyl-amin | 5(443) |

| | | | | | |
|---|---|---|---|---|---|
| * für die letzte Stufe. | | | | | |

In Anlehnung an eine Vorschrift wie im Beispiel 86 beschrieben wurden mit der Ausnahme, daß Amine und Edukte, wie in der Tabelle 1-6 angeführt, verwendet und im Falle von höhersiedenden Aminen auch ohne Lösungsmittel gearbeitet wurde, folgenden Verbindungen erhalten.

**Tabelle 1-6:**

| **BSP.-Nr.** | **Edukt** | **Amin** | **Produkt** | **Cy (%) / MS (m/z)** |
|---|---|---|---|---|
| 129 | Bsp. 91 | Piperidin | Dimethyl-[4-[methyl-(4-methylamino-6-piperidin-1-yl-[1,3,5]triazin-2-yl)-amino]-1,4-diphenyl-cyclohexyl]-amin (polares Diastereomer) | 89 (500) |
| 141 | Bsp.87 | Anilin | [4-[(4-Anilino-6-methylamino-[1,3,5]triazin-2-yl)-methyl-amino]-1,4-diphenyl-cyclohexyl]-dimethyl-amin (unpolares Diastereomer) | 38 (508) |
| 142 | Bsp.91 | *N-*Isopropylmethylamin | [4-[[4-(Isopropyl-methyl-amino)-6-methylamino-[1,3,5]triazin-2-yl]-methyl-amino]-1,4-diphenyl-cyclohexyl]-dimethyl-amin (polares Diastereomer) | 44 (488) |
| 143 | Bsp.91 | Anilin | [4-[(4-Anilino-6-methylamino-[1,3,5]triazin-2-yl)-methyl-amino]-1,4-diphenyl-cyclohexyl]-dimethyl-amin (polares Diastereomer) | 84 (508) |
| 144 | Bsp.91 | Benzylamin | [4-[[4-(Benzylamino)-6-methylamino-[1,3,5]triazin-2-yl]-methyl-amino]-1,4-diphenyl-cyclohexyl]-dimethyl-amin (polares Diastereomer) | 90 (522) |
| 145 | Bsp.91 | Butylamin | [4-[(4-Butylamino-6-methylamino-[1,3,5]triazin-2-yl)-methyl-amino]-1,4-diphenyl-cyclohexyl]-dimethyl-amin (polares Diastereomer) | 86(488) |

### Beispiel 100:

### [4-[[4,6-Bis(4-methoxy-phenoxy)-[1,3,5]-triazin-2-yl]-methyl-amino]-1,4-diphenyl-cyclohexyl]-dimethyl-amin (polares Diastereomer)

Eine Lösung der Titelverbindung von Beispiel 9 (616 mg, 2.0 mmol) und 4-Methoxyphenylcyanat (895 mg, 6.0 mmol) in abs. Aceton (20 mL) wurde 3 d bei RT gerührt. Anschließend wurde i. Vak. das Lösungsmittel entfernt und der verbliebene Rückstand durch Flash-Chromatographie mit Essigester/MeOH (20:1) gereinigt.
Ausbeute: 1.16 g (92 %)
¹H-NMR (CDCl₃): 1.77 (4 H, m); 1.89 (6 H, s); 2.50 (4 H, m); 3.07 (3 H, s); 3.76 (6 H, s); 6.84 - 7.36 (18 H, m).

### Beispiel 103:

### [4-[[4,6-Bis(4-methoxy-phenoxy)-[1,3,5]triazin-2-yl]-methyl-amino]-1,4-diphenyl-cyclohexyl]-dimethyl-amin (unpolares Diastereomer)

Eine Lösung der Titelverbindung von Beispiel 8 (154 mg, 0.5 mmol) und 4-Methoxyphenylcyanat (224 mg, 1.5 mmol) in abs. Aceton (10 mL) wurde 3 d bei RT gerührt. Anschließend wurde i. Vak. das Lösungsmittel entfernt und der verbliebene Rückstand durch Flash-Chromatographie mit Essigester/Cyclohexan (1:1) gereinigt.
Ausbeute: 226 mg (72 %)
¹H-NMR (CDCl₃): 1.80 (4 H, m); 1.96 (6 H, s); 2.28 (2 H, m); 2.43 (2 H, m); 3.04 (3 H, s); 3.80 (6 H, s); 6.89 - 7.40 (18 H, m).

In Anlehnung an eine Vorschrift wie im Beispiel 24 beschrieben wurden mit der Ausnahme, daß Acylierungs- oder Sulfonylierungsreagenzien und Amine, wie in der Tabelle 1-7 angeführt, verwendet werden, folgenden Verbindungen erhalten.

**Tabelle 1-7:**

| **BSP.-Nr.** | **Amin** | **Reagenz** | **Produkt** | **Cy (%) / MS (m/z)** |
|---|---|---|---|---|
| 25 | Bsp. 8 | Acetylchlorid | N-(4-Dimethylamino-1,4-diphenyl-cyclohexyl)-N-methyl-acetamid (unpolares Diastereomer) | 68 (351) |
| 26 | Bsp. 9 | Methansulfonylchlorid | N-(4-Dimethylamino-1,4-diphenyl-cyclohexyl)-N-methyl-methansulfonsäureamid (polares Diastereomer) | 36(38 7) |
| 27 | Bsp. 9 | Zimtsäurechlorid | (E)-N-(4-Dimethylamino-1,4-diphenyl-cyclohexyl)-N-methyl-3-phenyl-acrylamid (polares Diastereomer) | 90(43 9) |
| 28 | Bsp. 9 | Acetylchlorid | N-(4-Dimethylamino-1,4-diphenyl-cyclohexyl)-N-methyl-acetamid (polares Diastereomer) | 65 (351) |
| 29 | Bsp. 8 | Benzylisocyanat | 3-Benzyl-1-(4-dimethylamino-1,4-diphenyl-cyclohexyl)-1-methylharnstoff (unpolares Diastereomer) | 79 (442) |
| 30 | Bsp. 9 | Benzylisocyanat | 3-Benzyl-1-(4-dimethylamino-1,4-diphenyl-cyclohexyl)-1-methylharnstoff (polares Diastereomer) | 88 (442) |
| 31 | Bsp. 8 | Ethylisocyanat | 1-(4-Dimethylamino-1,4-diphenyl-cyclohexyl)-3-ethyl-1-methylharnstoff (unpolares Diastereomer) | 60 (380) |
| 32 | Bsp. 9 | Ethylisocyanat | 1-(4-Dimethylamino-1,4-diphenyl-cyclohexyl)-3-ethyl-1-methylharnstoff (polares Diastereomer) | 100 (380) |
| 43 | Bsp. 42, Stufe 4 (unpolar) | Zimtsäurechlorid | (E)-N-[[4-Dimethylamino-4-(3-fluorphenyl)-1-methyl-cyclohexyl]-methyl]-3-phenyl-acrylamid (unpolares Diastereomer) | 96(39 5) |
| 44 | Bsp. 42, Stufe 4 (unpolar) | (E)-2-phenylethenesulfonyl chloride | (E)-N-[[4-Dimethylamino-4-(3-fluorphenyl)-1-methyl-cyclohexyl]-methyl]-2-phenyl-ethensulfonsäureamid (unpolares Diastereomer) | 68(43 1) |
| 45 | Bsp. 42, Stufe 4 (polar) | E)-2-phenylethenesulfonyl chloride | (E)-N-[[4-Dimethylamino-4-(3-fluorphenyl)-1-methyl-cyclohexyl]-methyl]-2-phenyl-ethensulfonsäureamid (polares Diastereomer) | 66(43 1) |
| 52 | Bsp. 9 | Diphenylacetylchlorid | N-[4-(Dimethyl-amino)-1,4-diphenyl-cyclohexyl]-N-methyl-2,2-diphenyl-acetamid (polares Diastereomer) | 23 (337) |
| 57 | Bsp. 8 | Methansulfonylchlorid | N-(4-Dimethylamino-1,4-diphenyl-cyclohexyl)-N-methyl-methansulfonsäureamid (unpolares Diastereomer) | 15 (387) |
| 64 | Bsp. 60 | Zimtsäurechlorid | (E)-N-[4-(Cyclopentyl-methyl)-4-dimethylamino-1-phenyl-cyclohexyl]-N-methyl-3-phenyl-acrylamid (unpolares Diastereomer) | 75 (445) |
| 65 | Bsp. 61 | Zimtsäurechlorid | (E)-N-[4-(Cyclopentyl-methyl)-4-dimethylamino-1-phenyl-cyclohexyl]-N-methyl-3-phenyl-acrylamid (polares Diastereomer) | 63 (445) |
| 81 | Bsp. 82 | Zimtsäurechlorid | (E)-N-[4-Dimethylamino-1-(3-methyl-1H-indol-2-yl)-4-phenyl-cyclohexyl]-N-methyl-3-phenyl-acrylamid (polares Diastereomer) | 17 (492) |
| 88 | Bsp. 8 | Nicotinsäurechlorid Hydrochlorid | N-[4-(Dimethyl-amino)-1,4-diphenyl-cyclohexyl]-N-methyl-pyridin-3-carbonsäureamid (unpolares Diastereomer) | 58 (414) |
| 92 | Bsp. 9 | 1-Methyl-1*H*-pyrazol-3-carbonsäurechlorid | N-[4-(Dimethyl-amino)-1,4-diphenyl-cyclohexyl]-N,1-dimethyl-1H-pyrazol-3-carbonsäureamid (polares Diastereomer) | 93 (417) |
| 98 | Bsp. 8 | 3-Trifluormethylbenzoylchlorid | N-(4-Dimethylamino-1,4-diphenyl-cyclohexyl)-N-methyl-3-(trifluormethyl)-benzamid (unpolares Diastereomer) | 43 (481) |
| 99 | Bsp. 9 | 3-(Trifluormethyl)benzoylch lorid | N-(4-Dimethylamino-1,4-diphenyl-cyclohexyl)-N-methyl-3-(trifluormethyl)-benzamid (polares Diastereomer) | 67 (481) |
| 104 | Bsp. 8 | 4-Methoxy-phenylcarbonsäurechlorid | N-(4-Dimethylamino-1,4-diphenyl-cyclohexyl)-4-methoxy-N-methyl-benzamid (unpolares Diastereomer) | 59 (443) |
| 105 | Bsp. 9 | 4-Methoxy-phenylcarbonsäurechlorid | N-(4-Dimethylamino-1,4-diphenyl-cyclohexyl)-4-methoxy-N-methyl-benzamid (polares Diastereomer) | 90 (443) |
| 116 | Bsp. 8 | 3-Fluorbenzoylchlorid | N-(4-Dimethylamino-1,4-diphenyl-cyclohexyl)-3-fluor-N-methyl-benzamid (unpolares Diastereomer) | 49 (431) |
| 117 | Bsp. 9 | 3-Fluorbenzoylchlorid | N-(4-Dimethylamino-1,4-diphenyl-cyclohexyl)-3-fluor-N-methyl-benzamid (polares Diastereomer) | 84 (431) |
| **165** | Bsp. 9 | Cyclohexancarbonsäurechlorid | N-(4-Dimethylamino-1,4-diphenyl-cyclohexyl)-N-methyl-cyclohexancarbonsäure amid (polares Diastereomer) | 50 (419) |
| **166** | Bsp. 9 | Tetrahydro-pyran-4-carbonsäurechlorid | N-(4-Dimethylamino-1,4-diphenyl-cyclohexyl)-N-methyl-tetrahydropyran-4-carbonsäureamid (polares Diastereomer) | 30 (421) |
| **169** | Bsp. 9 | 1-Methyl-piperidin-4-carbonsäurechlorid | N-(4-Dimethylamino-1,4-diphenyl-cyclohexyl)-N,1-dimethyl-piperidin-4-carbonsäureamid (polares Diastereomer) | 79 (434) |

In Anlehnung an eine Vorschrift wie im Beispiel 48, Stufe 2 beschrieben wurden mit der Ausnahme, daß die Amide, wie in der Tabelle 1-8 angeführt, verwendet werden, folgenden Verbindungen erhalten.

**Tabelle 1-8:**

| BB-1: | | | |
|---|---|---|---|
| N-(4-Dimethylamino-1,4-diphenyl-cyclohexyl)-N-methyl-nicotinamid | | | |
| Eine Lösung der Titelverbindung aus Beispiel 9 (308 mg, 1.0 mmol) und Triethylamin (334 µl, 2.4 mmol) in abs. THF (15 mL) wurde mit Nicotinsäurechlorid Hydrochlorid (214 mg, 1.2 mmol) versetzt und 3 d bei RT gerührt. Anschließend wurde i. Vak. das Lösungsmittel entfernt, der verbliebene Rückstand in Essigester gelöst, mit ges. NaHCO₃-Lösung und ges. NaCl-Lösung gewaschen, über Na₂SO₄ getrocknet und durch Flash-Chromatographie mit Essigester/MeOH 1:1 gereinigt. | | | |
| Ausbeute: 300 mg (73 %), poröser Feststoff | | | |
| ¹H-NMR (DMSO): 1.67 (2 H, m); 1.92 (2 H, m); 1.98 (8 H, s); 2.48 (2 H, m); 2.80 (3 H, s); 7.15-7.41 (10 H, m); 7.52 (1 H, m); 7.92 (1 H, m); 8.69 (2 H, m). | | | |
| **BSP.- Nr.** | **Amid** | **Produkt** | **Cy (%) / MS (m/z)** |
| 85 | BB-1 | Dimethyl-[4-[methyl-(pyridin-3-yl-methyl)-amino]-1,4-diphenyl-cyclohexyl]-amin (polares Diastereomer) | 37 (400) |
| 89 | Bsp. 89 | Dimethyl-[4-[methyl-(pyridin-3-yl-methyl)-amino]-1,4-diphenyl-cyclohexyl]-amin (unpolares Diastereomer) | 73 (400) |
| 101 | Bsp. 92 | (4-Dimethylamino-1,4-diphenyl-cyclohexyl)-methyl-[(1-methyl-1H-pyrazol-3-yl)-methyl]-amin (polares Diastereomer) | 71 (403) |
| 102 | Bsp. 93 | (4-Dimethylamino-1,4-diphenyl-cyclohexyl)-methyl-[(1-methyl-1H-pyrazol-3-yl)-methyl]-amin (unpolares Diastereomer) | 67 (403) |
| 106 | Bsp. 105 | (4-Dimethylamino-1,4-diphenyl-cyclohexyl)-[(4-methoxyphenyl)-methyl]-methyl-amin (polares Diastereomer) | 85 (429) |
| 107 | Bsp. 104 | (4-Dimethylamino-1,4-diphenyl-cyclohexyl)-[(4-methoxyphenyl)-methyl]-methyl-amin (unpolares Diastereomer) | 77 (429) |
| 114 | Bsp. 99 | (4-Dimethylamino-1,4-diphenyl-cyclohexyl)-methyl-[[3-(trifluormethyl)phenyl]-methyl]-amin (polares Diastereomer) | 81 (467) |
| 115 | Bsp. 98 | (4-Dimethylamino-1,4-diphenyl-cyclohexyl)-methyl-[[3-(trifluormethyl)phenyl]-methyl]-amin (unpolares Diastereomer) | 42 (467) |
| 118 | Bsp. 116 | (4-Dimethylamino-1,4-diphenyl-cyclohexyl)-[(3-fluorphenyl)-methyl]-methylamin (unpolares Diastereomer) | 98 (417) |
| 119 | Bsp. 117 | (4-Dimethylamino-1,4-diphenyl-cyclohexyl)-[(3-fluorphenyl)-methyl]-methylamin (polares Diastereomer) | 99 (417) |
| 167 | Bsp. 165 | Cyclohexyl-methyl-(4-dimethylamino-1,4-diphenyl-cyclohexyl)-methyl-amin (polares Diastereomer) | 84 (405) |
| 168 | Bsp. 166 | (4-Dimethylamino-1,4-diphenyl-cyclohexyl)-methyl-(tetrahydro-pyran-4-yl-methyl)-amin (polares Diastereomer) | 44 (407) |
| 170 | Bsp. 169 | (4-Dimethylamino-1,4-diphenyl-cyclohexyl)-methyl-[(1-methyl-piperidin-4-yl)-methyl]-amin (polares Diastereomer) | 93 (420) |

### Beispiel 120:

### 2-[(4-Dimethylamino-1,4-diphenyl-cyclohexyl)-methyl-amino]-ethanol (polares Diastereomer)

### Stufe 1:

### [(4-Dimethylamino-1,4-diphenyl-cyclohexyl)-methyl-amino]-essigsäure-methylester

Die Titelverbindung aus Beispiel 9(463 mg, 1.50 mmol) wurde in abs. DMF (10 mL) vorgelegt und mit Kaliumcarbonat (347 mg, 1.65 mmol) und Bromessigsäure-methylester (157 µL, 1.65 mmol) versetzt. Der Ansatz wurde 3 d bei Raumtemperatur gerührt und anschließend i. Vak. zur Trockene eingeengt. Der Rückstand wurde in Dichlormethan (50 mL) aufgenommen und mit Wasser (2 x 50 mL) und gesättigter NaCl-Lösung (50 mL) gewaschen, die organische Phase anschließend über Na₂SO₄ getrocknet und i. Vak. eingeengt. Der Rückstand wurde mittels Flash-Chromatographie mit Essigester/Methanol (9:1) gereinigt.
Ausbeute: 338 mg (59 %)
¹H-NMR (DMSO-d₆): 1.73 (4 H, m); 1.96 (6 H, s); 2.04 (3 H, s); 2.31 (4 H, m); 2.96 (2 H, m); 3.58 (3 H, s); 7.17 (2 H, m); 7.28 (8 H, m).

### Stufe 2:

### 2-[(4-Dimethylamino-1,4-diphenyl-cyclohexyl)-methyl-amino]-ethanol (polares Diastereomer)

Die Titelverbindung aus Stufe 1 (322 mg, 0.85 mmol) wurde in abs. THF (15 mL) gelöst, mit LiAlH₄ (64 mg, 1.69 mmol) unter Argon versetzt und 3 h unter Rückfluss gekocht. Anschließend wurde der Ansatz auf Raumtemperatur abgekühlt, unter Eiskühlung mit THF (10 mL) und H₂O (5 mL) versetzt und 30 min nachgerührt. Der Ansatz wurde über eine Fritte mit Kieselgur filtriert und das Kieselgur mit Dichlormethan (50 mL) nachgewaschen. Die vereinigten Filtrate wurden i. Vak. eingeengt. Das Rohprodukt wurde mit Wasser (10 mL) versetzt und mit Dichlormethan (3 x 20 mL) extrahiert. Die organische Phase wurde über Na₂SO₄ getrocknet und i. Vak. eingeengt. Der Rückstand wurde mittels Flash-Chromatographie mit Essigester/Methanol (1:1) gereinigt.
Ausbeute: 213 mg (71 %)
¹H-NMR (DMSO-d₆): 1.72 (4 H, m); 1.95 (6 H, s); 2.06 (3 H, s); 2.19 (2 H, m); 2.29 (4 H, m); 3.39 (2 H, m); 4.25 (1 H, m); 7.17 (2 H, m); 7.27 (8 H, m).

### Beispiel 122:

### 2-[(4-Dimethylamino-1,4-diphenyl-cyclohexyl)-methyl-amino]-N-methyl-acetamid (polares Diastereomer)

Die Titelverbindung aus Beispiel 66 (293 mg, 0.8 mmol) wurde in abs. DMF (10 mL) gelöst und mit *N*-Hydroxybenzotriazol Hydrat (135 mg, 0.88 mmol) und TEA (1.11 mL, 8.0 mmol) versetzt. Nach 30 min wurde *N*-(3-Dimethylaminopropyl)-*N'*-ethyl-carbodiimid Hydrochlorid (460 mg, 2.4 mmol) und Methylamin (440 µL, 0.88 mmol, 2M Lösung in THF) addiert und über Nacht bei RT gerührt. Die Lösung wurde filtriert und i. Vak. eingeengt. Durch Flash-Chromatographie des Rückstandes mit Essigester/MeOH (4:1→1:1) wurde ein Salz des Produktes erhalten, welches mit 1N NaOH freigesetzt, mit CH₂Cl₂ extrahiert, über Na₂SO₄ getrocknet und i. Vak. vom Lösungsmittel befreit wurde.
Ausbeute: 182 mg (60 %)
¹H-NMR (DMSO): 1.47 (2 H, m); 1.96 (7 H, s); 1.99 (3 H, s); 2.24 (3 H, m); 2.42 (2 H, m); 2.64 (6 H, m); 7.24 (9 H, m); 7.60 (1 H, m).

In Anlehnung an eine Vorschrift wie im Beispiel 122 beschrieben wurden mit der Ausnahme, daß die in der Tabelle 1-9 angeführten Säuren und Amine verwendet werden, folgende Verbindungen erhalten.

**Tabelle 1-9:**

| **BSP.- Nr.** | **Edukt** | **Amin** | **Produkt** | **Cy (%) / MS (m/z)** |
|---|---|---|---|---|
| 121 | Bsp.66 | Dimethylamin | 2-[(4-Dimethylamino-1,4-diphenyl-cyclohexyl)-methyl-amino]-N, N-dimethyl-acetamid (polares Diastereomer) | 17 (394) |
| 123 | Bsp.67 | Dimethylamin | 2-[(4-Dimethylamino-1,4-diphenyl-cyclohexyl)-methyl-amino]-N,N-dimethyl-acetamid (unpolares Diastereomer) | 55 (380) |
| 124 | Bsp.67 | Methylamin | 2-[(4-Dimethylamino-1,4-diphenyl-cyclohexyl)-methyl-amino]-N-methyl-acetamid (unpolares Diastereomer) | 55 (394) |

### Beispiel 127:

### 2-[[4-(Dimethyl-amino)-1,4-diphenyl-cyclohexyl]-methyl-amino]-ethanol (unpolares Diastereomer)

### Stufe 1:

### 4[(4-Dimethylamino-1,4-diphenyl-cyclohexyl)-methyl-amino]-essigsäure-methylester

Die Titelverbindung aus Beispiel 8 (463 mg, 1.50 mmol) wurde in abs. DMF (10 mL) vorgelegt und mit Kaliumcarbonat (347 mg, 1.65 mmol) und Bromessigsäure-methylester (157 µL, 1.65 mmol) versetzt. Der Ansatz wurde 3 d bei Raumtemperatur gerührt und anschließend i. Vak. zur Trockene eingeengt. Der Rückstand wurde in Dichlormethan (50 mL) aufgenommen und mit Wasser (2 x 50 mL) und gesättigter NaCl-Lösung (50 mL) gewaschen, die organische Phase anschließend über Na₂SO₄ getrocknet und i. Vak. eingeengt. Der Rückstand wurde mittels Flash-Chromatographie mit Essigester/Methanol (9:1) gereinigt.
Ausbeute: 234 mg (41 %)
¹H-NMR (DMSO-d₆): 1.72 (4 H, m); 1.84 (6 H, s); 1.93 (3 H, s); 2.27 (4 H, m); 2.87 (2 H, m); 3.48 (3 H, s); 7.26 (2 H, m); 7.38 (8 H, m).

### Stufe 2:

### 2-[[4-(Dimethyl-amino)-1,4-diphenyl-cyclohexyl]-methyl-amino]-ethanol (unpolares Diastereomer)

Die Titelverbindung aus Stufe 1(228 mg, 0.60 mmol) wurde in abs. THF (10 mL) gelöst, mit LiAlH₄ (45 mg, 1.20 mmol) unter Argon versetzt und 3 h unter Rückfluss gekocht. Anschließend wurde der Ansatz auf Raumtemperatur abgekühlt, unter Eiskühlung mit THF (10 mL) und H₂O (5 mL) versetzt und 30 min nachgerührt. Der Ansatz wurde über eine Fritte mit Kieselgur filtriert und das Kieselgur mit Dichlormethan (50 mL) nachgewaschen. Die vereinigten Filtrate wurden i. Vak. eingeengt. Das Rohprodukt wurde mit Wasser (10 mL) versetzt und mit Dichlormethan (3 x 20 mL) extrahiert, die organische Phase anschließend über Na₂SO₄ getrocknet und i. Vak. eingeengt. Der Rückstand wurde mittels Flash-Chromatographie mit Essigester/Methanol (9:1→4:1) gereinigt.
Ausbeute: 174 mg (82 %)
Schmelzpunkt: 144-149 °C
¹H-NMR (DMSO-d₆): 1.73 (4 H, m); 1.84 (6 H, s); 1.96 (3 H, s); 2.09 (2 H, m); 2.27 (4 H, m); 3.23 (2 H, m); 4.14 (1 H, m); 7.25 (2 H, m); 7.38 (8 H, m).

### Beispiel 128:

### [4-[[4,6-Bis(dimethylamino)-[1,3,5]triazin-2-yl]-methyl-amino]-1,4-diphenyl-cyclohexyl]-dimethyl-amin (unpolares Diastereomer)

### Stufe 1:

### N-(4,6-Dichlor-[1,3,5]triazin-2-yl)-N,N',N'-trimethyl-1,4-diphenyl-cyclohexan-1,4-diamin

Cyanurchlorid (86 mg, 0.49 mmol) wurde in abs. THF (3 mL) vorgelegt, mit einer Lösung der Titelverbindung aus Beispiel 8 (150 mg, 0.49 mmol) in abs. THF (6 mL) und N-Ethyldiisopropylamin (80 µL, 0.49 mmol) versetzt und 16 h bei RT gerührt. Die Lösung wurde i. Vak. eingeengt, der Rückstand in Essigester (20 mL) aufgenommen und mit gesättigter NaHCO₃-Lösung (2 x 10 mL) und gesättigter NaCl-Lösung (10 mL) gewaschen. Die organische Phase wurde über Na₂SO₄ getrocknet und i. Vak. eingeengt. Das Rohprodukt wurde durch Flash-Chromatographie mit Essigester/MeOH (20:1) gereinigt.
Ausbeute: 67 mg (30 %)
¹³C-NMR (CDCl₃): 30.4, 31.4, 33.6, 38.0, 59.3, 66.4, 126.4, 126.7, 127.0, 127.1, 127.7, 128.2, 137.6, 143.1, 165.4, 168.0, 169.1

### Stufe 2:

### [4-[[4,6-Bis(dimethylamino)-[1,3,5]triazin-2-yl]-methyl-amino]-1,4-diphenyl-cyclohexyl]-dimethyl-amin (unpolares Diastereomer)

Die Titelverbindung aus Stufe 1 (57 mg, 0.12 mmol) wurde in 2M-Dimethylamin-Lösung in THF (2.0 mL, 4 mmol) gelöst und in der Mikrowelle 2 h bei 120 °C gerührt. Die Reaktionslösung wurde i. Vak. eingeengt, der verbliebene Rückstand in Essigester (10 mL) aufgenommen und mit gesättigter NaHCO₃-Lösung (2 x 5 mL) und gesättigter NaCl-Lösung (5 mL) gewaschen. Die organische Phase wurde über Na₂SO₄ getrocknet und i. Vak. eingeengt. Das Rohprodukt wurde durch Flash-Chromatographie mit Essigester/MeOH (20:1) gereinigt.
Schmelzpunkt: 195-197 °C
Ausbeute: 45 mg (76 %)
¹H-NMR (DMSO-d₆): 1.62 (2 H, m); 1.98 (6 H, s); 2.39 (2 H, m); 2.46 (2 H, m); 2.91 (12 H, s); 3.13 (3 H, s); 7.15 (1 H, m); 7.22 - 7.38 (9 H, m).

### Beispiel 130:

### 4-[[4-(Dimethyl-amino)-1,4-diphenyl-cyclohexyl]-methyl-amino]-butan-1-ol (polares Diastereomer)

### Stufe 1:

### N-(4-Dimethylamino-1,4-diphenyl-cyclohexyl)-N-methyl-bernsteinsäuretert-butylester

Die Titellverbindung aus Beispiel 131 (100 mg, 0.244 mmol) wurde in abs. Dichlormethan (5 mL) vorgelegt, mit Trifluoressigsäureanhydrid (135 µL, 0.976 mmol) versetzt und 10 min gerührt. Zu dem Ansatz wurde *tert*-Butanol (2 mL) gegeben und 30 min nachgerührt. Anschließend wurde der Ansatz mit 10%-iger NaOH versetzt und die Phasen getrennt. Die organische Phase wurde mit H₂O (1 x 10 mL) gewaschen, über Na₂SO₄ getrocknet und i. Vak. eingeengt.
Ausbeute: 80 mg (70 %)
¹H-NMR (DMSO-d₆): 1.38 (9 H, s); 1.53 (2 H, m); 1.78 (2 H, m); 1.92 (6 H, s); 2.37 (3 H, m); 2.62 (2 H, m); 2.93 (3 H, s), 7.11-7.27 (6 H, m); 7.36 (4 H, m).

### Stufe 2:

### 4-[[4-(Dimethyl-amino)-1,4-diphenyl-cyclohexyl]-methyl-amino]-butan-1-ol (polares Diastereomer)

Die Titelverbindung aus der Stufe 1 (836 mg, 1.8 mmol) wurde in abs. THF (15 mL) gelöst. LiAlH₄ (136 mg, 3.6 mmol) wurde unter Argon zugegeben, 2 h unter Rückfluss gekocht, auf Raumtemperatur abgekühlt und über Nacht gerührt. Unter Eiskühlung wurde zu dem Ansatz THF (2 mL) und H₂O (2 mL) gegeben und 30 min gerührt. Der Ansatz wurde über eine Fritte mit Kieselgur gegeben, das Kieselgur mit Dichlormethan (50 mL) gewaschen, die organischen Phasen vereinigt und i. Vak. eingeengt. Der Rückstand wurde durch Flash-Chromatograpie mit Chloroform/Methanol (9:1) gereinigt.
Ausbeute: 405 mg (59 %)
¹H-NMR (DMSO-d₆): 1.39 (4 H, m); 1.74 (3 H, m); 1.96 (6 H, s); 2.01 (3 H, s); 2.11 (2 H, m); 2.30 (3 H, m); 3.36 (2 H, m); 4.41 (1 H, m); 7.18 (2 H, m); 7.28 (8 H, m).

### Beispiel 131:

### 3-[[4-(Dimethyl-amino)-1,4-diphenyl-cyclohexyl]-methyl-carbamoyl]-propionsäure (polares Diastereomer)

Bernsteinsäureanhydrid (0.97 g, 9.27 mmol) wurde auf 130 °C erwärmt und geschmolzen. Anschließend wurde die Titelverbindung aus Beispiel 9 (1.00 g, 3.24 mmol) zugegeben und 7 h bei dieser Temperatur weiter erwärmt. Der Ansatz wurde durch Flash-Chromatographie mit Chloroform/Methanol (9:1→4:1 → 1:1 →1:2→Methanol) gereinigt.
Ausbeute: 1.08 g (81 %)
¹H-NMR (DMSO-d₆): 1.55 (2 H, m); 1.81 (2 H, m); 1.94 (6 H, s); 2.37 (4 H, m); 2.62 (2 H, m); 2.76 (1 H, m); 2.94 (3 H, s); 7.14 (3 H, m); 7.17 (2 H, m); 7.26 (1 H, m); 7.38 (4 H, m).

### Beispiel 146:

### [4-[[4-(4-Methoxy-phenoxy)-[1,3,5]triazin-2-yl]-methyl-amino]-1,4-diphenyl-cyclohexyl]-dimethyl-amin (polares Diastereomer)

### Stufe 1:

### N-(4-Chlor-[1,3,5]triazin-2-yl)-N,N',N'-trimethyl-1,4-diphenyl-cyclohexan-1,4-diamin

Eine Lösung der Titelverbindung aus Beispiel 9 (462 mg, 1.5 mmol), 2,4-Dichlor-1,3,5-triazin (225 mg, 1.5 mmol) und Diisopropylethylamin (248 µL, 1.5 mmol) in abs. THF (10 mL) wurde über Nacht bei RT gerührt. Anschließend wurde i. Vak. das Lösungsmittel entfernt, der verbliebene Rückstand in Essigester gelöst, mit ges. NaHCO₃-Lösung und ges. NaCl-Lösung gewaschen, über Na₂SO₄ getrocknet und durch Flash-Chromatographie mit Essigester/MeOH (9:1) gereinigt.
Ausbeute: 166 mg (26 %)
¹H-NMR (CDCl₃): 1.97 (4 H, m); 2.06 (6 H, s); 2.47 (4 H, bs); 3.01 (2 H, breit); 3.34 (3 H, s); 7.14 - 7.40 (10 H, m); 8.29 (1 H, s).

### Stufe 2:

### [4-[[4-(4-Methoxy-phenoxy)-[1,3,5]triazin-2-yl]-methyl-amino]-1,4-diphenyl-cyclohexyl]-dimethyl-amin (polares Diastereomer)

Die Titelverbindung der Stufe 1 (166 mg, 0.39 mmol), 4-Methoxyphenol (56 mg, 0.45 mmol) und Natriumhydrid (18 mg, 0.45 mmol, 60%-ige Dispersion in Mineralöl) wurden in abs. Dioxan (10 mL) bei RT 4 h gerührt. Anschließend wurde i. Vak. das Lösungsmittel entfernt, der verbliebene Rückstand in Essigester gelöst, mit ges. NaHCO₃-Lösung und ges. NaCl-Lösung gewaschen, über Na₂SO₄ getrocknet und durch Flash-Chromatographie mit Essigester/MeOH (4:1) gereinigt.
Ausbeute: 126 mg (63 %), poröser Feststoff
¹H-NMR (CDCl₃): 1.84 (4 H, m); 2.03 (6 H, s); 2.60 (4 H, breit); 3.23 (3 H, s); 3.80 (3 H, s); 6.87 - 7.38 (14 H, m); 8.37 (1 H, s).

### Beispiel 149:

### [4-[(Benzyl-methyl-amino)-methyl]-1,4-diphenyl-cyclohexyl]-dimethyl-amin

### Stufe 1:

### 4-Cyano-4-phenyl-heptandicarbonsäure-dimethylester

Phenylacetonitril (11.7 g, 100 mmol) und Methylacrylat (47 mL, 500 mmol) wurden in *ter.t-*Butanol (60 mL) vorgelegt und zum Sieden erhitzt. Anschließend wurde die Heizquelle entfernt. Das in *tert*.-Butanol (23 mL) gelöste Triton B (Benzyltrimethylammoniumhydroxid, 40%-ig in Methanol, 15.2 mL) wurde erst langsam, später zügig zugetropft. Nach dem Zutropfen wurde der Ansatz 4 h zum Sieden erhitzt. Über Nacht wurde die Reaktionsmischung auf Raumtemperatur abgekühlt. Zur Aufarbeitung wurde der Ansatz mit Toluol (100 mL) und Wasser (70 mL) versetzt, die organische Phase abgetrennt und mit Wasser (70 mL) und gesättigter NaCl-Lösung (50 mL) gewaschen. Nach dem Trocknen mit Na₂SO₄ wurde das Lösungsmittel destilliert. Die Reinigung erfolgte durch Kugelrohrdestillation bei einer Temperatur von ca. 235 °C. Das Produkt konnte als farblose, zähflüssige Substanz isoliert werden.
Ausbeute: 22. 5 g (75 %)
¹H-NMR (DMSO-d₆): 2.32 (8 H, m); 3.51 (6 H; s); 7.40 (5 H, m).
¹³C-NMR (DMSO-d₆): 22.47; 27.16; 39.28; 44.11; 113.82; 118.55; 120.83; 121.78; 129.10; 164.44.

### Stufe 2:

### 5-Cyano-2-oxo-5-phenyl-cyclohexancarbonsäure-methylester

4-Cyano-4-phenylheptandicarbonsäure-dimethylester (19.8 g, 68 mmol) wurde in trockenem Tetrahydrofuran (480 mL) gelöst. Anschließend wurde portionsweise Kalium-*tert*.-butylat (13.2 g, 120 mmol) zugegeben. Bei dieser Zugabe verfärbte sich die Reaktionsmischung orange. Danach wurde der Ansatz 5 h unter Rückfluss gekocht. Während des Kochens entstand eine braune Lösung. Über Nacht wurde die Reaktionsmischung auf Raumtemperatur abgekühlt. Unter Eiskühlung wurde 2.5N Essigsäure (230 mL) langsam zum Reaktionsgemisch getropft. Anschließend wurde der Ansatz mit Toluol (100 mL) versetzt, die organische Phase abgetrennt und mit gesättigter NaHCO₃-Lösung (3 x 100 mL), H₂O (3 x 50 mL) und NaCl-Lösung (1 x 100 mL) gewaschen. Nach dem Trocknen mit Na₂SO₄ wurde das Lösungsmittel i. Vak. abdestilliert. Es verblieb ein gelblicher Feststoff.
Ausbeute: 16.1 g (92 %)
Schmelzpunkt: 75-77 °C
¹H-NMR (DMSO-d₆): 2.23-2.74 (6 H, m); 3.74 (3 H; s); 7.35-7.60 (5 H, m); 12.08 (1 H, bs). ¹³C-NMR (DMSO-d₆): 26.95; 30.18; 34.04; 51.90; 94.79; 121.90; 125.46; 128.05; 128.85; 138.92; 169.95; 171.09.

### Stufe 3:

### 4-Oxo-1-phenyl-cyclohexancarbonitril

5-Cyano-2-oxo-5-phenylcyclohexancarbonsäure-methylester (16.1 g, 63 mmol) wurde in 10%-iger Schwefelsäure (218 mL) und konz. Essigsäure (502 mL) gelöst und 21 h bei 100 °C gerührt.
Zur Aufarbeitung wurde der Ansatz unter Eiskühlung vorsichtig mit Wasser (400 mL) verdünnt, mit Ethylacetat (3 x 100 mL) extrahiert, die organische Phase gründlich mit Wasser (6 x 100 mL), gesättigter NaHCO₃-Lösung (10 x 100 mL) und gesättigter NaCl-Lösung (1 x 100 mL) gewaschen. Nach dem Trocknen mit Na₂SO₄ wurde das Lösungsmittel i. Vak. abdestilliert.
Ausbeute: 8.91 g (72 %)
Schmelzpunkt: 106-107 °C
¹H-NMR (DMSO-d₆): 2.38-2.48 (6 H, m); 2.70 (2 H; m); 7.36 (1 H, m); 7.44 (2 H, m); 7.62 (2 H, m).
¹³C-NMR (DMSO-d₆): 35.31; 38.10; 42.33; 121.73; 125.65; 128.19; 129.02; 139.17; 208.79.

### Stufe 4:

### 8-Phenyl-1,4-dioxa-spiro[4.5]decan-8-carbonitril

Die Titelverbindung aus Stufe 3 (8.91 g, 44.73 mmol) wurde in Toluol (300 mL) aufgenommen und mit Ethylenglykol (6 mL, 106.8 mmol) versetzt. Nach Zugabe von p-Toluolsulfonsäure (0.128 g, 0.745 mmol) wurde der Ansatz 3.5 h am Wasserabscheider zum Sieden erhitzt. Der Verlauf der Reaktion wurde durch DC verfolgt.
Nach Abkühlung des Reaktionsansatzes wurde die Toluol-Lösung mit Wasser (5 x 60 mL) und gesättigter NaCl-Lösung (3 x 40 mL) extrahiert und über Na₂SO₄ getrocknet. Nach Entfernung des Lösungsmittels i. Vak. fiel das Ketal als gelber Feststoff an.
Ausbeute: 11.6 g (100 %)
Schmelzpunkt: 108-110 °C
¹H-NMR (DMSO-d₆): 1.86 (4 H, m); 2.01-2.30 (4 H; m); 3.92 (4 H, s); 7.38-7.53 (5 H, m). ¹³C-NMR (DMSO-d₆): 32.10; 34.07; 42.49; 63.86: 106.11; 122.14; 125.51; 128.16; 129.02; 139.90.

### Stufe 5:

### 8-Phenyl-1,4-dioxa-spiro[4.5]decan-8-carbonsäure

Die Titelverbindung aus Stufe 4 (10.9 g, 46.9 mmol) wurde in Ethylenglykol (92 mL) gelöst, mit NaOH (4.00 g, 100 mmol) versetzt und anschließend unter Rückfluss zum Sieden erhitzt. Nach 20 h war kein Nitril mehr nachweisbar. Zur Aufarbeitung wurde der Ansatz mit Eis (ca. 250 g) versetzt, mit Ether (90 mL) überschichtet und durch langsame Zugabe von halbkonz. HCl (118 mL) angesäuert. Die wässrige Phase wurde mit Ether (3 x 70 mL) extrahiert, die vereinigten organischen Extrakte mit gesättigter NH₄Cl-Lösung gewaschen (2 x 70 mL), über Na₂SO₄ getrocknet und i. Vak. zur Trockene eingeengt. Durch Umkristallisation des verbliebenen Rückstandes aus Toluol wurde die gewünschte Carbonsäure als kristalliner Feststoff gewonnen.
Ausbeute: 7.42 g (59 %)
Schmelzpunkt: 134-139 °C
¹H-NMR (DMSO-d₆): 1.64 (4 H, m); 1.91 (2 H; m); 2.41 (2 H, m); 3.86 (4 H, s); 7.36 (5 H, m); 12.52 (1 H, bs).
¹³C-NMR (DMSO-d₆): 31.51; 32.05; 49.19; 63.65: 107.23; 125.70; 126.94; 128.39; 142.82; 175.53.

### Stufe 6:

### 8-Phenyl-1,4-dioxa-spiro[4.5]decan-8-carbonsäure-benzyl-methyl-amid

Die Titelverbindung aus Stufe 5 (8.00 g, 30.48 mmol) wurde in Dichlormethan (240 mL) gelöst und bei 0 °C mit 1,3-Diisopropylcarbodiimid (4.44 g, 5.44 mL, 35.52 mmol) und 1-Hydroxy-1*H*-benzotriazol Hydrat (5.44 g, 35.5 mmol) versetzt. Der Reaktionsansatz wurde 5 min unter Eiskühlung gerührt und anschließend *N*-Benzylmethylamin (3.87 g, 4.12 mL, 32.0 mmol) hinzugegeben. Die Reaktionsmischung wurde 3 d bei Raumtemperatur gerührt. Zur Aufarbeitung wurde der Ansatz i. Vak. zur Trockene eingeengt. Der Rückstand wurde durch Flash-Chromatographie mit Cyclohexan/Ethylacetat (1:1) gereinigt.
Ausbeute: 7.31 g (66 %)
¹H-NMR (DMSO-d₆): 1.61 (4 H, m); 1.68 (4 H, m); 2.35 (3 H, m); 3.85 (6 H, s); 7.28 (10 H, br, m).

### Stufe 7:

### Benzyl-methyl-(8-phenyl-1,4-dioxa-spiro[4.5]dec-8-ylmethyl)-amin

Die Titelverbindung aus Stufe 6 (1.20 g, 3.28 mmol) wurde in abs. Tetrahydrofuran (160 mL) gelöst, LiAlH₄ (0.25 g, 6.59 mmol) unter Argon zugegeben und 5 h unter Rückfluss gerührt. Anschließend wurde der Ansatz auf Raumtemperatur abgekühlt und über Nacht gerührt. Unter Eiskühlung wurde der Ansatz mit THF (20 mL) und H₂O (20 mL) hydrolysiert und 30 min nachgerührt. Der Ansatz wurde über eine Fritte mit Kieselgur filtriert, mit THF und Dichlormethan (50 mL) nachgewaschen und i. Vak. eingeengt. Der Rückstand wurde mittels Flash-Chromatographie und Cyclohexan/Ethylacetat (1:1) gereinigt.
Ausbeute: 0.50 g (43 %)
¹H-NMR (DMSO-d₆): 1.35 (2 H, m); 1.38 (2 H, m); 1.72 (5 H, m); 2.20 (2 H, d); 2.48 (2 H, m); 3.22 (2 H, s); 3.84 (4 H, m); 7.25 (8 H, m), 7.44 (2 H d).

### Stufe 8:

### 4-[(Benzyl-methyl-amino)-methyl]-4-phenyl-cyclohexanon

Die Titelverbindung aus Stufe 7 (3.40 g, 9.67 mmol) wurde mit 5 %-iger Schwefelsäure (300 mL) versetzt und 48 h bei Raumtemperatur gerührt. Zur Aufarbeitung wurde der Reaktionsansatz mit Ether (100 mL) versetzt, die Phasen getrennt und die wässrige Phase mit Ether (2 x 100 mL) extrahiert. Anschließend wurde die wässrige Phase mit 5N NaOH basisch eingestellt und mit Dichlormethan (3 x 100 mL) extrahiert. Die organische Phase wurde über Na₂SO₄ getrocknet, filtriert und i. Vak. zur Trockene eingeengt.
Ausbeute: 2.74 g (92 %)
¹H-NMR (DMSO-d₆): 1.79 (3 H, s); 2.07 (2 H, m); 2.16 (5 H, m); 2.22 (1 H, m); 3.26 (2 H, s); 7.22 (6 H, m); 7.37 (2 H, t), 7.55 (2 H, d).

### Stufe 9:

### 4-[(Benzyl-methyl-amino)-methyl]-1-methylamino-4-phenyl-cyclohexancarbonitril

Zu einer auf 0 °C gekühlten Lösung von 4N Salzsäure (2.33 mL) und Methanol (1.40 mL) wurde 40 %-ige wässrige Methylaminlösung (5.40 mL, 42.7 mmol) und der Titelverbindung aus Stufe 8 (2.74 g, 8.91 mmol), gelöst in Methanol (10 mL), gegeben. Anschließend wurde die Reaktionsmischung mit Kaliumcyanid (1.40 g, 21.1 mmol) versetzt und 1 d bei Raumtemperatur gerührt. Zur Aufarbeitung wurde das Gemisch mit Wasser (30 mL) versetzt und mit Ether (3 x 50 mL) extrahiert. Die vereinigten organischen Phasen wurden mit Na₂SO₄ getrocknet, filtriert und i. Vak. eingeengt.
Ausbeute: 2.69 g (90 %)
¹H-NMR (DMSO-d₆): 1.11 (2 H, m); 1.68 (1 H, m); 1.72 (2 H, m); 1.78 (1 H, m); 1.86 (2 H, s); 1.92 (2 H, m); 2.22 (2 H, d). 2.28 (1 H, m); 2.38 (2 H, m); 2.67 (1 H, m); 3.17 (1 H, m); 3.29 (2 H, m); 7.25 (10 H, m).

### Stufe 10:

### {4-[(Benzyl-methyl-amino)-methyl]-1,4-diphenyl-cyclohexyl}-methyl-amin

Phenyllithium (12.9 mL, 23.2 mmol, 1.8 M in Dibutylether) wurde unter Argon vorgelegt, tropfenweise mit der Titelverbindung aus Stufe 9 (2.69 g, 7.74 mmol) in THF (15 mL) versetzt und die Reaktionslösung 1 h unter Rückfluss gerührt. Unter Eisbadkühlung wurde der Reaktionsansatz mit gesättigter NH₄Cl-Lösung (27 mL) hydrolysiert und die Phasen getrennt. Die wässrige Phase wurde mit Ether (3 x 50 mL) extrahiert. Die vereinigten organischen Phasen wurde über Na₂SO₄ getrocknet, filtriert und i. Vak. zur Trockene eingeengt. Der Rückstand wurde mittels Chromatotron und Dichlormethan → Dichlormethan/Methanol (9:1) → Methanol getrennt. Es wurden 1.20 g Keton isoliert. Das gewünschte Produkt wurde als Diastereomergemisch erhalten und wurde als solches weiter umgesetzt.
Ausbeute: 0.360 g (12 %)
¹H-NMR (DMSO-d₆): 1.75 (1 H, m); 1.79 (3 H, s); 1.92 (1 H, m); 2.02 (3 H, m); 2.17 (6 H, m); 2.46 (1 H, m); 2.61 (2 H, m); 7.25 (13 H, m); 7.54 (2 H, m).

### Stufe 11:

### [4-[(Benzyl-methyl-amino)-methyl]-1,4-diphenyl-cyclohexyl]-dimethyl-amin

Eine Lösung der Titelverbindung aus Stufe 10 (Diastereomerengemisch) (0.350 g, 0.878 mmol) und Formalin (1.23 mL, 37 %-ige wässrige Lösung) in Acetonitril (15 mL) wurde portionsweise mit Natriumcyanoborhydrid (0.250 g, 3.86 mmol) versetzt und 45 min bei Raumtemperatur gerührt. Anschließend wurde konz. Essigsäure bis zur neutralen Reaktion zugegeben und 45 min bei Raumtemperatur nachgerührt. Zur Aufarbeitung wurde das Lösungsmittel i. Vak. entfernt, der Rückstand in 2N NaOH (40 mL) aufgenommen und anschließend mit Ether (3 x 40 mL) extrahiert. Die organische Lösung wurde über Na₂SO₄ getrocknet, filtriert und i. Vak. eingeengt. Der verbliebene Rückstand wurde mittels Chromatotron und mit Cyclohexan/Ethylacetat 1:1 gereinigt. Eine Trennung der Diastereomeren konnte nicht erreicht werden.
Ausbeute: 70 mg (19 %)
¹H-NMR (DMSO-d₆): 1.60 (4 H, m); 1.72 (3 H, s); 1.82 (6 H, s); 2.14 (2 H, m); 2.49 (4 H, s); 3.19 (2 H, s); 6.93 (2 H, m); 7.21 (5 H, m); 7.40 (8 H, m).

### Beispiel 153:

### [4-[[4-(Benzylamino)-[1,3,5]triazin-2-yl]-methyl-amino]-1,4-diphenyl-cyclohexyl]-dimethyl-amin (polares Diastereomer)

Die Titelverbindung aus Beispiel 146, Stufe 1 (100 mg, 0.236 mmol), Benzylamin (55 µL, 0.5 mmol) und Diisopropylethylamin (50 µL, 0.3 mmol), gelöst in abs. THF (2.0 mL), wurden bei 70 °C in einem geschlossenen Gefäß 5 h gerührt. Anschließend wurde i. Vak. das Lösungsmittel entfernt, der verbliebene Rückstand in Dichlormethan gelöst, mit ges. NaHCO₃-Lösung gewaschen, über Na₂SO₄ getrocknet und durch Flash-Chromatographie mit Essigester/MeOH (4:1→1:1) gereinigt. Das Produkt enthielt noch Benzylamin, das i. Vak. bei 90 °C entfernt wurde.
Ausbeute: 82 mg (70 %), Öl
¹H-NMR (CDCl₃): 1.77 (4 H, m); 2.02 (6 H, s); 2.37 (2 H, m); 2.97 (2 H, breit); 3.28 (3 H, s); 4.38 (2 H, s); 6.01 (1 H, s); 7.12 - 7.40 (15 H, m); 8.00 (1 H, s).

In Anlehnung an eine Vorschrift wie im Beispiel 153 beschrieben wurden mit der Ausnahme, daß die in der Tabelle 1-10 angeführten Amine verwendet werden, folgende Verbindungen erhalten.

**Tabelle 1-10:**

| **BSP.- Nr.** | **Amin** | **Produkt** | **Cy (%) / MS (m/z)** |
|---|---|---|---|
| **154** | Cyclohexylamin | Dimethyl-[4-[methyl-(4-piperidin-1-yl-[1,3,5]triazin-2-yl)-amino]-1,4-diphenyl-cyclohexyl]-amin (polares Diastereomer) | 71 (471) |
| **155** | n-Butylamin | [4-[(4-Butylamino-[1,3,5]triazin-2-yl)-methyl-amino]-1,4-diphenyl-cyclohexyl]-dimethyl-amin (polares Diastereomer) | 68 (459) |
| **156** | Anilin | [4-[(4-Anilino-[1,3,5]triazin-2-yl)-methyl-amino]-1,4-diphenyl-cyclohexyl]-dimethyl-amin (polares Diastereomer) | 59 (479) |
| **157** | Isopropylmethylamin | [4-[[4-(Isopropyl-methyl-amino)-[1,3,5]triazin-2-yl]-methyl-amino]-1,4-diphenyl-cyclohexyl]-dimethyl-amin (polares Diastereomer) | 75 (459) |
| **158** | t-Butylamin | [4-[[4-(Tert-butylamino)-[1,3,5]triazin-2-yl]-methyl-amino]-1,4-diphenyl-cyclohexyl]-dimethyl-amin (polares Diastereomer) | 64 (459) |

### Beispiel 163:

### [4-[(Butyl-methyl-amino)-methyl]-1,4-diphenyl-cyclohexyl]-dimethyl-amin (unpolares Diastereomer)

### Stufe 1:

### 8-Phenyl-1,4-dioxa-spiro[4.5]decan-8-carbonsäure-butyl-methylamid

Die Titelverbindung aus Beispiel 149, Stufe 5 (6.50 g, 24.8 mmol) wurde in Dichlormethan (200 mL) gelöst und bei 0 °C mit Diisopropylcarbodiimid (3.60 g, 4.41 mL, 28.8 mmol) und 1-Hydroxy-1*H*-benzotriazol Hydrat (4.41 g, 28.8 mmol) versetzt. Der Reaktionsansatz wurde 5 min unter Eiskühlung gerührt und anschließend *N*-Methylbutylamin (2.34 g, 3.08 mL, 26.0 mmol) hinzugegeben. Die Reaktionsmischung wurde 2 d bei Raumtemperatur gerührt. Zur Aufarbeitung wurde der Ansatz i. Vak. zur Trockene eingeengt. Der Rückstand wurde durch Flash-Chromatographie mit Cyclohexan/Ethylacetat (2:1) gereinigt.
Ausbeute: 3.50 g (43 %)
¹H-NMR (DMSO-d₆): 0.95 (6 H, m); 1.39 (2 H, s); 1.80 (2 H, m); 1.85 (6 H, m); 2.24 (2 H, m); 2.51 (1 H, m); 3.10 (1 H, br m).); 3.84 (4 H, s); 7.23 (3 H, m); 7.34 (2 H, m).

### Stufe 2:

### Butyl-methyl-(8-phenyl-1,4-dioxa-spiro[4.5]dec-8-ylmethyl)-amin

Die Titelverbindung aus Stufe 1 (3.50 g, 10.6 mmol) wurde in abs. Tetrahydrofuran (400 mL) gelöst, LiAlH₄ (0.66 g, 17.5 mmol) unter Argon zugegeben und 5 h unter Rückfluss gerührt. Anschließend wurde der Ansatz auf Raumtemperatur abgekühlt und über Nacht gerührt. Unter Eiskühlung wurde der Ansatz mit THF (20 mL) und H₂O (20 mL) hydrolysiert und 30 min nachgerührt. Der Ansatz wurde über eine Fritte mit Kieselgur filtriert, mit THF und Dichlormethan (50 mL) nachgewaschen und i. Vak. eingeengt. Der Rückstand wurde mittels Flash-Chromatographie und Cyclohexan/Ethylacetat (9:1→ 1:1) gereinigt.
Ausbeute: 2.50 g (76 %)
¹H-NMR (DMSO-d₆): 0.77 (3 H, t); 1.19 (6 H, m); 1.52 (2 H, m); 1.77 (2 H, m); 1.83 (3 H, s); 2.05 (2 H, m); 2.18 (2 H, m); 2.31 (2 H, s); 3.84 (4 H, br m); 7.19 (1 H, m); 7.33 (4 H, m).

### Stufe 3:

### 4-[(Butyl-methyl-amino)-methyl]-4-phenyl-cyclohexanon

Die Titelverbindung aus Stufe 2 (2.50 g, 7.8 mmol) wurde mit 5%-iger Schwefelsäure (300 mL) versetzt und 48 h bei Raumtemperatur gerührt. Zur Aufarbeitung wurde der Reaktionsansatz mit Ether (100 mL) versetzt, die Phasen getrennt und die wässrige Phase mit Ether (2 x 100 mL) extrahiert. Anschließend wurde die wässrige Phase mit 5N NaOH basisch eingestellt und mit Dichlormethan (3 x 100 mL) extrahiert. Die organische Phase wurde über Na₂SO₄ getrocknet, filtriert und i. Vak. zur Trockene eingeengt.
Ausbeute: 1.53 g (73 %)
¹H-NMR (DMSO-d₆): 0.78 (3 H, t); 1.15 (4 H, br, m); 1.87 (3 H, s); 1.93 (2 H, m); 2.13 (6 H, br m); 2.45 (4 H, m); 7.25 (1 H, t); 7.37 (2 H, t); 7.49 (2 H, d).

### Stufe 4:

### 4-[(Butyl-methyl-amino)-methyl]-1-methylamino-4-phenyl-cyclohexancarbonitril

Zu einer auf 0 °C gekühlten Lösung von 4N Salzsäure (1.50 mL) und Methanol (0.89 mL) wurde 40 %-ige wässrige Methylaminlösung (3.42 mL, 27 mmol) und die Titelverbindung aus Stufe 3 (1.54 g, 5.60 mmol), gelöst in Methanol (5 mL), gegeben. Anschließend wurde die Reaktionsmischung mit Kaliumcyanid (0.901 g, 13.4 mmol) versetzt und 3 d bei Raumtemperatur gerührt. Zur Aufarbeitung wurde das Gemisch mit Wasser (50 mL) versetzt und mit Ether (3 x 100 mL) extrahiert. Die vereinigten organischen Phasen wurden mit Na₂SO₄ getrocknet, filtriert und i. Vak. eingeengt.
Ausbeute: 1.76 g (100 %)
¹H-NMR (DMSO-d₆): 0.77 (3 H, m); 1.07 (5 H, m); 1.68 (3 H, m); 1.77 (1 H, s); 1.84 (1 H, m); 1.92 (2 H, m); 2.03 (1 H, m); 2.12 (2 H, m); 2.21 (2 H, m); 2.31 (3 H, m); 2.43 (1 H, m); 2.63 (1 H, m); 7.19 (1 H, m); 7.37 (4 H, m).

### Stufe 5:

### {4-[(Butyl-methyl-amino)-methyl]-1,4-diphenyl-cyclohexyl}-methyl-amin

Phenyllithium (9.33 mL, 16.8 mmol, 1.8 M in Dibutylether) wurde unter Argon vorgelegt, tropfenweise mit der Titelverbindung aus Stufe 4 (1.76 g, 5.61 mmol) in Ether (15 mL) versetzt und die Reaktionslösung 1 h bei 50 °C gerührt. Unter Eisbadkühlung wurde der Reaktions-ansatz mit gesättigter NH₄Cl-Lösung (100 mL) hydrolysiert und die Phasen getrennt. Die wässrige Phase wurde mit Ether (3 x 50 mL) extrahiert. Die vereinigten organischen Phasen wurde über Na₂SO₄ getrocknet, filtriert und i. Vak. zur Trockene eingeengt. Der Rückstand wurde mittels Chromatotron und Dichlormethan getrennt. Ausbeute: 0.400 g (20 %), unpolares Diastereomer
¹H-NMR (DMSO-d₆): 0.71 (3 H, t); 1.05 (5 H, m); 1.59 (3 H, m); 1.76 (6 H, s); 2.01 (6 H, m); 2.40 (2 H, br s); 7.19 (2 H, m); 7.34 (6 H, m); 7.47 (2 H, d).
Ausbeute: 0.170 g (9 %), polares Diastereomer
¹H-NMR (DMSO-d₆): 0.76 (3 H, t); 1.13 (4 H, m); 1.37 (2 H, m); 1.75 (4 H, s); 1.86 (3 H, m); 2.06 (6 H, m); 2.41 (2 H, s); 3.17 (1 H, s); 7.13 (2 H, m); 7.26 (6 H, m); 7.38 (2 H, m).

### Stufe 6:

### [4-[(Butyl-methyl-amino)-methyl]-1,4-diphenyl-cyclohexyl]-dimethyl-amin (unpolares Diastereomer)

Eine Lösung der Titelverbindung aus Stufe 6 (unpolares Diastereomer) (0.400 g, 1.1 mmol) und Formalin (1.54 mL, 37 %-ige wässrige Lösung) in Acetonitril (20 mL) wurde portionsweise mit Natriumcyanoborhydrid (0.313 g, 4.84 mmol) versetzt und 45 min bei Raumtemperatur gerührt. Anschließend wurde konz. Essigsäure bis zur neutralen Reaktion zugegeben und 45 min bei Raumtemperatur nachgerührt. Zur Aufarbeitung wurde das Lösungsmittel i. Vak. entfernt, der Rückstand in 2N NaOH (40 mL) aufgenommen und anschließend mit Ether (3 x 40 mL) extrahiert. Die organische Phase wurde über Na₂SO₄ getrocknet, filtriert und i. Vak. eingeengt.
Der Rückstand wurde mittels Chromatotron und Dichlormethan → Methanol gereinigt.
Ausbeute: 220 mg (53 %)
¹H-NMR (DMSO-d₆): 0.70 (3 H, t); 0.99 (4 H, m); 1.42 (2 H, m); 1.58 (2 H, m); 1.75 (3 H, s); 1.86 (6 H, s); 1.95 (2 H, m); 2.16 (4 H, m); 2.32 (2 H, m); 7.18 (1 H, m); 7.38 (9 H, m).

### Beispiel 164:

### [4-[(Butyl-methyl-amino)-methyl]-1,4-diphenyl-cyclohexyl]-dimethyl-amin (polares Diastereomer)

Eine Lösung der Titelverbindung aus Beispiel 163, Stufe 6 (polares Diastereomer) (0.170 g, 0.47 mmol) und Formalin (0.66 mL, 37 %-ige wässrige Lösung) in Acetonitril (8.2 mL) wurde portionsweise mit Natriumcyanoborhydrid (0.134 g, 2.07 mmol) versetzt und 45 min bei Raumtemperatur gerührt. Anschließend wurde konz. Essigsäure bis zur neutralen Reaktion zugegeben und 45 min bei Raumtemperatur gerührt. Zur Aufarbeitung wurde das Lösungsmittel i. Vak. entfernt, der Rückstand in 2N NaOH (40 mL) aufgenommen und anschließend mit Ether (3 x 40 mL) extrahiert. Die organische Phase wurde über Na₂SO₄ getrocknet, filtriert und i. Vak. eingeengt.
Der Rückstand wurde mittels Chromatotron und Dichlormethan → Methanol gereinigt.
Ausbeute: 75 mg (41 %)
¹H-NMR (DMSO-d₆): 0.77 (3 H, t); 1.18 (5 H, m); 1.51 (2 H, m); 1.76 (5 H, m); 1.94 (6 H, s); 2.04 (3 H, m); 2.27 (2 H, m); 2.40 (2 H, s); 7.23 (10 H, m).

### Nephelometrische Löslichkeitsuntersuchung (Phosphatpuffer pH 7.4):

Diese Methode untersucht die Löslichkeit einer Substanz bei festgelegten Konzentrationen (1 µM, 3 µM, 10 µM, 30 µM und 100 µM) in 10 mM Phosphatpufferlösung bei pH 7.4.
Initial wird eine 10 mM Lösung der Substanzen in DMSO benötigt, aus der 100-fach Stammlösungen der oben genannten Konzentrationslevel wiederum in DMSO hergestellt werden, die finale DMSO Konzentration im Versuchsansatz beträgt 1% (v/v). Das Experiment wird in mehrfach Bestimmung durchgeführt. Nach Zugabe der DMSO Stammlösungen zum Puffer, wird der Ansatz 2h bei 37°C inkubiert, bevor eine Absorptionsbestimmung bei 620 nm stattfindet. Steigt die Absorption der Proben über die der reinen Puffer/DMSO Lösung an, so gilt dies als Indikator für eine Präzipitatbildung. Die untere Löslichkeitsgrenze ("lower bound") ist die Konzentration, die derjenigen mit erster Präzipitatbildung vorangegangen ist (z.B. 3 µM, wenn Präzipitatbildung bei 10 µM detektiert wurde).

### Untersuchungen zur Wirksamkeit der erfindungsgemäßen Verbindungen

### Messung der ORL 1-Bindung

Die Verbindungen wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl2 und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschließende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einigen Fällen wurde auf die Bestimmung des Ki-Wertes verzichtet und nur die Hemmung bei einer Testkonzentration von 1 µM bestimmt.

### Messung der kappa-Bindung

Die Bestimmung erfolgte in einem homogenen Ansatz in Mikrotiterplatten. Hierzu wurden Verdünnungsreihen der jeweils zu prüfenden Substanzen mit einer Rezeptormembranpräparation (7 µg Protein pro 250 µl Inkubationsansatz) von CHO-K1-Zellen, welche den humanen κ-Opiatrezeptor exprimieren in Gegenwart von 1 nmol/l des radioaktiven Liganden [³H]-Cl-977 sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 100 µmol/l Naloxon zugegeben. Nach Beendigung der neunzigminütigen Inkubationszeit wurden die Mikrotiterplatten für 20 Minuten bei 500 Umdrehungen/Minute abzentrifugiert und die Radioaktivität in einem β-Counter (Microbeta-Trilux 1450, Firma PerkinElmer Wallac, Freiburg, Deutschland) vermessen. Es wurde die prozentuale Verdrängung des radioaktiven Liganden aus seiner Bindung zum humanen κ-Opiatrezeptor bei einer Konzentration der Prüfsubstanzen von 1 µmol/l bestimmt und als prozentuale Hemmung (%Hemmung) der spezifischen Bindung angegeben. Ausgehend von der prozentualen Verdrängung durch unterschiedliche Konzentrationen der zu prüfenden Verbindungen können IC₅₀ Hemmkonzentrationen berechnet werden, die eine 50-prozentige Verdrängung des radioaktiven Liganden bewirken. Durch Umrechnung mittels der Cheng-Prusoff-Beziehung können daraus die Kᵢ-Werte für die Prüfsubstanzen berechnet werden.
Die Ergebnisse sind in nachfolgender Tabelle zusammengefasst:

| Bsp. | % Hemmung (ORL1) [1 µM] | Ki(ORL1) Mittel [µM] | % Hemmung (µ) [1 µM] | Ki (µ) Mittel [µM] |
|---|---|---|---|---|
| 1 | 26 | n.d. | 46 | n.d. |
| 2 | 9 | n.d. | 22 | n.d. |
| 3 | 81 | n.d. | 0 | n.d. |
| 4 | 33 | 0,94 | 38 | n.d. |
| 5 | 37 | 0,635 | 82 | 0,0705 |
| 6 | 51 | 0,18 | 83 | 0,049 |
| 7 | 34 | 0,99 | 48. | 1,16 |
| 8 | 14 | n.d. | 30 | n.d. |
| 9 | 80 | 0,01775 | 49 | 0,64 |
| 10 | 18 | n.d. | 34 | n.d. |
| 11 | 87 | 0,006 | 59 | 0,6 |
| 12 | 53 | 0,365 | 86 | 0,0805 |
| 13 | 36 | 1,31 | 66 | 0,155 |
| 14 | 17 | n.d. | 67 | n.d. |
| 15 | 83 | n.d. | 98 | n.d. |
| 16 | 18 | n.d. | 65 | n.d. |
| 17 | 17 | 1,17 | 61 | 0,14 |
| 18 | 95 | 0,0027 | 100 | 0,00125 |
| 19 | 94 | 0,017 | 96 | 0,0475 |
| 20 | 92 | 0,011 | 101 | 0,0011 |
| 21 | 43 | 0,49 | 57 | 0,78333 |
| 22 | 43 | n.d. | 92 | n.d. |
| 23 | 13 | n.d. | 15 | n.d. |
| 24 | 82 | 0,063 | 97 | 0,01425 |
| 25 | 60 | 0,19 | 91 | 0,0154 |
| 26 | 76 | n.d. | 69 | n.d. |
| 27 | 95 | 0,0049 | 98 | 0,00805 |
| 28 | 30 | 1,01 | 36 | 0,895 |
| 29 | 70 | 0,09 | 98 | 0,0015 |
| 30 | 93 | 0,0016 | 99,5 | 0,0022 |
| 31 | 60 | 0,0955 | 90 | 0,022 |
| 32 | 65 | 0,051 | 81 | 0,052 |
| 33 | 9 | 3,955 | 42 | 1,11 |
| 34 | 27 | 1,89 | 81 | 0,215 |
| 35 | 29 | 0,99333 | n.d. | 0,41 |
| 36 | 30 | n.d. | n.d. | n.d. |
| 37 | 66 | 0,1025 | n.d. | 2,455 |
| 38 | 35 | n.d. | 29 | n.d. |
| 39 | 73 | 0,0535 | 93 | 0,555 |
| 40 | 84 | 0,0255 | 46 | 0,445 |
| 41 | 19 | n.d. | 42 | 4,11 |
| 42 | 28 | 1,395 | 101 | 0,0018 |
| 43 | 35 | 0,755 | 58 | 0,1305 |
| 44 | 68 | 0,0775 | 96 | 0,01335 |
| 45 | 72 | 0,0485 | 99 | 0,00124 |
| 46 | 49 | 0,72 | 84 | 0,188 |
| 47 | 39 | 1,605 | 65 | 0,755 |
| 48 | 22 | 1,25 | 48 | 0,9 |
| 49 | 97 | 0,00155 | 93 | 0,0615 |
| 50 | 58 | 0,1045 | 69 | 0,12 |
| 51 | 91 | 0,00128 | 90 | 0,044 |
| 52 | 87 | 0,01 | 93 | 0,018 |
| 53 | 61 | n.d. | 18 | n.d. |
| 54 | 36 | n.d. | 61 | n.d. |
| 56 | 22 | n.d. | 56 | n.d. |
| 57 | 43 | n.d. | 80 | n.d. |
| 58 | 52 | 0,425 | 71 | 0,115 |
| 59 | 69 | 0,175 | 80 | 0,034 |
| 60 | 47 | 0,53 | 77 | 0,107 |
| 61 | 56 | 0,375 | 84 | 0,032 |
| 62 | 49 | 0,0895 | 85 | 0,0475 |
| 63 | 60 | 0,087 | 91 | 0,0066 |
| 64 | 83 | 0,0345 | 96 | 0,0053 |
| 65 | 49 | 0,435 | 74 | 0,0925 |
| 66 | 35 | 4,01 | 18 | 10,23 |
| 67 | 31 | n.d. | 38 | 5,205 |
| 68 | 15 | n.d. | 43 | n.d. |
| 69 | 50 | 0,20667 | 40 | 0,65 |
| 70 | 28 | n.d. | 77 | n.d. |
| 71 | 38 | 0,36 | 55 | 1,19 |
| 72 | 58 | 0,115 | 40 | 1,695 |
| 73 | 45 | 0,165 | 84 | 0,027 |
| 74 | 67 | 0,057 | 52 | 0,66 |
| 75 | 24 | 2,955 | 34 | 0,67 |
| 76 | 55 | 0,295 | 83 | 0,18 |
| 77 | 98 | 0,00071 | 100 | 0,0004 |
| 78 | 39 | 0,73 | 67 | 0,385 |
| 79 | 96 | n.d. | 95 | n.d. |
| 80 | 91 | n.d. | 99 | n.d. |
| 81 | 82 | n.d. | 91 | n.d. |
| 82 | 53 | n.d. | 78 | n.d. |
| 83 | 47 | n.d. | 82 | n.d. |
| 84 | 72 | n.d. | 97 | n.d. |
| 85 | 99 | 0,00081 | n.d. | 0,115 |
| 86 | 35 | n.d. | n.d. | n.d. |
| 87 | 53 | n.d. | n.d. | n.d. |
| 88 | 54 | n.d. | n.d. | n.d. |
| 89 | 24 | 1,075 | n.d. | 0,325 |
| 90 | 44 | 0,525 | n.d. | 1,055 |
| 91 | 98 | 0,00535 | n.d. | 0,74 |
| 92 | 97 | 0,015 | n.d. | 0,00635 |
| 93 | 84 | 0,235 | n.d. | 0,045 |
| 94 | 68 | n.d. | 83 | n.d. |
| 95 | 32 | n.d. | 49 | n.d. |
| 96 | 64 | 0,108 | 62 | 0,235 |
| 97 | 18 | n.d. | 32 | n.d. |
| 98 | 91 | n.d. | 101 | n.d. |
| 99 | 97,5 | 0,00054 | 100 | 0,0012 |
| 100 | 85 | 0,21 | 97 | 2,4 |
| 101 | 93 | 0,00245 | 79 | 0,063 |
| 102 | 18 | n.d. | 37 | 3,73 |
| 103 | 25 | n.d. | 64 | n.d. |
| 104 | 67 | 0,033 | 93 | 0,01 |
| 105 | 93 | 0,00065 | 101 | 0,0012 |
| 106 | 87 | 0,02 | 98 | 0,435 |
| 107 | 19 | 1,485 | 38 | 1,995 |
| 108 | 53 | n.d. | 89 | n.d. |
| 109 | 60 | n.d. | 43 | n.d. |
| 110 | 38 | 0,62 | 78 | 0,155 |
| 111 | 27 | 1,055 | 60 | 0,42 |
| 112 | 47 | n.d. | n.d. | n.d. |
| 113 | 48 | n.d. | n.d. | n.d. |
| 114 | 91 | 0,01385 | n.d. | 0,13 |
| 115 | 24 | 1,165 | n.d. | 1,66667 |
| 116 | 85 | 0,021 | n.d. | 0,014 |
| 117 | 76 | 0,00013 | n.d. | 0,00035 |
| 118 | 52 | n.d. | n.d. | n.d. |
| 119 | 89 | 0,00143 | n.d. | 0,039 |
| 120 | 36 | 0,465 | n.d. | 2,9 |
| 121 | 70 | 0,23 | n.d. | 1,425 |
| 122 | 83 | 0,0074 | n.d. | 0,17667 |
| 123 | 65 | n.d. | n.d. | 2,595 |
| 124 | 23 | n.d. | n.d. | 0,735 |
| 125 | 35 | 2,05 | n.d. | 0,255 |
| 126 | 23 | n.d. | n.d. | n.d. |
| 127 | 18 | n.d. | 28 | n.d. |
| 128 | 15 | 0,93 | 46 | 0,255 |
| 129 | 67 | n.d. | 33 | n.d. |
| 130 | 88 | 0,018 | 62 | 0,415 |
| 131 | 37 | n.d. | 22 | 9,02 |
| 132 | 52 | 0,19 | 21 | 1,58 |
| 133 | 34 | n.d. | 37 | |

| | | | | |
|---|---|---|---|---|
| n.d.- nicht bestimmt | | | | |

Untersuchung der pharmakologischen Eigenschaften der Beispielverbindungen

### Chung-Modell: Mononeuropathieschmerz nach spinaler Nervenligatur

Tiere: Männliche Sprague Dawley Ratten (140-160g), von einem kommerziellen Züchter (Janvier, Genest St. Isle, Frankreich), wurden unter einem 12:12h Licht-Dunkel Rhythmus gehalten. Die Tiere wurden mit Futter und Leitungswasser ad libitum gehalten. Zwischen der Lieferung der Tiere und der Operation wurde eine Pause von einer Woche eingehalten. Die Tiere wurden nach Operation über einen Zeitraum von 4-5 Wochen mehrfach getestet, wobei eine Auswaschzeit von mindestens einer Woche eingehalten wurde.

Modellbeschreibung: Unter Pentobarbital Narkose (Narcoren®, 60 mg/kg i.p., Merial GmbH, Hallbergmoos, Deutschland), wurden die linken L5, L6 Spinalnerven exponiert, indem ein Stück des paravertebralen Muskels und ein Teil des linken spinalen Prozesses des L5 lumbalen Wirbelkörpers entfernt wurden. Die Spinalnerven L5 und L6 wurden vorsichtig isoliert und mit einer festen Ligatur abgebunden (NC-silk black,USP 5/0, metric 1, Braun Melsungen AG, Melsungen, Deutschland) (Kim and Chung 1992). Nach Ligatur wurden Muskel und benachbarte Gewebe zugenäht und die Wunde mittels Metallklammern geschlossen.

Nach einer Woche Erholungszeit wurden die Tiere zur Messung der mechanischen Allodynie in Käfige mit einem Drahtboden gesetzt. An ipsi- und/oder kontralateraler Hinterpfote wurde die Wegziehschwelle mittels eines elektronischen von Frey Filamentes (Somedic AB, Malmö, Schweden) bestimmt. Der Median von fünf Stimulierungen ergab einen Datenpunkt. Die Tiere wurden 30 min vor und zu verschiedenen Zeiten nach Applikation von Testsubstanz- oder Vehikellösung getestet. Die Daten wurden als % maximal mögliche Wirkung (%MPE) aus den Vortesten der Einzeltiere (=0%MPE) und den Testwerten einer unabhängigen Sham-Kontrollgruppe (=100%MPE) bestimmt. Alternativ wurden die Wegziehschwellen in Gramm dargestellt.

Statistische Auswertung: ED₅₀ Werte und 95% Vertrauensbereiche wurden über semilogarithmische Regressionsanalyse zum Zeitpunkt der maximalen Wirkung bestimmt. Die Daten wurden über eine Varianzanalyse mit wiederholten Messungen sowie einer post hoc Analyse nach Bonferroni analysiert. Die Gruppengröße betrug üblicherweise n=10.

Referenzen: Kim, S.H. and Chung,J.M., An experimental model for peripheral neuropathy produced by segmental spinal nerve ligation in the rat, Pain, 50 (1992) 355-363.

Die Ergebnisse sind in nachfolgender Tabelle (*Chung Modell*) zusammengefasst:

| Bsp.-Nr. | MPE (%), (Dosis in µg/kg, Ratte, i.v.) |
|---|---|
| 11 | 25 (100) |
| 51 | 29 (100) |
| 30 | 16 (100) |

Die früheren erfindungsgemäßen Verbindungen vom Typ E mit W = -NHMe oder -NMe₂ (Bsp. 9, 11 und 13) wurden mit entsprechenden Verbindungen vom Typ E mit W = -OH (V-1 und V-2) verglichen:

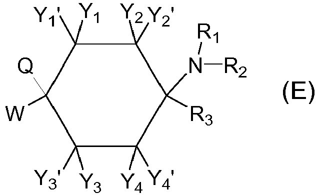

| Bsp. | W | Q | R₃ | Ki (µ)/Ki (ORL1) | Ki (kappa)/Ki (ORL1) | Ki (ORL1) Mittel [µM] | Ki (µ) Mittel [µM] | Ki (kappa) Mittel [µM] |
|---|---|---|---|---|---|---|---|---|
| 9 | 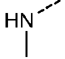 | 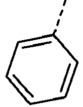 | 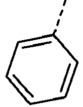 | 36 | 65 | 0,018 | 0,640 | 1,170 |
| 11 | 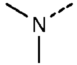 | 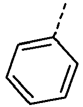 | 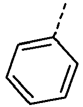 | 100 | 193 | 0,006 | 0,600 | 1,160 |
| V-1: | 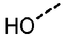 | 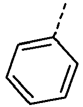 | 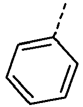 | 0,7 | 0,8 | 2,92 | 1,89 | 2,24 |
| 13 | 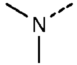 | 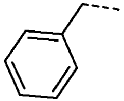 | 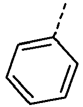 | 0,1 | 2,9 | 1,310 | 0,155 | 3,745 |
| V-2: | 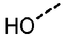 | 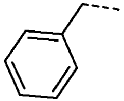 | 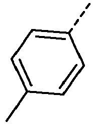 | 0,003 | 1,46 | 1,000 | 0,003 | 1,460 |

Wie die vorstehenden Vergleichsdaten belegen, weisen die erfindungsgemäßen Verbindungen (W= -NHMe oder -NMe₂) im Vergleich zu den strukturell ähnlichen Substanzen (W=-OH) eine höhere Selektivität gegenüber dem kappa-Opioidrezeptor (definiert als 1/[K_{i(ORL1)}/ Kᵢ₍ₖₐₚₚₐ₎]) auf. Darüber hinaus weisen die erfindungsgemäßen Substanzen bei günstigem Verhältnis der ORL1/µ-Affinität auch eine höhere Selektivität gegenüber dem µ-Opioidrezeptor (definiert als 1/[K_{i(ORL1)}/K_{i(µ)}]) auf.

Die (früheren) erfindungsgemäßen Verbindungen vom Typ 1 mit n = 0, X = -NMe und Q = Phenyl (Bsp. 27, 30, 31, 32, 49, 85 und 92) wurden mit Verbindungen vom Typ F (V-3 bis V-5) mit Z = NMe oder NCOR, R₅,R₆= H, W= NH, A₁₋₄ = CH und R₁ bis R₃, Y₁ bis Y₄ und Y₁' bis Y₄' entsprechend (1) verglichen:

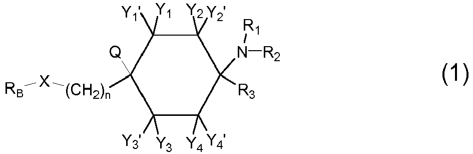

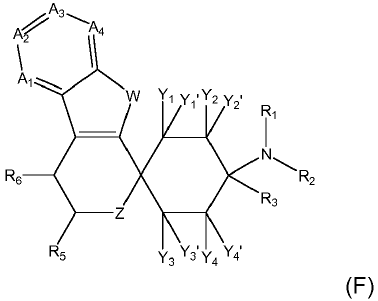

| Bsp. | R_{B} | Z | Diastereomer | Nephelometrie (lower bound) µM |
|---|---|---|---|---|
| 1 | Me | entfällt | polar | 100 |
| 49 | 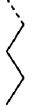 | entfällt | polar | 100 |
| 85 | 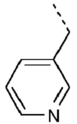 | entfällt | polar | 100 |
| V-3: | entfällt | NMe | polar | 3 |
| 92 | 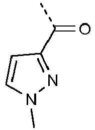 | entfällt | polar | 100 |
| 27 | 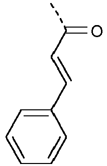 | entfällt | polar | 100 |
| V-4: | entfällt | 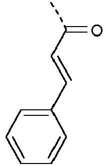 | polar | 12 |
| 30 | 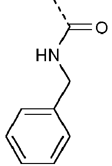 | entfällt | polar | 100 |
| V-5: | entfällt | 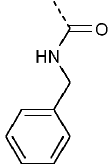 | unpolar | 12 |
| 31 | 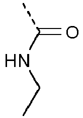 | entfällt | unpolar | 100 |
| 32 | 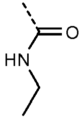 | entfällt | polar | 100 |

Wie der vorstehende Vergleich belegt, weisen die (früheren) erfindungsgemäßen Verbindungen aus den Beispielen 27, 30, 31, 32, 49, 85 und 92 im Vergleich zu strukturell ähnlichen Verbindungen (V-3 bis V-5) eine bessere Löslichkeit in wässrigen Medien auf, was insbesondere Vorteile im Hinblick auf die Resorptionseigenschaften und/oder die Bioverfügbarkeit mit sich bringen sollte.

## Patentansprüche

1. Verbindung der allgemeinen Formel (2.2) 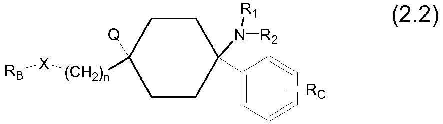 wobei
Q für -C₁₋₈-Aliphat, -Aryl, -C₁₋₈-Aliphat-Aryl, -Heteroaryl, -C(=O)-Heteroaryl oder -C(=NH)-Heteroaryl steht;
R₁ für -CH₃ steht;
R₂ für -H oder -CH₃ steht; oder
R₁ und R₂ gemeinsam einen Ring bilden und für -(CH₂)₃₋₄- stehen;
X für -O- oder -NR_{A}- steht;
R_{A} für -H oder -C₁₋₈-Aliphat steht;
R_{B} für -C(=O)-C₃₋₈-Cycloaliphat-Aryl, -C(=O)-C₃₋₈-Cycloaliphat-Heteroaryl, -C(=O)NH-C₁₋₈-Aliphat, -S(=O)₁₋₂-C₁₋₈-Aliphat, -S(=O)₁₋₂-Aryl, -S(=O)₁₋₂-Heteroary, -S(=O)₁₋₂-C₁₋₈-Aliphat-Aryl, -S(=O)₁₋₂-C₁₋₈-Aliphat-Heteroaryl, -S(=O)₁₋₂-C₃₋₈-CyCloaliphat-Aryl oder -S(=O)₁₋₂-C₃₋₈-Cycloaliphat-Heteroaryl steht; oder R_{A} und R_{B} gemeinsam einen Ring bilden und für -(CH₂)₃₋₄- stehen;
R_{C} für -H, -F, -Cl, -Br, -I, -CN, -NO₂, -CF₃, -OH oder -OCH₃ steht; und
n für 0, 1, 2, 3 oder 4 steht;
wobei
"Aliphat" jeweils ein verzweigter oder unverzweigter, gesättigter oder ein- oder mehrfach ungesättigter, unsubstituierter oder ein- oder mehrfach substituierter, aliphatischer Kohlenwasserstoffrest ist;
"Cycloaliphat" jeweils ein gesättigter oder ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf); wobei ggf. ein oder zwei Kohlenstoffatome unabhängig voneinander durch ein Heteroatom S, N oder O ersetzt sind;
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=0)R₀, -NHC(=O)OR₀, -NHC(=O)NH₂, -NHC(=O)-NHR₀, -NH-C(=O)N(R₀)₂, -Si(R₀)₃ und -PO(OR₀)₂ verstanden wird;
wobei R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃ oder -PO(OR₀)₂ verstanden wird; wobei ggf. vorhandene N-Ringatome jeweils oxidiert sein können;
wobei R₀ jeweils unabhängig für -C₁₋₈-Aliphat, -C₃₋₁₂-Cycloaliphat, -Aryl, -Heteroaryl, -C₁₋₈-Aliphat-C₃₋₁₂-CyCloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
in Form eines einzelnen Stereoisomers oder deren Mischung, der freien Verbindungen und/oder ihrer physiologisch verträglichen Salze.

2. Verbindung nach Anspruch 1, wobei
Q für -C₁₋₈-Alkyl, -Phenyl, -C₁₋₈-Alkyl-Phenyl, -Indolyl, -C(=O)-Indolyl oder -C(=NH)-Indolyl steht;
R₁ für -CH₃ steht;
R₂ für -H oder -CH₃ steht; oder
R₁ und R₂ gemeinsam einen Ring bilden und für -(CH₂)₃₋₄- stehen;
X für -O- oder -NR_{A}- steht;
R_{A} für -H oder -C₁₋₈-Alkyl steht;
R_{B} für -C(=O)-Cyclopropyl-Aryl, -C(=O)-Cyclopropyl-Heteroaryl, -C(=O)NH-C₁₋₈-Alkyl, -S(=O)₂C₁₋₈-Alkyl, -S(=O)₂-Phenyl, -S(=O)₂-Cyclopropyl-Aryl oder -S(=O)₂-Cyclopropyl-Heteroaryl steht;
R_{C} für -H, -F, -Cl, -Br, -I, -CN, -NO₂, -CF₃, -OH oder -OCH₃ steht; und
n für 0, 1, 2, 3 oder 4 steht;
wobei Aliphat, Aryl und Heteroaryl jeweils unsubstituiert oder ein- oder mehrfach substituiert sind.

3. Verbindung nach Anspruch 1, ausgewählt aus der Gruppe bestehend aus
• 4-(Dimethylamino)-4-phenyl-1-(pyrrolidin-1-yl)cyclohexyl)(1-methyl-1H-indol-2-yl)methanon;
• 4-(Dimethylamino)-4-phenyl-1-(pyrrolidin-1-yl)cyclohexyl)(1-methyl-1H-indol-2-yl)methanon;
• 4-Chloro-N-((-4-(dimethylamino)-1-methyl-4-phenylcyclohexyl)methyl)benzol-sulfonamid 2-hydroxypropan-1,2,3-tricarboxylat;
• N-((1-Butyl-4-(dimethylamino)-4-phenylcyclohexyl)methyl)-4-chlorobenzol-sulfonamid 2-hydroxypropan-1,2,3-tricarboxylat;
• N-(4-Dimethylamino-1,4-diphenyl-cyclohexyl)-N-methyl-methansulfonsäureamid (polares Diastereomer);
• 3-Benzyl-1-(4-dimethylamino-1,4-diphenyl-cyclohexyl)-1-methyl-harnstoff (unpolares Diastereomer);
• 3-Benzyl-1-(4-dimethylamino-1,4-diphenyl-cyclohexyl)-1-methyl-harnstoff (polares Diastereomer);
• 1-(4-Dimethylamino-1,4-diphenyl-cyclohexyl)-3-ethyl-1-methyl-harnstoff (unpolares Diastereomer);
• 1-(4-Dimethylamino-1,4-diphenyl-cyclohexyl)-3-ethyl-1-methyl-harnstoff (polares Diastereomer);
• (E)-N-[[4-Dimethylamino-4-(3-fluorphenyl)-1-methyl-cyclohexyl]-methyl]-2-phenyl-ethensulfonsäureamid (unpolares Diastereomer);
• (E)-N-[[4-Dimethylamino-4-(3-fluorphenyl)-1-methyl-cyclohexyl]-methyl]-2-phenyl-ethensulfonsäureamid (polares Diastereomer);
• Dimethyl-[4-(3-methyl-1H-indol-2-yl)-1-phenyl-4-pyrrolidin-1-yl-cyclohexyl]-amindihydrochlorid (polares Diastereomer);
• Dimethyl-[4-(3-methyl-1H-indol-2-yl)-1-phenyl-4-pyrrolidin-1-yl-cyclohexyl]-amin;
• [4-(Azetidin-1-yl)-4-(3-methyl-1H-indol-2-yl)-1-phenyl-cyclohexyl]-dimethyl-amin (unpolares Diastereomer);
• N-(4-Dimethylamino-1,4-diphenyl-cyclohexyl)-N-methyl-methansulfonsäureamid (unpolares Diastereomer);
• 4-(Azetidin-1-yl)-1-(3-fluorophenyl)-N,N-dimethyl-4-(3-methyl-1H-indol-2-yl)cyclohexanamin (unpolares Diastereomer);
• 4-(Azetidin-1-yl)-1-(3-fluorophenyl)-N,N-dimethyl-4-(3-methyl-1H-indol-2-yl)cyclohexanamin (polares Diastereomer);
• [1-(3-Fluorphenyl)-4-(3-methyl-1H-indol-2-yl)-4-pyrrolidin-1-yl-cyclohexyl]-dimethyl-amin (unpolares Diastereomer);
• [1-(3-Fluorphenyl)-4-(3-methyl-1H-indol-2-yl)-4-pyrrolidin-1-yl-cyclohexyl]-dimethyl-amin (polares Diastereomer);
• [4-(5-Fluor-3-methyl-1H-indol-2-yl)-1-phenyl-4-pyrrolidin-1-yl-cyclohexyl]-dimethyl-amin (unpolares Diastereomer);
• [4-(5-Fluor-3-methyl-1H-indol-2-yl)-1-phenyl-4-pyrrolidin-1-yl-cyclohexyl]-dimethyl-amin (polares Diastereomer);
• [4-(Azetidin-1-yl)-4-(5-fluor-3-methyl-1H-indol-2-yl)-1-(3-fluorphenyl)-cyclohexyl]-dimethyl-amin (unpolares Diastereomer);
• [4-(Azetidin-1-yl)-4-(5-fluor-3-methyl-1H-indol-2-yl)-1-(3-fluorphenyl)-cyclohexyl]-dimethyl-amin (polares Diastereomer);
• (1,4-Diphenyl-4-pyrrolidin-1-yl-cyclohexyl)-methyl-amin (polares Diastereomer);
• (1,4-Diphenyl-4-pyrrolidin-1-yl-cyclohexyl)-methyl-amin (unpolares Diastereomer);
• (1,4-Diphenyl-4-pyrrolidin-1-yl-cyclohexyl)-dimethyl-amin (polares Diastereomer);
• (1,4-Diphenyl-4-pyrrolidin-1-yl-cyclohexyl)-dimethyl-amin (unpolares Diastereomer);
und deren physiologisch verträgliche Salze.

4. Arzneimittel enthaltend wenigstens eine Verbindung nach einem der Ansprüche 1 bis 3 in Form eines einzelnen Stereoisomers oder deren Mischung, der freien Verbindungen und/oder ihrer physiologisch verträglichen Salze, sowie ggf. geeignete Zusatz- und/oder Hilfsstoffe und/oder gegebenenfalls weiterer Wirkstoffe.

5. Verwendung einer Verbindung nach einem der Ansprüche 1 bis 3 in Form eines einzelnen Stereoisomers oder deren Mischung, der freien Verbindung und/oder ihrer physiologisch verträglichen Salze, zur Herstellung eines Arzneimittels zur Behandlung von Schmerz.

6. Verwendung einer Verbindung nach einem der Ansprüche 1 bis 3 in Form eines einzelnen Stereoisomers oder deren Mischung, der freien Verbindungen und/oder ihrer physiologisch verträglichen Salze und/oder Solvate zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anästhetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. A compound of the general formula (2.2) 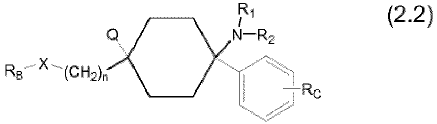 wherein
Q represents -C₁₋₈-aliphatic, -aryl, -C₁₋₈-aliphatic-aryl,-heteroaryl, -C(=O)-heteroaryl or -C(=NH)-heteroaryl;
R₁ represents -CH₃;
R₂ represents -H or -CH₃; or
R₁ and R₂ together form a ring and represent -(CH₂)₃₋₄-;
X represents -O- or -NR_{A}-;
R_{A} represents -H or -C₁₋₈-aliphatic;
R_{B} represents -C(=O)-C₃₋₈-cycloaliphatic-aryl, -C(=O)-C₃₋₈-cycloaliphatic-heteroaryl, -C(=O)NH-C₁₋₈-aliphatic, -S(=O)₁₋₂-C₁₋₈-aliphatic, -S(=O)₁₋₂-aryl, -S(=O)₁₋₂-heteroaryl, -S(=O)₁₋₂-C₁₋₈-aliphatic-aryl, -S(=O)₁₋₂-C₁₋₈-aliphatic-heteroaryl, -S(=O)₁₋₂-C₃₋₈-cycloaliphatic-aryl or -S(=O)₁₋₂-C₃₋₈-cycloaliphatic heteroaryl; or R_{A} and R_{B} together form a ring and represent-(CH₂)₃₋₄- ;
Rc represents -H, -F, -Cl, -Br, -I, -CN, -NO₂, -CF₃, -OH or-OCH₃; and
n represents 0, 1, 2, 3 or 4;
wherein
"aliphatic" is in each case a branched or unbranched, saturated or mono- or polyunsaturated, unsubstituted or mono- or polysubstituted, aliphatic hydrocarbon radical;
"Cycloaliphatic" is in each case a saturated or mono- or polyunsaturated, unsubstituted or mono- or polysubstituted, alicyclic, monocyclic or multicyclic hydrocarbon radical whose number of ring carbon atoms is preferably in the stated range (i. e. "C₃₋₈-"cycloaliphatic preferably has 3, 4, 5, 6, 7 or 8 ring carbon atoms); wherein, optionally, one or two carbon atoms independently are substituted by a heteroatom S, N or O; wherein, with respect to "aliphatic" and "cycloaliphatic", "mono- or polysubstituted" means the single or multiple substitution of one or more hydrogen atoms, e.g., the single, double, triple or complete substitution, by substituents independently selected from the group consisting of -F, -Cl,-Br, -I, -CN, -NO₂, -CHO, =O, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀,-OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R_{C}, -S(=O)₁₋₂NH₂, -NH₂, -NHR₀, -N(R₀)₂,-N⁺(R₀)₃, -N⁺ (R₀)₂O⁻, -NHC(=O)R₀, -NHC(=O)OR₀, -NHC(=O)NH₂,-NHC(=O)-NHR₀, -NH-C(=O)N(R₀)₂, -Si(R₀)₃ and -PO(OR₀)₂;
wherein R₀ in each case independently represents of -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, C₃₋₈-cycloaliphatic-aryl or C₃₋₈-cycloaliphatic heteroaryl;
"Aryl" in each case independently represents a carbocyclic ring system having at least one aromatic ring, but without heteroatoms in this ring, wherein the aryl radicals, optionally, may be fused with further saturated, (partially) unsaturated or aromatic ring systems and each aryl radical may be unsubstituted or may be monosubstituted or polysubstituted, wherein the aryl substituents may be the same or different and at any and possible position of the aryl;
"Heteroaryl" represents a 5-, 6- or 7-membered cyclic aromatic radical containing 1, 2, 3, 4 or 5 heteroatoms, wherein the heteroatoms are the same or different nitrogen, oxygen or sulfur, and the heterocycle may be unsubstituted or may be monosubstituted or polysubstituted; wherein, in the case of substitution on the heterocycle, the substituents may be the same or different and at any and possible position of the heteroaryl; and wherein the heterocycle may also be part of a bi- or polycyclic system;
wherein, with respect to "aryl" and "heteroaryl", "monosubstituted or polysubstituted" is understood to mean the single or multiple substitution of one or more hydrogen atoms of the ring system by substituents selected from the group consisting of -F, -Cl, -Br, -I, -CN, -NO₂, -CHO, =O, -R₀,-C(=O)R₀, -C(=O)H, -C(=O)OH, -C(=O)OR₀, -C(=O)NH₂, -C(=O)NHR₀,-C(=O)(R₀)₂, -OH, -O(CH₂)₁₋₂O-, -OR_{C}, -OC(=O)H, -OC(=O)R₀,-OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻,-NHC(=O)R₀, -NHC(=O)OR_{C}, C(=O)NH₂, -NHC(=O)NHR₀, -NHC(=O)N(R₀)₂, -Si(R₀)₃ or -PO(OR₀)₂; wherein, optionally, existing N-ring atoms may each be oxidized;
wherein R₀ in each case independently represents -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C-₁₋₈-aliphatic, C₃₋₈-cycloaliphatic-aryl, or C₃₋₈-cycloaliphatic-heteroaryl;
in the form of a single stereoisomer or a mixture thereof, the free compounds and/or their physiologically acceptable salts.

2. The compound according to claim 1, wherein
Q represents -C₁₋₈-alkyl, phenyl, -C₁₋₈-alkyl-phenyl, indolyl;-C(=O)-indolyl or -C(=NH)-indolyl;
R₁ represents -CH₃;
R₂ represents -H or -CH₃; or
R₁ and R₂ together form a ring and represent - (CH₂)₃₋₄-;
X represents -O- or -NR_{A}-;
R_{A} represents -H or -C₁₋₈-alkyl;
R_{B} represents -C(=O)-cyclopropyl-aryl, -C(=O)-cyclopropyl-heteroaryl, -C (=O) NH-C₁₋₈-alkyl, -S (=O) ₂-C₁₋₈-alkyl, -S (=O) ₂-phenyl, -S(=O)₂-cyclopropyl-aryl or -S(=O)₂-cyclopropyl-heteroaryl;
R_{c} represents -H, -F, -Cl, -Br, -I, -CN, -NO₂, -CF₃, -OH or - OCH₃; and
n represents 0, 1, 2, 3 or 4;
wherein aliphatic, aryl and heteroaryl are each unsubstituted or monosubstituted or polysubstituted.

3. The compound according to claim 1, selected from the group consisting of
• 4-(dimethylamino)-4-phenyl-1-(pyrrolidin-1-yl)cyclohexyl(1-methyl-1H-indol-2-yl)methanon;
• 4-(dimethylamino)-4-phenyl-1-(pyrrolidin-1-yl)cyclohexyl(1-methyl-1H-indol-2-yl)methanon;
• 4-chloro-N-((-4-(dimethylamino)-1-methyl-4-phenylcyclohexyl)methylbenzenesulfonamide-2-hydroxypropane-1,2,3-tricarboxylate;
• N-((1-butyl-4-(dimethylamino)-4-phenylcyclohexyl)methyl)-4-chlorobenzenesulfonamide-2-hydroxypropane-1,2,3-carboxylate;
• N-(4-dimethylamino-1,4-diphenylcyclohexyl)-N-methylmethanesulfonamide (polar diastereomer);
• 3-benzyl-1-(4-dimethylamino-1,4-diphenylcyclohexyl)-1-methyl-urea (nonpolar diastereomer);
• 3-benzyl-1-(4-dimethylamino-1,4-diphenylcyclohexyl)-1-methyl-urea(polar diastereomer);
• 1-(4-dimethylamino-1,4-diphenylcyclohexyl)-3-ethyl-1-methyl-urea(nonpolar diastereomer);
• 1-(4-dimethylamino-1,4-diphenylcyclohexyl)-3-ethyl-1-methyl-urea(polar diastereomer);
• (E)-N[[4-dimethylamino-4-(3-fluorophenyl)-1-methylcyclohexyl]methyl]-2-phenylethenesulfonamide (nonpolar diastereomer);
• (E)-N[[4-dimethylamino-4-(3-fluorophenyl)-1-methylcyclohexyl]methyl]-2-phenylethenesulfonamide (polar diastereomer);
• dimethyl-[4-(3-methyl-1H-indol-2-yl)-1-phenyl-4-pyrrolidinyl-1-cyclohexyl]amidine-dihydrochloride (polar diastereomer);
• dimethyl-[4-(3-methyl-1H-indol-2-yl)-1-pheny1-4-pyrrolidin-1-yl-cyclohexy]amine;
• [4-(azetidine-1-yl)-4-(3-methyl-1H-indol-2-yl)-1-phenylcyclohexyl]dimethylamine (nonpolar diastereomer);
• N-(4-dimethylamino-1,4-diphenylcyclohexyl)-N-methylmethanesulfonamide (nonpolar diastereomer);
• 4-(azetidin-l-yl)-1-(3-fluorophenyl)-N,N-dimethyl-4-(3-methyl-1H-indol-2-yl)cyclohexanamine (nonpolar diastereomer);
• 4-(azetidin-1-yl)-1-(3-fluorophenyl)-N,N-dimethyl-4-(3-methyl-1H-indol-2-yl)cyclohexanamine (polar diastereomer);
• [1-(3-fluorophenyl)-4-(3-methyl-1H-indol-2-yl)-4-pyrrolidin-1-ylcyclohexyl]dimethylamine (nonpolar diastereomer);
• [1-(3-fluorophenyl)-4-(3-methyl-1H-indol-2-yl)-4-pyrrolidin-1-ylcyclohexyl]dimethylamine(polar diastereomer);
• [4-(5-fluoro-3-methyl-1H-indol-2-yl)-1-phenyl-4-pyrrolidin-1-ylcyclohexyl]dimethylamine (nonpolar diastereomer);
• [4-(5-fluoro-3-methyl-1H-indol-2-yl)-1-phenyl-4-pyrrolidin-1-ylcyclohexyl]dimethylamine (polar diastereomer);
• [4-(azetidine-1-yl)-4-(5-fluoro-3-methyl-1H-indol-2-yl)-1-(3-fluorophenyl)cyclohexyl]dimethylamine(nonpolar diastereomer);
• [4-(azetidine-1-yl)-4-(5-fluoro-3-methyl-1H-indol-2-yl)-1-(3-fluorophenyl)cyclohexyl]dimethylamine (polar diastereomer);
• (1,4-diphenyl-4-pyrrolidin-1-ylcyclohexyl)methylamine (polar diastereomer);
• (1,4-diphenyl-4-pyrrolidin-1-ylcyclohexyl)methylamine (nonpolar diastereomer);
• (1,4-diphenyl-4-pyrrolidin-1-yl-cyclohexyl)dimethylamine (polar diastereomer);
• (1,4-diphenyl-4-pyrrolidin-1-yl-cyclohexyl)dimethylamine (nonpolar diastereomer);
and their physiologically acceptable salts.

4. Medicaments containing at least one compound according to any one of claims 1 to 3 in the form of a single stereoisomer or a mixture thereof, the free compounds and/or their physiologically acceptable salts, and, optionally, suitable additives and/or excipients and/or, optionally, other active ingredients.

5. Use of a compound according to any one of claims 1 to 3 in the form of a single stereoisomer or a mixture thereof, the free compound and/or its physiologically acceptable salts, for the manufacture of a medicament for the treatment of pain.

6. Use of a compound according to any one of claims 1 to 3 in the form of a single stereoisomer or a mixture thereof, the free compounds and/or their physiologically acceptable salts and/or solvates for the manufacture of a medicament for the treatment of anxiety, stress and stress-related syndromes, depression, epilepsy, Alzheimer's disease, senile dementia, general cognitive dysfunctions, learning and memory disorders (as nootropic agent), withdrawal symptoms, alcohol and/or drug and/or substance abuse and/or dependence, sexual dysfunctions, cardiovascular diseases, hypotension, hypertension, tinnitus, pruritus, migraine, hearing impairment, deficient bowel motility, impaired food intake, anorexia, obesity, locomotor disorders, diarrhea, cachexia, urinary incontinence or as muscle relaxant, anticonvulsant or anesthetic, or for coadministration in the treatment with an opioid analgesic or an anesthetic, for diuresis or antinatriuresis, anxiolysis, for modulation of movement activity, for modulation of neurotransmitter release and treatment of neurodegenerative diseases associated therewith, for the treatment of withdrawal symptoms and/or for the reduction of the addiction potential of opioids.

## Revendications

1. Composé de formule générale (2.2) 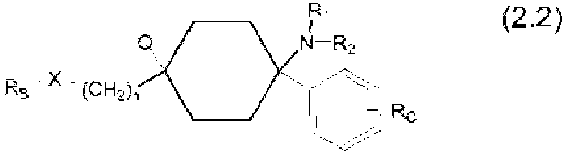 dans lequel
Q représente un groupe aliphatique en -C₁ à C₈, -aryle, aliphatique en -C₁ à C₈-aryle, -hétéroaryle, -C(=O)-hétéroaryle ou -C(=NH)-hétéroaryle ;
R₁ représente -CH₃ ;
R₂ représente -H ou -CH₃ ; ou
R₁ et R₂ forment conjointement un cycle et représentent -(CH₂)₃-4 ;
X représente -O- ou -NR_{A}- ;
R_{A} représente -H ou un groupe aliphatique en -C₁ à C₈ ;
R_{B} représente un groupe -C(=O)-cycloaliphatique en C₃ à C₈-aryle, -C(=O)-cycloaliphatique en C₃ à C₈-hétéroaryle, - C (=O) NH-aliphatique en C₁ à C₈, -S (=O) ₁₋₂-aliphatique en C₁ à C₈, -S (=O)₁₋₂-aryle, -S (=O)₁₋₂-hétéroaryle, -S (=O) ₁₋₂-aliphatique en C₁ à C₈-aryle, -S (=O)₁₋₂-aliphatique en C₁ à C₈-hétéroaryle, - S(=O)₁₋₂-cycloaliphatique en C₃ à C₈ ou -S (=O)₁₋₂-cycloaliphatique en C₃ à C₈-hétéroaryle ; ou R_{A} et R_{B} forment conjointement un cycle et représentent -(CH₂)₃₋₄ ;
R_{C} représente -H, -F, -Cl, -Br, -I, -CN, -NO₂, -CF₃, -OH ou - OCH₃ ; et
n représente 0, 1, 2, 3 ou 4 ;
dans lequel
un groupe « aliphatique » est respectivement un radical hydrocarbure aliphatique ramifié ou non ramifié, saturé ou mono- ou poly-insaturé, non substitué ou mono- ou poly-substitué ;
un groupe « cycloaliphatique » est respectivement un radical hydrocarbure alicylique, mono- ou polycyclique saturé ou mono- ou poly-insaturé, non substitué ou mono- ou polysubstitué dont le nombre des atomes d'hydrocarbure cycliques se situe de préférence dans la plage indiquée (c'est-à-dire que le groupe cycloaliphatique « en C₃ à C₈ » présente de préférence 3, 4, 5, 6, 7 ou 8 atomes de carbone cycliques) ; dans lequel éventuellement un ou deux atomes de carbone sont remplacés indépendamment l'un de l'autre par un hétéroatome S, N ou O ; dans lequel concernant les groupes « aliphatique » et « cycloaliphatique », on comprend par « mono- ou polysubstitué », la mono- ou polysubstitution d'un ou plusieurs atomes d'hydrogène, par exemple la substitution unique, double, triple ou complète, par des substituants choisis indépendamment les uns des autres dans le groupe constitué de -F, -Cl, -Br, -I, -CN, -NO₂, -CHO, =O, -R₀, - C(=O)R₀, -C(=O)H, -C(=O)OH, -C(=O)OR₀, -C(=O)NH₂, -C(=O)NHR₀, - C(=O)N(R₀)₂, -OH, -OR_{C}, -OC(=O)H, -OC(=O)R₀, -OC(=O)OR₀, - OC (=O) NHR₀, -OC(=O)N(R_{C})₂, -SH, -SR₀, -SO₃H, -S (=O) ₁₋₂-R₀, - S (=O) ₁₋₂NH₂, -NH₂, -NHR₀, -N (R₀)₂, -N⁺ (R₀)₃, -N⁺ (R₀)₂O⁻, -NHC (=O)R₀, -NHC (=O) OR₀, -NHC(=O)NH₂, -NHC (=O) -NHR₀, -NH-C (=O) N(R₀)₂, - Si (R₀)₃ et -PO(OR₀)₂ ;
dans lequel R₀ représente respectivement indépendamment un groupe aliphatique en -C₁ à C₈, cycloaliphatique en -C₃ à C₁₂, - aryle, -hétéroaryle, aliphatique en -C₁ à C₈-cycloaliphatique en C₃ à C₁₂, aliphatique en -C₁ à C₈-aryle, aliphatique en -C₁ à C₈-hétéroaryle, cycloaliphatique en -C₃ à C₈-aliphatique en -C₁ à C₈, cycloaliphatique en -C₃ à C₈-aryle ou cycloaliphatique en -C₃ à C₈-hétéroaryle ;
« aryle » représente respectivement indépendamment un système cyclique carbocyclique comportant au moins un cycle aromatique, mais sans hétéroatomes dans ce cycle, dans lequel les radicaux aryle peuvent être condensés éventuellement avec d'autres systèmes cycliques saturés, (partiellement) insaturés ou aromatiques et chaque radical aryle peut se présenter non substitué ou mono- ou poly-substitué, dans lequel les substituants aryle peuvent être identiques ou différents et dans n'importe quelle position et chaque position possible du groupe aryle,
« hétéroaryle » représente un radical aromatique cyclique à 5, 6 ou 7 chaînons qui contient 1, 2, 3, 4 ou 5 hétéroatomes, dans lequel les hétéroatomes, identiques ou différents, sont l'azote, l'oxygène ou le soufre, et l'hétérocycle peut être non substitué ou mono- ou polysubstitué ; dans lequel dans le cas de la substitution sur l'hétérocycle, les substituants peuvent être identiques ou différents et dans n'importe quelle position et chaque position possible du groupe hétéroaryle ; et dans lequel l'hétérocycle peut également faire partie d'un système bicyclique ou polycyclique ;
dans lequel concernant les groupes « aryle » et « hétéroaryle », on comprend par « mono- ou polysubstitué », la mono- ou polysubstitution d'un ou plusieurs atomes d'hydrogène du système cyclique par des substituants choisis dans le groupe constitué de -F, -Cl, -Br, -I, -CN, -NO₂, -CHO, =O, -R₀, -C(=O)R₀, -C(=O)H, -C(=O)OH, -C(=O)OR₀, -C(=O)NH₂, - C(=O)NHR₀, -C(=O)N(R₀)₂, -OH, -O(CH₂)₁₋₂O-, -OR₀, -OC(=O)H, - OC(=O)R₀, -OC(=O)OR_{C}, -OC(=O)NHR₀, -OC(=O)N(R₀)₂, -SH, -SR₀, - SO₃H, -S (=O)₁₋₂-R₀, -S (=O)₁₋₂NH₂, -NH₂, -NHR₀, -N(R₀)₂, -N⁺(R₀)₃, - N⁺(R₀)₂0⁻, -NHC(=O)R₀, -NHC(=O)OR₀, -NH-C (=O) NH₂, -NHC (=O) -NHR₀, -NHC (=O)N(R₀)₂, -Si (R₀)₃ ou -PO(OR₀)₂ ;
dans lequel des atomes cycliques de N éventuellement présents peuvent être oxydés respectivement ;
dans lequel R₀ représente respectivement indépendamment un groupe aliphatique en -C₁ à C₈, cycloaliphatique en -C₃ à C₁₂, - aryle, -hétéroaryle, aliphatique en -C₁ à C₈-cycloaliphatique en C₃ à C₁₂, aliphatique en -C₁ à C₈-aryle, aliphatique en -C₁ à C₈-hétéroaryle, cycloaliphatique en -C₃ à C₈-aliphatique en -C₁ à C₈, cycloaliphatique en -C₃ à C₈-aryle ou cycloaliphatique en -C₃ à C₈-hétéroaryle ;
sous la forme d'un stéréo-isomère individuel ou leur mélange, des composés libres et/ou leurs sels physiologiquement acceptables.

2. Composé selon la revendication 1, dans lequel
Q représente un groupe alkyle en -C₁ à C₈, -phényle, -alkyle en -C₁ à C₈-phényle, -indolyle, -C(=O)-indolyle ou -C(=NH)-indolyle ;
R₁ représente -CH₃ ;
R₂ représente -H ou -CH₃ ; ou
R₁ et R₂ forment conjointement un cycle et représentent -(CH₂)₃-4⁻ ;
X représente -O- ou -NR_{A}- ;
R_{A} représente -H ou un groupe alkyle en -C₁ à C₈ ;
R_{B} représente un groupe -C(=O)-cylopropyl-aryle, -C(=O)-cyclopropyl-hétéroaryle, -C(=O)NH-alkyle en C₁ à C₈, -S(=O)₂-alkyle en C₁ à C₈, -S(=O)₂-phényle, -S(=O)₂-cyclopropyl-aryle ou -S(=O)₂-cyclopropyl-hétéroaryle ;
R_{C} représentent -H, -F, -Cl, -Br, -I, -CN, -NO₂, -CF₃, -OH ou - OCH₃ ; et
n représente 0, 1, 2, 3 ou 4 ;
dans lequel les groupes aliphatique, aryle et hétéroaryle sont respectivement non substitués ou mono- ou polysubstitués.

3. Composé selon la revendication 1, choisi dans le groupe constitué de
* 4-(diméthylamino)-4-phényl-1-(pyrrolidin-1-yl)cyclohexyl)(1-méthyl-1H-indol-2-yl)méthanone ;
* 4-(diméthylamino)-4-phényl-1-(pyrrolidin-1-yl)cyclohexyl)(1-méthyl-1H-indol-2-yl)méthanone ;
* 4-chloro-N-((4-(diméthylamino)-1-méthyl-4-phénylcyclohexyl)méthyl)benzol-sulfonamide-2-hydroxypropane-1,2,3-tricarboxylate ;
* N-((1-butyl-4-(diméthylamino)-4-phénylcyclohexyl)méthyl)-4-chlorobenzol-sulfonamide-2-hydroxypropane-1,2,3-tricarboxylate ;
* amide de l'acide N-(4-(diméthylamino)-1,4-diphényl-cyclohexyl)-N-méthyl-méthanesulfonique (diastéréomère polaire) ;
* 3-benzyl-1-(4-diméthylamino-1,4-diphényl-cyclohexyl)-1-méthylurée (diastéréomère non polaire) ;
* 3-benzyl-1-(4-diméthylamino-1,4-diphényl-cyclohexyl)-1-méthylurée (diastéréomère polaire) ;
* 1-(4-(diméthylamino-1,4-diphényl-cyclohexyl)-3-éthyl-1-méthylurée (diastéréomère non polaire) ;
* 1-(4-(diméthylamino-1,4-diphényl-cyclohexyl)-3-éthyl-1-méthylurée (diastéréomère polaire) ;
* amide de l'acide (E)-N-[[4-diméthylamino-4-(3-fluorophényl)-1-méthyl-cyclohexyl]-méthyl]-2-phényléthénsulfonique (diastéréomère non polaire) ;
* amide de l'acide (E)-N-[[4-diméthylamino-4-(3-fluorophényl)-1-méthyl-cyclohexyl]-méthyl]-2-phényléthénsulfonique (diastéréomère polaire) ;
* dichlorhydrate de diméthyl-[4-(3-méthyl-1H-indol-2-yl)-1-phényl-4-pyrrolidin-1-yl-cyclohexyl]-amine (diastéréomère polaire) ;
* diméthyl-[4-(3-méthyl-1H-indol-2-yl)-1-phényl-4-pyrrolidin-1-yl-cyclohexyl]-amine ;
* [4-(azétidin-1-yl)-4-(3-méthyl-1H-indol-2-yl)-1-phényl-cyclohexyl]-diméthyl-amine (diastéréomère non polaire) ;
* amide de l'acide N-(4-diméthylamino-1,4-diphényl-cyclohexyl)-N-méthyl-méthanesulfonique (diastéréomère non polaire) ;
* 4-(azétidin-1-yl)-1-(3-fluorophényl)-N,N-diméthyl-4-(3-méthyl-1H-indol-2-yl)cyclohexanamine (diastéréomère non polaire) ;
* 4-(azétidin-1-yl)-1-(3-fluorophényl)-N,N-diméthyl-4-(3-méthyl-1H-indol-2-yl)cyclohexanamine (diastéréomère polaire) ;
* [1-(3-fluorophényl)-4-(3-méthyl-1H-indol-2-yl)-4-pyrrolidin-1-yl-cyclohexyl]-diméthyl-amine (diastéréomère non polaire) ;
* [1-(3-fluorophényl)-4-(3-méthyl-1H-indol-2-yl)-4-pyrrolidin-1-yl-cyclohexyl]-diméthyl-amine (diastéréomère polaire) ;
* [4-(5-fluoro-3-méthyl-1H-indol-2-yl)-1-phényl-4-pyrrolidin-1-yl-cyclohexyl]-diméthyl-amine (diastéréomère non polaire) ;
* [4-(5-fluoro-3-méthyl-1H-indol-2-yl)-1-phényl-4-pyrrolidin-1-yl-cyclohexyl]-diméthyl-amine (diastéréomère polaire) ;
* [4-(azétidin-1-yl)-4-(5-fluoro-3-méthyl-1H-indol-2-yl)-1-(3-fluorophényl)-cyclohexyl]-diméthyl-amine (diastéréomère non polaire) ;
* [4-(azétidin-1-yl)-4-(5-fluoro-3-méthyl-1H-indol-2-yl)-1-(3-fluorophényl)-cyclohexyl]-diméthyl-amine (diastéréomère polaire) ;
* (1,4-diphényl-4-pyrrolidin-1-yl-cyclohexyl)-méthyl-amine (diastéréomère polaire) ;
* (1,4-diphényl-4-pyrrolidin-1-yl-cyclohexyl)-méthyl-amine (diastéréomère non polaire) ;
* (1,4-diphényl-4-pyrrolidin-1-yl-cyclohexyl)-diméthyl-amine (diastéréomère polaire) ;
* (1,4-diphényl-4-pyrrolidin-1-yl-cyclohexyl)-diméthyl-amine (diastéréomère non polaire) ;
et leurs sels physiologiquement acceptables.

4. Médicament contenant au moins un composé selon l'une des revendications 1 à 3 sous la forme d'un stéréo-isomère individuel ou leur mélange, des composés libres et/ou leurs sels physiologiquement acceptables, ainsi que, éventuellement, des additifs et/ou adjuvants appropriés et/ou éventuellement d'autres principes actifs.

5. Utilisation d'un composé selon l'une des revendications 1 à 3, sous la forme d'un stéréo-isomère individuel ou leur mélange, du composé libre et/ou leurs sels physiologiquement acceptables, pour la production d'un médicament pour le traitement de la douleur.

6. Utilisation d'un composé selon l'une des revendications 1 à 3, sous la forme d'un stéréo-isomère individuel ou leur mélange, des composés libres et/ou leurs sels et/ou solvates physiologiquement acceptables, pour la production d'un médicament pour le traitement des états anxieux, du stress et des syndromes liés au stress, des dépressions, de l'épilepsie, de la maladie d'Alzheimer, de la démence sénile, des dysfonctionnements cognitifs généraux, des troubles de l'apprentissage et de la mémoire (en tant que nootropes), des symptômes de sevrage, de l'abus et/ou de la dépendance à l'alcool et/ou aux drogues et/ou aux médicaments, des dysfonctionnements sexuels, des maladies cardiovasculaires, de l'hypotension, de l'hypertension, des acouphènes, du prurit, de la migraine, de la surdité, de défaut de motilité intestinale, de troubles de l'absorption de nourriture, de l'anorexie, de l'obésité, de troubles locomoteurs, de diarrhée, de cachexie, de l'incontinence urinaire ou en tant que myorelaxant, anticonvulsivant ou anesthésique ou pour la co-administration dans le cas d'un traitement avec un analgésique opiacé ou avec un anesthésique, pour la diurèse ou l'anti-natriurèse, l'anxiolyse, pour la modulation de l'activité motrice, pour la modulation de la libération de neurotransmetteurs et le traitement de maladies neurodégénératives liées à celle-ci, pour le traitement des syndromes de sevrage et/ou pour la réduction du potentiel addictif des opiacés.
